(19) European Patent Office / Europäisches Patentamt / Office européen des brevets

(11) **EP 4 778 921 A1**

(12) **EUROPEAN PATENT APPLICATION**
published in accordance with Art. 153(4) EPC

(43) Date of publication:
**22.07.2026 Bulletin 2026/30**

(21) Application number: **24864768.7**

(22) Date of filing: **14.09.2024**

(51) International Patent Classification (IPC):
***C07D 401/14*** *(2006.01)* ***C07D 471/04*** *(2006.01)*
***A61P 35/00*** *(2006.01)* ***A61K 31/506*** *(2006.01)*

(52) Cooperative Patent Classification (CPC):
**A61K 31/506; A61P 35/00; C07D 401/14; C07D 471/04**

(86) International application number:
**PCT/CN2024/119020**

(87) International publication number:
**WO 2025/056053 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.09.2023 CN 202311197754**
**15.09.2023 CN 202311198733**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**

(72) Inventors:
- **ZHAN, Xiaolan**
  **Shanghai 201203 (CN)**
- **GUO, Linsong**
  **Shanghai 201203 (CN)**

(74) Representative: **Cabinet Beau de Loménie**
**103, rue de Grenelle / CS 90800**
**75340 Paris Cedex 07 (FR)**

(54) **SALT FORM AND CRYSTAL FORM OF NITROGEN-CONTAINING HETEROCYCLIC DERIVATIVE INHIBITOR, AND PREPARATION METHOD THEREFOR AND USE THEREOF**

(57) The present invention relates to a salt form and a crystal form of a nitrogen-containing heterocyclic derivative inhibitor, and a preparation method therefor and the use thereof. In particular, the present invention relates to a salt form and a crystal form of a compound as shown in the general formula, and relates to a preparation method therefor, a pharmaceutical composition containing the compound, and the use thereof as an inhibitor for treating cancers.

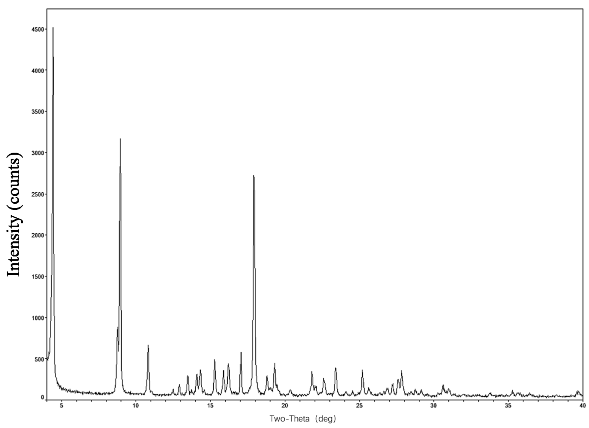


*FIG. 1*

EP 4 778 921 A1

## Description

## Technical Field

The present invention belongs to the field of biomedicine and particularly relates to a salt form and a crystal form of a nitrogen-containing heterocyclic derivative inhibitor, and a preparation method therefor and the use thereof.

## Background Art

EGFR (Epidermal Growth Factor Receptor) is a member of the ErbB family of transmembrane receptor tyrosine kinases, which is activated by binding to its ligand epidermal growth factor (EGF) or transforming growth factor $\alpha$ (TGF$\alpha$). Activated EGFR forms homodimers on the cell membrane, or forms heterodimers with other receptors of the family (such as ErbB-2, ErbB-3, or ErbB-4), resulting in phosphorylation of the key tyrosine residues in EGFR cells, and activation of intracellular downstream signalling pathways, which plays an important role in cell proliferation, survival and anti-apoptosis. Activating mutations, overexpression or gene amplification of EGFR can lead to excessive activation of EGFR, accelerate the transformation of cells into tumour cells, and play an important role in the proliferation, invasion, metastasis and angiopoiesis of tumour cells. It is an important target for the development of anti-cancer drugs, especially drugs for treating lung cancer.

First-generation EGFR small molecule inhibitors including gefitinib (Iressa) and erlotinib (Tarceva) have shown good efficacy in the treatment of lung cancer and have been used as first-line drugs for the treatment of non-small cell lung cancer (NSCLC) with EGFR-activating mutations (including L858R and delE746_A750). However, after 10 to 12 months of treatment with first-generation small molecule EGFR inhibitors, almost all NSCLC patients develop resistance to the first-generation small molecule inhibitors, and according to the resistance mechanism, more than half of the cases are due to a secondary mutation in the EGFR gatekeeper gene residue, T790M.

Osimertinib (AZD9291) is a third-generation EGFR TKI inhibitor that has a high response rate and good therapeutic effect against drug resistance caused by EGFR T790M mutation. It received accelerated approval from the US FDA in November 2015 and is clinically effective in treating advanced non-small cell lung cancer patients with drug-resistant EGFR T790M mutation. Although osimertinib has achieved great success in clinical treatment of non-small cell lung cancer with EGFR T790M mutation, patients still inevitably develop drug resistance after 9 to 14 months of treatment. Studies have shown that up to 20%-40% of patients are drug-resistant due to the EGFR C797S mutation. The EGFR C797S mutation causes the change of cysteine at position 797 to serine, resulting in the inability of osimertinib to form a covalent bond with the EGFR protein, thus causing drug resistance. Currently, there are no effective clinical inhibitors targeting the drug-resistant EGFR C797S mutation. Therefore, there is an urgent need to develop new and highly active EGFR inhibitors to solve the problem of drug resistance caused by EGFR C797S mutation.

Novartis reported compound EAI0450, an EGFR allosteric inhibitor, targeting drug-resistant EGFR C797S mutation. When combined with EGFR monoclonal antibodies such as cetuximab, it showed good anti-tumour effects in a mouse *in vivo* pharmacodynamic model with L858R/T790M/C797S mutations. However, as a single drug, this compound is ineffective and cannot inhibit drug-resistant C797S mutation (including deIE746_A750) and has not been included in clinical research. In 2017, Ken Uchibori et al. reported that the combination of Brigatinib (AP26113) and EGFR monoclonal antibodies (such as cetuximab) can overcome the problem of drug resistance to third-generation EGFR inhibitors caused by the C797S mutation. In the PC9 (EGFR-C797S/T790M/de119) mouse pharmacodynamic model, it showed good anti-tumour efficacy. However, Brigatinib, as a single drug, also faces the problem of low *in vitro* activity and no significant anti-tumour activity *in vivo,* and further clinical research has not been conducted.

Lung cancer is a major disease that threatens human health, and its mortality rate ranks first among all malignant tumours. In China, the incidence of lung cancer is increasing year by year, with about 700,000 new cases every year. The cases of lung cancer with EGFR activating mutations in China account for about 35% of all NSCLC cases. The use of first- or third-generation EGFR inhibitors can achieve good therapeutic effects, but new drug-resistant mutations will occur in the later stages. Therefore, the development of new generation of anti-drug-resistant EGFR inhibitors has huge clinical and market value.

Patent PCT/CN2023/082178 discloses a series of nitrogen-containing heterocyclic derivative inhibitors. In subsequent research and development, in order to make the product easy to handle, filter and dry, and to find a suitable crystal that is easy to store, and has long-term stability and high bioavailability, the present invention conducts a comprehensive study on the crystal form of the above-mentioned compounds.

## Summary of the Invention

All contents involved in the patent PCT/CN2023/082178 are incorporated herein by reference.

The objective of the present invention is to provide a crystal form of a compound as shown in general formula (I),

or a stereoisomer thereof, or an acid addition salt of any of the foregoing,

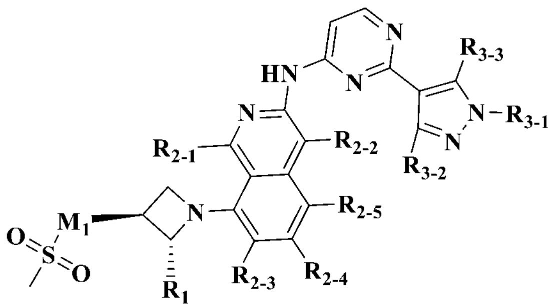

**(I)**

wherein: $M_1$ is independently selected from a bond, $NR_4$ or $CR_5R_6$;
$R_1$ is selected from hydrogen, deuterium, halogen, $C_{1-6}$ alkyl, halogenated $C_{1-6}$ alkyl or deuterated $C_{1-6}$ alkyl;
$R_{2-1}$, $R_{2-2}$, $R_{2-3}$ and $R_{2-4}$ are each independently selected from hydrogen, deuterium, halogen, $C_{1-6}$ alkyl, halogenated $C_{1-6}$ alkyl or deuterated $C_{1-6}$ alkyl;
$R_{2-5}$ is selected from amino, $C_{1-6}$ alkyl, halogenated $C_{1-6}$ alkyl or deuterated $C_{1-6}$ alkyl, and the amino, $C_{1-6}$ alkyl, halogenated $C_{1-6}$ alkyl or deuterated $C_{1-6}$ alkyl may be optionally further substituted with one or more of deuterium, halogen, cyano, hydroxyl, nitro, $C_{1-6}$ alkyl, halogenated $C_{1-6}$ alkyl and deuterated $C_{1-6}$ alkyl;
$R_{3-1}$, $R_{3-2}$ and $R_{3-3}$ are each independently selected from hydrogen, deuterium, halogen, $C_{1-6}$ alkyl, halogenated $C_{1-6}$ alkyl or deuterated $C_{1-6}$ alkyl;
$R_4$ is selected from hydrogen, deuterium, $C_{1-6}$ alkyl, halogenated $C_{1-6}$ alkyl or deuterated $C_{1-6}$ alkyl;
$R_5$ and $R_6$ are each independently selected from hydrogen, deuterium, halogen, $C_{1-6}$ alkyl, halogenated $C_{1-6}$ alkyl or deuterated $C_{1-6}$ alkyl;
the acid in the acid addition salt is selected from an inorganic acid or an organic acid, wherein the inorganic acid is selected from hydrochloric acid, sulphuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid, or L-malic acid; preferably, the acid in the acid addition salt is selected from phosphoric acid, hydrochloric acid, sulphuric acid, hydrobromic acid, citric acid, oxalic acid, maleic acid, salicylic acid or p-hydroxybenzoic acid.

In certain embodiments of the present invention,

$R_1$ is selected from hydrogen, deuterium, halogen, $C_{1-3}$ alkyl, halogenated $C_{1-3}$ alkyl or deuterated $C_{1-3}$ alkyl;
$R_{2-1}$, $R_{2-2}$, $R_{2-3}$ and $R_{2-4}$ are each independently selected from hydrogen, deuterium, halogen, $C_{1-3}$ alkyl, halogenated $C_{1-3}$ alkyl or deuterated $C_{1-3}$ alkyl;
$R_{2-5}$ is selected from amino, $C_{1-3}$ alkyl, halogenated $C_{1-3}$ alkyl or deuterated $C_{1-3}$ alkyl, and the amino, $C_{1-3}$ alkyl, halogenated $C_{1-3}$ alkyl or deuterated $C_{1-3}$ alkyl may be optionally further substituted with one or more of deuterium, halogen, cyano, hydroxyl, nitro, $C_{1-3}$ alkyl, halogenated $C_{1-3}$ alkyl and deuterated $C_{1-3}$ alkyl;
$R_{3-1}$, $R_{3-2}$ and $R_{3-3}$ are each independently selected from hydrogen, deuterium, halogen, $C_{1-3}$ alkyl, halogenated $C_{1-3}$ alkyl or deuterated $C_{1-3}$ alkyl;
$R_4$ is selected from hydrogen, deuterium, $C_{1-3}$ alkyl, halogenated $C_{1-3}$ alkyl or deuterated $C_{1-3}$ alkyl;
$R_5$ and $R_6$ are each independently selected from hydrogen, deuterium, halogen, $C_{1-3}$ alkyl, halogenated $C_{1-3}$ alkyl or deuterated $C_{1-3}$ alkyl.

In certain embodiments of the present invention, the number of the acid molecules in the acid addition salt is 1, 2 or 3.

In certain embodiments of the present invention, the acid addition salt is a hydrate or an anhydrate, preferably an anhydrate.

In certain embodiments of the present invention, the acid addition salt is an amorphous form or a crystal form.

In certain embodiments of the present invention, the crystal form is a hydrate or an anhydrate, preferably an anhydrate.

In certain embodiments of the present invention, the crystal form is crystal form A of phosphate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide,

wherein the crystal form A has an X-ray powder diffraction pattern comprising a diffraction peak at 2θ of 4.5±0.2°; a diffraction peak at 20 of 8.8+0.2°, a diffraction peak at 20 of 10.8±0.2°, a diffraction peak at 20 of 12.9±0.2°, a diffraction peak at 2θ of 14.4±0.2°, a diffraction peak at 20 of 15.3±0.2°, a diffraction peak at 20 of 16.3±0.2°, a diffraction peak at 20 of 17.1±0.2°, a diffraction peak at 20 of 17.9±0.2°, or a diffraction peak at 20 of 25.2±0.2°; preferably comprising any 2-5, 3-5, 3-6, 3-8, 5-8, 6-8, or 8-10 of the diffraction peaks; and more preferably comprising any 6, 7, 8, 9 or 10 of the diffraction peaks;

preferably, the crystal form A has an X-ray powder diffraction pattern comprising at least one or more, preferably 2, and more preferably 3 of the diffraction peaks at 20 of 4.5±0.2°, 8.8±0.2°, and 10.8±0.2°; optionally, the X-ray powder diffraction pattern can further comprise at least one, preferably 2, 3, 4 or 5 of the diffraction peaks at 2θ of 12.9±0.2°, 14.4±0.2°, 16.3±0.2°, and 17.1±0.2°;

preferably, the crystal form A has an X-ray powder diffraction pattern optionally further comprising one or more, preferably at least any 2-3 or 4-5, and further preferably any 2, 3, 4 or 5 of the diffraction peaks at 20 of 9.0±0.2°, 15.3:1:0.2°, 18.0±0.2°, 19.3±0.2°, 21.8+0.2°, 25.2±0.2°, and 27.9±0.2°;

further preferably, the crystal form A has an X-ray powder diffraction pattern comprising diffraction peaks at 20 of 4.5 ±0.2° and 8.8±0.2°; preferably, further comprising diffraction peaks at 10.8±0.2° and 17.1±0.2°; more preferably, further comprising diffraction peaks at 12.9±0.2° and 14.4±0.2°; further preferably, further comprising diffraction peaks at 15.3±0.2° and 16.3±0.2°; even further preferably, further comprising diffraction peaks at 18.0±0.2° and 25.2±0.2°;

for example, the crystal form A has an X-ray powder diffraction pattern comprising diffraction peaks at 2θ of
8.8±0.2° and 10.8±0.2°;
4.5±0.2° and 10.8±0.2';
4.5±0.2° and 8.8±0.2°;
4.5±0.2°, 8.8±0.2°, and 10.8±0.2°;
8.8±0.2°, 10.8±0.2°, and 12.9±0.2°;
4.5±0.2°, 10.8±0.2°, and 16.3±0.2°;
4.5±0.2°, 8.8+0.2°, and 17.1±0.2°;
4.5±0.2°, 8.8+0.2°, 10.8±0.2°, and 12.9±0.2°;
4.5±0.2°, 8.8+0.2°, 10.8±0.2°, and 14.4±0.2°;
8.8±0.2°, 10.8±0.2°, 12.9±0.2°, and 16.3±0.2°;
4.5±0.2°, 10.8±0.2°, 14.4±0.2°, and 16.3±0.2°;
4.5±0.2°, 8.8+0.2°, 12.9±0.2°, and 17.1±0.2°;
8.8±0.2°, 10.8±0.2°, 12.9±0.2°, 14.4±0.2°, and 17.1±0.2°;
4.5±0.2°, 10.8±0.2°, 12.9±0.2°, 14.4±0.2°, and 17.1±0.2°;
4.5±0.2°, 8.8+0.2°, 12.9±0.2°, 14.4±0.2°, and 17.1±0.2°;
4.5±0.2°, 8.8+0.2°, 10.8±0.2°, 14.4±0.2°, and 17.1±0.2°;
4.5±0.2°, 8.8±0.2°, 10.8+0.2°, 12.9±0.2°, and 17.1±0.2°;
4.5±0.2°, 8.8±0.2°, 10.8±0.2°, 12.9±0.2°, and 14.4±0.2°;
4.5±0.2°, 8.8±0.2°, 10.8±0.2°, 12.9±0.2°, 14.4±0.2°, and 17.1±0.2°;
8.8±0.2°, 10.8±0.2°, 12.9±0.2°, 14.4±0.2°, 15.3±0.2°, and 17.1±0.2°;
4.5±0.2°, 10.8±0.2°, 12.9±0.2°, 14.4±0.2°, 16.3±0.2°, and 17.1±0.2°;
4.5±0.2°, 8.8+0.2°, 12.9±0.2°, 14.4±0.2°, 15.3±0.2°, and 17.1±0.2°;
4.5±0.2°, 8.8+0.2°, 10.8±0.2°, 14.4±0.2°, 16.3±0.2°, and 17.1±0.2°;
4.5±0.2°, 8.8±0.2°, 10.8±0.2°, 12.9±0.2°, 15.3±0.2°, and 17.1±0.2°;
4.5±0.2°, 8.8±0.2°, 10.8±0.2°, 12.9±0.2°, 14.4±0.2°, and 16.3±0.2°;
8.8±0.2°, 10.8±0.2°, 12.9±0.2°, 14.4±0.2°, 15.3±0.2°, 16.3±0.2°, and 17.1±0.2°;
4.5±0.2°, 10.8±0.2°, 12.9±0.2°, 14.4±0.2°, 15.3±0.2°, 16.3±0.2°, and 17.1±0.2°;
4.5±0.2°, 8.8+0.2°, 12.9±0.2°, 14.4±0.2°, 15.3±0.2°, 16.3±0.2°, and 17.1±0.2°;

4.5±0.2°, 8.8+0.2°, 10.8±0.2°, 14.4±0.2°, 15.3±0.2°, 16.3±0.2°, and 17.1±0.2°;
4.5±0.2°, 8.8+0.2°, 10.8±0.2°, 12.9±0.2°, 15.3±0.2°, 16.3±0.2°, and 17.1±0.2°;
4.5±0.2°, 8.8+0.2°, 10.8±0.2°, 12.9±0.2°, 14.4±0.2°, 16.3±0.2°, and 17.1±0.2°;
4.5±0.2°, 8.8+0.2°, 10.8±0.2°, 12.9±0.2°, 14.4±0.2°, 15.3±0.2°, and 17.1±0.2°;
4.5±0.2°, 8.8±0.2°, 10.8±0.2°, 12.9±0.2°, 14.4±0.2°, 15.3±0.2°, and 16.3±0.2°;
4.5±0.2°, 8.8±0.2°, 10.8+0.2°, 12.9±0.2°, 14.4±0.2°, 15.3±0.2°, 16.3±0.2°, and 17.1±0.2°;
8.8±0.2°, 10.8±0.2°, 12.9±0.2°, 14.4±0.2°, 15.3±0.2°, 16.3±0.2°, 17.1±0.2°, and 18.0±0.2°;
4.5±0.2°, 10.8±0.2°, 12.9±0.2°, 14.4±0.2°, 15.3±0.2°, 16.3±0.2°, 17.1±0.2°, and 25.2±0.2°;
4.5±0.2°, 8.8±0.2°, 12.9±0.2°, 14.4±0.2°, 15.3±0.2°, 16.3±0.2°, 17.1±0.2°, and 18.0±0.2°;
4.5±0.2°, 8.8±0.2°, 10.8+0.2°, 14.4±0.2°, 15.3±0.2°, 16.3±0.2°, 17.1±0.2°, and 25.2±0.2°;
4.5±0.2°, 8.8±0.2°, 10.8±0.2°, 12.9±0.2°, 15.3±0.2°, 16.3±0.2°, 17.1±0.2°, and 18.0±0.2°;
4.5±0.2°, 8.8±0.2°, 10.8±0.2°, 12.9±0.2°, 14.4±0.2°, 16.3±0.2°, 17.1±0.2°, and 25.2±0.2°;
4.5±0.2°, 8.8±0.2°, 10.8±0.2°, 12.9±0.2°, 14.4±0.2°, 15.3±0.2°, 17.1±0.2°, and 18.0±0.2°;
4.5±0.2°, 8.8±0.2°, 10.8±0.2°, 12.9±0.2°, 14.4±0.2°, 15.3±0.2°, 16.3±0.2°, and 25.2±0.2°;
8.8±0.2°, 10.8±0.2°, 12.9±0.2°, 14.4±0.2°, 15.3±0.2°, 16.3±0.2°, 17.1±0.2°, 18.0±0.2°, and 25.2±0.2°;
4.5±0.2°, 9.0±0.2°, 10.8±0.2°, 12.9±0.2°, 14.4±0.2°, 15.3±0.2°, 16.3±0.2°, 17.1±0.2°, 18.0±0.2°, and 25.2 ±0.2°;
4.5±0.2°, 8.8±0.2°, 12.9±0.2°, 14.4±0.2°, 15.3:1:0.2°, 16.3±0.2°, 17.1±0.2°, 18.0±0.2°, 19.3±0.2°, and 25.2 ±0.2°;
4.5±0.2°, 8.8±0.2°, 10.8±0.2°, 14.4±0.2°, 15.3:1:0.2°, 16.3±0.2°, 17.1±0.2°, 18.0±0.2°, 21.8±0.2°, and 25.2 ±0.2°;
4.5±0.2°, 8.8±0.2°, 10.8±0.2°, 12.9±0.2°, 15.3:1:0.2°, 16.3±0.2°, 17.1±0.2°, 18.0±0.2°, 25.2±0.2°, and 27.9 ±0.2°;
4.5±0.2°, 8.8±0.2°, 9.0±0.2°, 10.8+0.2°, 12.9±0.2°, 14.4±0.2°, 16.3±0.2°, 17.1±0.2°, 18.0±0.2°, and 25.2±0.2°;
4.5±0.2°, 8.8±0.2°, 10.8±0.2°, 12.9±0.2°, 14.4±0.2°, 15.3±0.2°, 17.1±0.2°, 18.0±0.2°, 19.3±0.2°, and 25.2 ±0.2°;
4.5±0.2°, 8.8±0.2°, 10.8±0.2°, 12.9±0.2°, 14.4±0.2°, 15.3±0.2°, 16.3±0.2°, 18.0±0.2°, 21.8±0.2°, and 25.2 ±0.2°;
4.5±0.2°, 8.8±0.2°, 10.8+0.2°, 12.9±0.2°, 14.4±0.2°, 15.3±0.2°, 16.3±0.2°, 17.1±0.2°, 25.2±0.2°, and 27.9 ±0.2°;
4.5±0.2°, 8.8±0.2°, 10.8±0.2°, 12.9±0.2°, 14.4±0.2°, 15.3±0.2°, 16.3±0.2°, 17.1±0.2°, 18.0±0.2°, and 19.3 ±0.2°;
4.5±0.2°, 8.8±0.2°, 10.8+0.2°, 12.9±0.2°, 14.4±0.2°, 15.3±0.2°, 16.3±0.2°, 17.1±0.2°, 18.0±0.2°, and 25.2 ±0.2°;
8.8±0.2°, 9.0±0.2°, 10.8±0.2°, 12.9±0.2°, 14.4±0.2°, 15.3±0.2°, 16.3±0.2°, 17.1±0.2°, 18.0±0.2°, and 25.2 ±0.2°;
4.5±0.2°, 10.8±0.2°, 12.9±0.2°, 14.4±0.2°, 15.3:1:0.2°, 16.3±0.2°, 17.1±0.2°, 18.0±0.2°, 19.3±0.2°, and 25.2 ±0.2°;
4.5±0.2°, 8.8±0.2°, 12.9±0.2°, 14.4±0.2°, 15.3±0.2°, 16.3±0.2°, 17.1±0.2°, 18.0±0.2°, 21.8±0.2°, and 25.2 ±0.2°;
4.5±0.2°, 8.8±0.2°, 10.8±0.2°, 14.4±0.2°, 15.3:1:0.2°, 16.3±0.2°, 17.1±0.2°, 18.0±0.2°, 25.2±0.2°, and 27.9 ±0.2°;
4.5±0.2°, 8.8±0.2°, 9.0±0.2°, 10.8±0.2°, 12.9±0.2°, 15.3±0.2°, 16.3±0.2°, 17.1±0.2°, 18.0±0.2°, and 25.2±0.2°;
4.5±0.2°, 8.8±0.2°, 10.8±0.2°, 12.9±0.2°, 14.4±0.2°, 16.3±0.2°, 17.1±0.2°, 18.0±0.2°, 19.3±0.2°, and 25.2 ±0.2°;
4.5±0.2°, 8.8±0.2°, 10.8±0.2°, 12.9±0.2°, 14.4±0.2°, 15.3±0.2°, 17.1±0.2°, 18.0±0.2°, 21.8±0.2°, and 25.2 ±0.2°;
4.5±0.2°, 8.8±0.2°, 10.8±0.2°, 12.9±0.2°, 14.4±0.2°, 15.3±0.2°, 16.3±0.2°, 18.0±0.2°, 25.2±0.2°, and 27.9 ±0.2°;
4.5±0.2°, 8.8±0.2°, 9.0±0.2°, 10.8±0.2°, 12.9±0.2°, 14.4±0.2°, 15.3±0.2°, 16.3±0.2°, 17.1±0.2°, and 25.2±0.2°;
4.5±0.2°, 8.8±0.2°, 10.8±0.2°, 12.9±0.2°, 14.4±0.2°, 15.3±0.2°, 16.3±0.2°, 17.1±0.2°, 18.0±0.2°, and 19.3 ±0.2°.

In certain embodiments of the present invention, by using Cu-Kα radiation, the X-ray characteristic diffraction peaks of the crystal form A of phosphate represented by 20 angles and interplanar spacing d values are as shown in Table 1.

Table 1

| No. | XRPD data of crystal form A of phosphate | | | | | |
|---|---|---|---|---|---|---|
| | 2θ (±0.2°) | d value | Peak height | Relative intensity (I%) | Area | Relative intensity (I%) |
| 1 | 4.458 | 19.8028 | 4407 | 100 | 36018 | 100 |
| 2 | 8.785 | 10.0575 | 806 | 18.3 | 13438 | 37.3 |
| 3 | 8.964 | 9.8572 | 3101 | 70.4 | 20723 | 57.5 |
| 4 | 10.848 | 8.1486 | 601 | 13.6 | 4225 | 11.7 |
| 5 | 12.519 | 7.0645 | 71 | 1.6 | 470 | 1.3 |
| 6 | 12.935 | 6.8383 | 125 | 2.8 | 867 | 2.4 |
| 7 | 13.501 | 6.5532 | 216 | 4.9 | 1159 | 3.2 |
| 8 | 13.752 | 6.4339 | 61 | 1.4 | 806 | 2.2 |
| 9 | 14.119 | 6.2675 | 251 | 5.7 | 2541 | 7.1 |
| 10 | 14.357 | 6.1643 | 285 | 6.5 | 2411 | 6.7 |
| 11 | 15.315 | 5.7808 | 415 | 9.4 | 2649 | 7.4 |
| 12 | 15.898 | 5.5699 | 287 | 6.5 | 2334 | 6.5 |
| 13 | 16.229 | 5.4571 | 361 | 8.2 | 3327 | 9.2 |
| 14 | 17.067 | 5.191 | 498 | 11.3 | 2709 | 7.5 |
| 15 | 17.945 | 4.9388 | 2648 | 60.1 | 20835 | 57.8 |
| 16 | 18.835 | 4.7076 | 223 | 5.1 | 2341 | 6.5 |
| 17 | 19.327 | 4.5888 | 380 | 8.6 | 4516 | 12.5 |
| 18 | 20.376 | 4.3548 | 72 | 1.6 | 740 | 2.1 |
| 19 | 21.84 | 4.0661 | 280 | 6.4 | 2839 | 7.9 |
| 20 | 22.054 | 4.0272 | 104 | 2.4 | 1212 | 3.4 |
| 21 | 22.638 | 3.9246 | 190 | 4.3 | 1624 | 4.5 |
| 22 | 23.434 | 3.793 | 322 | 7.3 | 2364 | 6.6 |
| 23 | 24.551 | 3.623 | 53 | 1.2 | 269 | 0.7 |
| 24 | 25.229 | 3.5271 | 305 | 6.9 | 2538 | 7 |
| 25 | 25.64 | 3.4715 | 93 | 2.1 | 874 | 2.4 |
| 26 | 26.669 | 3.3399 | 38 | 0.9 | 321 | 0.9 |
| 27 | 26.867 | 3.3157 | 73 | 1.7 | 712 | 2 |
| 28 | 27.268 | 3.2678 | 135 | 3.1 | 1052 | 2.9 |
| 29 | 27.624 | 3.2265 | 183 | 4.2 | 2129 | 5.9 |
| 30 | 27.859 | 3.1998 | 287 | 6.5 | 2816 | 7.8 |
| 31 | 28.516 | 3.1276 | 36 | 0.8 | 292 | 0.8 |
| 32 | 28.788 | 3.0987 | 73 | 1.7 | 944 | 2.6 |
| 33 | 29.183 | 3.0576 | 70 | 1.6 | 790 | 2.2 |
| 34 | 29.58 | 3.0174 | 27 | 0.6 | 151 | 0.4 |
| 35 | 30.314 | 2.946 | 37 | 0.8 | 431 | 1.2 |
| 36 | 30.644 | 2.915 | 136 | 3.1 | 2288 | 6.4 |
| 37 | 31.031 | 2.8795 | 93 | 2.1 | 1853 | 5.1 |
| 38 | 33.072 | 2.7063 | 25 | 0.6 | 347 | 1 |

(continued)

| No. | XRPD data of crystal form A of phosphate | | | | | |
|---|---|---|---|---|---|---|
| | 2θ (±0.2°) | d value | Peak height | Relative intensity (I%) | Area | Relative intensity (I%) |
| 39 | 33.834 | 2.6471 | 45 | 1 | 391 | 1.1 |
| 40 | 35.296 | 2.5407 | 71 | 1.6 | 777 | 2.2 |
| 41 | 35.633 | 2.5175 | 42 | 1 | 586 | 1.6 |
| 42 | 36.451 | 2.4629 | 41 | 0.9 | 376 | 1 |
| 43 | 37.12 | 2.42 | 28 | 0.6 | 165 | 0.5 |
| 44 | 39.705 | 2.2682 | 71 | 1.6 | 1080 | 3 |
| 45 | 42.59 | 2.121 | 38 | 0.9 | 363 | 1 |
| 46 | 43.432 | 2.0818 | 37 | 0.8 | 304 | 0.8 |
| 47 | 43.785 | 2.0658 | 31 | 0.7 | 270 | 0.7 |
| 48 | 44.867 | 2.0185 | 31 | 0.7 | 440 | 1.2 |

In certain embodiments of the present invention, the crystal form A of phosphate has an X-ray powder diffraction pattern substantially as shown in FIG. 1; and has a DSC pattern substantially as shown in FIG. 2.

In certain embodiments of the present invention, the crystal form is crystal form B of phosphate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form B of phosphate has an X-ray powder diffraction pattern comprising diffraction peaks at 2θ (±0.2°) of 5.3±0.2°, 16.0±0.2°, 19.9±0.2°, 20.3±0.2°, 21.4±0.2° and 22.3±0.2°; preferably, the X-ray powder diffraction pattern further comprises diffraction peaks at 20 (±0.2°) of 12.5 ±0.2° and 13.0±0.2°; more preferably, the X-ray powder diffraction pattern further comprises diffraction peaks at 20 (±0.2') of 14.1+0.2° and 24.7±0.2°.

In certain embodiments of the present invention, by using Cu-Kα radiation, the X-ray characteristic diffraction peaks of the crystal form B of phosphate represented by 2θ angles and interplanar spacing d values are as shown in Table 2.

Table 2

| No. | XRPD data of crystal form B of phosphate | | | | | |
|---|---|---|---|---|---|---|
| | 2θ (±0.2°) | d value | Peak height | Relative intensity (I%) | Area | Relative intensity (I%) |
| 1 | 5.339 | 16.5385 | 1291 | 59.8 | 7059 | 56.8 |
| 2 | 8.406 | 10.5101 | 79 | 3.7 | 341 | 2.7 |
| 3 | 12.491 | 7.0803 | 788 | 36.5 | 4928 | 39.7 |
| 4 | 13.04 | 6.7837 | 814 | 37.7 | 5043 | 40.6 |
| 5 | 14.079 | 6.2854 | 539 | 25 | 3249 | 26.1 |
| 6 | 14.688 | 6.0261 | 38 | 1.8 | 96 | 0.8 |
| 7 | 15.984 | 5.5401 | 2159 | 100 | 12427 | 100 |
| 8 | 16.322 | 5.4263 | 160 | 7.4 | 1317 | 10.6 |
| 9 | 16.856 | 5.2554 | 94 | 4.4 | 1069 | 8.6 |
| 10 | 17.277 | 5.1283 | 191 | 8.8 | 938 | 7.5 |
| 11 | 18.188 | 4.8736 | 74 | 3.4 | 480 | 3.9 |
| 12 | 19.874 | 4.4637 | 688 | 31.9 | 5182 | 41.7 |
| 13 | 20.258 | 4.3799 | 707 | 32.7 | 5880 | 47.3 |
| 14 | 20.927 | 4.2413 | 110 | 5.1 | 472 | 3.8 |
| 15 | 21.369 | 4.1547 | 707 | 32.7 | 5180 | 41.7 |

(continued)

| No. | XRPD data of crystal form B of phosphate | | | | | |
|---|---|---|---|---|---|---|
| | 2θ (±0.2°) | d value | Peak height | Relative intensity (I%) | Area | Relative intensity (I%) |
| 16 | 22.332 | 3.9777 | 775 | 35.9 | 5236 | 42.1 |
| 17 | 23.378 | 3.802 | 244 | 11.3 | 1507 | 12.1 |
| 18 | 24.133 | 3.6848 | 364 | 16.9 | 2080 | 16.7 |
| 19 | 24.719 | 3.5987 | 435 | 20.1 | 4525 | 36.4 |
| 20 | 25.17 | 3.5352 | 132 | 6.1 | 1234 | 9.9 |
| 21 | 26.036 | 3.4196 | 34 | 1.6 | 285 | 2.3 |
| 22 | 26.749 | 3.33 | 204 | 9.4 | 1800 | 14.5 |
| 23 | 27.581 | 3.2314 | 373 | 17.3 | 3235 | 26 |
| 24 | 28.291 | 3.1519 | 113 | 5.2 | 1006 | 8.1 |
| 25 | 28.817 | 3.0956 | 41 | 1.9 | 471 | 3.8 |
| 26 | 29.12 | 3.064 | 94 | 4.4 | 899 | 7.2 |
| 27 | 29.872 | 2.9886 | 43 | 2 | 156 | 1.3 |
| 28 | 30.191 | 2.9577 | 124 | 5.7 | 3188 | 25.7 |
| 29 | 30.541 | 2.9247 | 47 | 2.2 | 1349 | 10.9 |
| 30 | 30.859 | 2.8952 | 34 | 1.6 | 542 | 4.4 |
| 31 | 31.367 | 2.8495 | 45 | 2.1 | 405 | 3.3 |
| 32 | 32.304 | 2.7689 | 27 | 1.3 | 243 | 2 |
| 33 | 32.967 | 2.7148 | 36 | 1.7 | 373 | 3 |
| 34 | 33.395 | 2.6809 | 57 | 2.6 | 1058 | 8.5 |
| 35 | 33.68 | 2.6589 | 40 | 1.9 | 459 | 3.7 |
| 36 | 34.314 | 2.6112 | 126 | 5.8 | 1192 | 9.6 |
| 37 | 34.919 | 2.5674 | 90 | 4.2 | 1057 | 8.5 |
| 38 | 36.218 | 2.4782 | 86 | 4 | 746 | 6 |
| 39 | 36.787 | 2.4412 | 57 | 2.6 | 383 | 3.1 |
| 40 | 37.458 | 2.399 | 29 | 1.3 | 192 | 1.5 |
| 41 | 37.816 | 2.3771 | 61 | 2.8 | 1081 | 8.7 |
| 42 | 38.024 | 2.3645 | 109 | 5 | 1634 | 13.1 |
| 42 | 39.337 | 2.2885 | 49 | 2.3 | 327 | 2.6 |

In certain embodiments of the present invention, the crystal form B of phosphate has an X-ray powder diffraction pattern substantially as shown in FIG. 3; and has a DSC pattern substantially as shown in FIG. 4.

In certain embodiments of the present invention, the crystal form is crystal form A of hydrochloride of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of hydrochloride has an X-ray powder diffraction pattern comprising diffraction peaks at 20 (±0.2°) of 6.4±0.2°, 7.1±0.2°, 14.2±0.2°, 19.3±0.2°, 24.6±0.2° and 25.1±0.2°; preferably, the X-ray powder diffraction pattern further comprises diffraction peaks at 20 (±0.2°) of 9.3±0.2° and 12.7±0.2°; more preferably, the X-ray powder diffraction pattern further comprises diffraction peaks at 2θ (±0.2°) of 11.2±0.2° and 15.5±0.2°.

In certain embodiments of the present invention, by using Cu-Kα radiation, the X-ray characteristic diffraction peaks of the crystal form A of hydrochloride represented by 20 angles and interplanar spacing d values are as shown in Table 3.

Table 3

| No. | XRPD data of crystal form A of hydrochloride | | | | | |
|---|---|---|---|---|---|---|
| | 2θ (±0.2°) | d value | Peak height | Relative intensity (I%) | Area | Relative intensity (I%) |
| 1 | 6.388 | 13.8244 | 586 | 95.1 | 4749 | 89.9 |
| 2 | 7.14 | 12.3697 | 616 | 100 | 5285 | 100 |
| 3 | 8.827 | 10.01 | 139 | 22.6 | 1185 | 22.4 |
| 4 | 9.31 | 9.4914 | 207 | 33.6 | 1598 | 30.2 |
| 5 | 11.191 | 7.8998 | 142 | 23.1 | 1326 | 25.1 |
| 6 | 11.458 | 7.7166 | 39 | 6.3 | 274 | 5.2 |
| 7 | 12.029 | 7.3512 | 59 | 9.6 | 738 | 14 |
| 8 | 12.273 | 7.2057 | 79 | 12.8 | 618 | 11.7 |
| 9 | 12.678 | 6.9763 | 211 | 34.3 | 1840 | 34.8 |
| 10 | 13.084 | 6.7609 | 83 | 13.5 | 1314 | 24.9 |
| 11 | 14.239 | 6.2152 | 303 | 49.2 | 4541 | 85.9 |
| 12 | 15.453 | 5.7293 | 133 | 21.6 | 1352 | 25.6 |
| 13 | 16.53 | 5.3585 | 59 | 9.6 | 812 | 15.4 |
| 14 | 16.865 | 5.2526 | 69 | 11.2 | 812 | 15.4 |
| 15 | 17.661 | 5.0177 | 75 | 12.2 | 494 | 9.3 |
| 16 | 18.27 | 4.8517 | 58 | 9.4 | 823 | 15.6 |
| 17 | 19.268 | 4.6027 | 241 | 39.1 | 4604 | 87.1 |
| 18 | 19.916 | 4.4543 | 72 | 11.7 | 1149 | 21.7 |
| 19 | 21.27 | 4.1738 | 52 | 8.4 | 670 | 12.7 |
| 20 | 21.796 | 4.0742 | 94 | 15.3 | 923 | 17.5 |
| 21 | 22.131 | 4.0132 | 94 | 15.3 | 555 | 10.5 |
| 22 | 23.283 | 3.8172 | 42 | 6.8 | 956 | 18.1 |
| 23 | 24.195 | 3.6754 | 49 | 8 | 300 | 5.7 |
| 24 | 24.558 | 3.622 | 84 | 13.6 | 2505 | 47.4 |
| 25 | 25.064 | 3.55 | 88 | 14.3 | 2164 | 40.9 |
| 26 | 25.673 | 3.467 | 73 | 11.9 | 1296 | 24.5 |
| 27 | 26.686 | 3.3377 | 42 | 6.8 | 754 | 14.3 |
| 28 | 27.456 | 3.2458 | 93 | 15.1 | 1080 | 20.4 |
| 29 | 28.534 | 3.1256 | 37 | 6 | 903 | 17.1 |

In certain embodiments of the present invention, the crystal form A of hydrochloride has an X-ray powder diffraction pattern substantially as shown in FIG. 5; and has a DSC pattern substantially as shown in FIG. 6.

In certain embodiments of the present invention, the crystal form is crystal form B of hydrochloride of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form B of hydrochloride has an X-ray powder diffraction pattern comprising diffraction peaks at 20 (±0.2°) of 9.6±0.2°, 15.8±0.2°, 16.9±0.2°, 25.7±0.2°, 27.5±0.2° and 27.9±0.2°; preferably, the X-ray powder diffraction pattern further comprises diffraction peaks at 2θ (±0.2°) of 15.1 ±0.2° and 23.4±0.2°; more preferably, the X-ray powder diffraction pattern further comprises diffraction peaks at 2θ (±0.2°) of 19.1±0.2° and 26.2±0.2°.

In certain embodiments of the present invention, by using Cu-Kα radiation, the X-ray characteristic diffraction peaks of the crystal form B of hydrochloride represented by 20 angles and interplanar spacing d values are as shown in Table 4.

Table 4

| No. | XRPD data of crystal form B of hydrochloride | | | | | |
|---|---|---|---|---|---|---|
| | 2θ (±0.2°) | d value | Peak height | Relative intensity (I%) | Area | Relative intensity (I%) |
| 1 | 8.014 | 11.0237 | 162 | 21.8 | 1917 | 16.9 |
| 2 | 9.574 | 9.2299 | 619 | 83.2 | 7338 | 64.5 |
| 3 | 10.128 | 8.7265 | 129 | 17.3 | 1198 | 10.5 |
| 4 | 11.096 | 7.9674 | 88 | 11.8 | 978 | 8.6 |
| 5 | 11.928 | 7.4135 | 133 | 17.9 | 1501 | 13.2 |
| 6 | 12.234 | 7.2287 | 71 | 9.5 | 602 | 5.3 |
| 7 | 13.69 | 6.4631 | 107 | 14.4 | 1374 | 12.1 |
| 8 | 14.213 | 6.2262 | 75 | 10.1 | 529 | 4.7 |
| 9 | 14.847 | 5.9619 | 198 | 26.6 | 3002 | 26.4 |
| 10 | 15.149 | 5.8438 | 298 | 40.1 | 3881 | 34.1 |
| 11 | 15.756 | 5.6197 | 415 | 55.8 | 4768 | 41.9 |
| 12 | 16.892 | 5.2445 | 505 | 67.9 | 6840 | 60.1 |
| 13 | 18.091 | 4.8993 | 68 | 9.1 | 843 | 7.4 |
| 14 | 18.435 | 4.8087 | 61 | 8.2 | 1056 | 9.3 |
| 15 | 19.061 | 4.6523 | 230 | 30.9 | 3296 | 29 |
| 16 | 19.429 | 4.5649 | 87 | 11.7 | 1040 | 9.1 |
| 17 | 19.851 | 4.4687 | 70 | 9.4 | 424 | 3.7 |
| 18 | 20.313 | 4.3682 | 96 | 12.9 | 1157 | 10.2 |
| 19 | 20.906 | 4.2455 | 111 | 14.9 | 2448 | 21.5 |
| 20 | 21.492 | 4.1312 | 34 | 4.6 | 168 | 1.5 |
| 21 | 22.15 | 4.0099 | 73 | 9.8 | 2095 | 18.4 |
| 22 | 22.43 | 3.9604 | 119 | 16 | 2297 | 20.2 |
| 23 | 23.401 | 3.7983 | 149 | 20 | 3833 | 33.7 |
| 24 | 23.806 | 3.7346 | 86 | 11.6 | 2251 | 19.8 |
| 25 | 24.724 | 3.598 | 85 | 11.4 | 1038 | 9.1 |
| 26 | 25.712 | 3.462 | 397 | 53.4 | 7145 | 62.8 |
| 27 | 26.199 | 3.3987 | 98 | 13.2 | 3553 | 31.2 |
| 28 | 26.646 | 3.3427 | 50 | 6.7 | 454 | 4 |
| 29 | 27.457 | 3.2458 | 744 | 100 | 11372 | 100 |
| 30 | 27.921 | 3.1928 | 124 | 16.7 | 4374 | 38.5 |
| 31 | 28.391 | 3.141 | 136 | 18.3 | 1286 | 11.3 |
| 32 | 29.362 | 3.0393 | 117 | 15.7 | 2252 | 19.8 |
| 33 | 30.679 | 2.9118 | 70 | 9.4 | 570 | 5 |
| 34 | 31.387 | 2.8477 | 55 | 7.4 | 373 | 3.3 |
| 35 | 32.115 | 2.7848 | 98 | 13.2 | 1978 | 17.4 |
| 36 | 32.362 | 2.7641 | 141 | 19 | 2614 | 23 |
| 37 | 33.373 | 2.6826 | 60 | 8.1 | 1241 | 10.9 |
| 38 | 34.616 | 2.5891 | 41 | 5.5 | 708 | 6.2 |

(continued)

| No. | XRPD data of crystal form B of hydrochloride | | | | | |
|---|---|---|---|---|---|---|
| | 2θ (±0.2°) | d value | Peak height | Relative intensity (I%) | Area | Relative intensity (I%) |
| 39 | 35.626 | 2.518 | 49 | 6.6 | 694 | 6.1 |
| 40 | 36.658 | 2.4494 | 65 | 8.7 | 610 | 5.4 |
| 41 | 37.444 | 2.3998 | 41 | 5.5 | 762 | 6.7 |
| 42 | 38.548 | 2.3336 | 36 | 4.8 | 596 | 5.2 |

In certain embodiments of the present invention, the crystal form B of hydrochloride has an X-ray powder diffraction pattern substantially as shown in FIG. 7; and has a DSC pattern substantially as shown in FIG. 8.

In certain embodiments of the present invention, the crystal form is crystal form A of sulphate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of sulphate has an X-ray powder diffraction pattern comprising diffraction peaks at 20 (±0.2°) of 5.5±0.2°, 6.1±0.2°, 6.3±0.2°, 16.1±0.2°, 26.1±0.2° and 26.8±0.2°; preferably, the X-ray powder diffraction pattern further comprises diffraction peaks at 20 (±0.2°) of 15.7 ±0.2° and 22.9±0.2°; more preferably, the X-ray powder diffraction pattern further comprises diffraction peaks at 2θ (±0.2°) of 14.1+0.2° and 22.0±0.2°.

In certain embodiments of the present invention, by using Cu-Kα radiation, the X-ray characteristic diffraction peaks of the crystal form A of sulphate represented by 2θ angles and interplanar spacing d values are as shown in Table 5.

Table 5

| No. | XRPD data of crystal form A of sulphate | | | | | |
|---|---|---|---|---|---|---|
| | 2θ (±0.2°) | d value | Peak height | Relative intensity (I%) | Area | Relative intensity (I%) |
| 1 | 5.459 | 16.1741 | 1216 | 81.2 | 9992 | 60.2 |
| 2 | 6.068 | 14.5522 | 596 | 39.8 | 16603 | 100 |
| 3 | 6.292 | 14.0366 | 1497 | 100 | 14815 | 89.2 |
| 4 | 7.913 | 11.1637 | 110 | 7.3 | 818 | 4.9 |
| 5 | 9.358 | 9.4433 | 194 | 13 | 1761 | 10.6 |
| 6 | 10.972 | 8.0567 | 309 | 20.6 | 4529 | 27.3 |
| 7 | 11.48 | 7.7019 | 39 | 2.6 | 122 | 0.7 |
| 8 | 13.38 | 6.6119 | 57 | 3.8 | 308 | 1.9 |
| 9 | 14.113 | 6.2702 | 422 | 28.2 | 5215 | 31.4 |
| 10 | 14.46 | 6.1206 | 182 | 12.2 | 1789 | 10.8 |
| 11 | 15.164 | 5.8377 | 41 | 2.7 | 211 | 1.3 |
| 12 | 15.677 | 5.6481 | 250 | 16.7 | 6485 | 39.1 |
| 13 | 16.144 | 5.4855 | 485 | 32.4 | 7293 | 43.9 |
| 14 | 16.695 | 5.306 | 120 | 8 | 5016 | 30.2 |
| 15 | 17.137 | 5.1701 | 289 | 19.3 | 5040 | 30.4 |
| 16 | 17.888 | 4.9546 | 180 | 12 | 2761 | 16.6 |
| 17 | 18.314 | 4.8402 | 159 | 10.6 | 2755 | 16.6 |
| 18 | 18.903 | 4.6908 | 124 | 8.3 | 1689 | 10.2 |
| 19 | 19.751 | 4.4912 | 122 | 8.1 | 1201 | 7.2 |
| 20 | 20.688 | 4.2899 | 124 | 8.3 | 1635 | 9.8 |
| 21 | 21.233 | 4.181 | 169 | 11.3 | 2203 | 13.3 |
| 22 | 22.023 | 4.0327 | 307 | 20.5 | 5363 | 32.3 |

(continued)

| No. | XRPD data of crystal form A of sulphate | | | | | |
|---|---|---|---|---|---|---|
| | 2θ (±0.2°) | d value | Peak height | Relative intensity (I%) | Area | Relative intensity (I%) |
| 23 | 22.338 | 3.9765 | 73 | 4.9 | 750 | 4.5 |
| 24 | 22.936 | 3.8743 | 335 | 22.4 | 5543 | 33.4 |
| 25 | 23.624 | 3.7629 | 239 | 16 | 5007 | 30.2 |
| 26 | 24.983 | 3.5613 | 229 | 15.3 | 3990 | 24 |
| 27 | 26.117 | 3.4091 | 461 | 30.8 | 6997 | 42.1 |
| 28 | 26.829 | 3.3203 | 478 | 31.9 | 6633 | 40 |
| 29 | 29.056 | 3.0706 | 74 | 4.9 | 1058 | 6.4 |
| 30 | 30.867 | 2.8944 | 35 | 2.3 | 598 | 3.6 |
| 31 | 31.229 | 2.8617 | 78 | 5.2 | 940 | 5.7 |
| 32 | 33.68 | 2.6589 | 86 | 5.7 | 1276 | 7.7 |
| 33 | 34.268 | 2.6146 | 37 | 2.5 | 319 | 1.9 |
| 34 | 37.915 | 2.371 | 48 | 3.2 | 455 | 2.7 |

In certain embodiments of the present invention, the crystal form A of sulphate has an X-ray powder diffraction pattern substantially as shown in FIG. 9; and has a DSC pattern substantially as shown in FIG. 10.

In certain embodiments of the present invention, the crystal form is crystal form A of hydrobromide of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of hydrobromide has an X-ray powder diffraction pattern comprising diffraction peaks at 2θ (±0.2°) of 7.1±0.2°, 14.3±0.2°, 21.6±0.2°, 22.1±0.2°, 25.1±0.2° and 27.0±0.2°; preferably, the X-ray powder diffraction pattern further comprises diffraction peaks at 2θ (±0.2°) of 11.3 ±0.2° and 21.2±0.2°; more preferably, the X-ray powder diffraction pattern further comprises diffraction peaks at 2θ (±0.2°) of 26.7±0.2° and 30.5±0.2°.

In certain embodiments of the present invention, by using Cu-Kα radiation, the X-ray characteristic diffraction peaks of the crystal form A of hydrobromide represented by 2θ angles and interplanar spacing d values are as shown in Table 6.

Table 6

| No. | XRPD data of crystal form A of hydrobromide | | | | | |
|---|---|---|---|---|---|---|
| | 2θ (±0.2°) | d value | Peak height | Relative intensity (I%) | Area | Relative intensity (I%) |
| 1 | 5.969 | 14.7953 | 148 | 49.7 | 1348 | 27.8 |
| 2 | 6.473 | 13.6433 | 73 | 24.5 | 489 | 10.1 |
| 3 | 7.143 | 12.365 | 298 | 100 | 2194 | 45.2 |
| 4 | 9.271 | 9.531 | 146 | 49 | 1103 | 22.7 |
| 5 | 11.295 | 7.8271 | 120 | 40.3 | 1985 | 40.9 |
| 6 | 11.827 | 7.4762 | 47 | 15.8 | 969 | 20 |
| 7 | 12.326 | 7.1751 | 38 | 12.8 | 271 | 5.6 |
| 8 | 12.912 | 6.8503 | 56 | 18.8 | 881 | 18.1 |
| 9 | 13.382 | 6.6111 | 48 | 16.1 | 656 | 13.5 |
| 10 | 14.256 | 6.2074 | 166 | 55.7 | 2039 | 42 |
| 11 | 15.415 | 5.7434 | 192 | 64.4 | 1600 | 33 |
| 12 | 16.5 | 5.3682 | 67 | 22.5 | 573 | 11.8 |
| 13 | 17.62 | 5.0293 | 161 | 54 | 1380 | 28.4 |

(continued)

| No. | XRPD data of crystal form A of hydrobromide | | | | | |
|---|---|---|---|---|---|---|
| | 2θ (±0.2°) | d value | Peak height | Relative intensity (I%) | Area | Relative intensity (I%) |
| 14 | 18.149 | 4.8839 | 91 | 30.5 | 1399 | 28.8 |
| 15 | 18.552 | 4.7788 | 69 | 23.2 | 882 | 18.2 |
| 16 | 19.206 | 4.6174 | 101 | 33.9 | 1148 | 23.6 |
| 17 | 19.941 | 4.4488 | 59 | 19.8 | 564 | 11.6 |
| 18 | 20.581 | 4.3119 | 47 | 15.8 | 381 | 7.8 |
| 19 | 21.209 | 4.1858 | 77 | 25.8 | 1963 | 40.4 |
| 20 | 21.555 | 4.1192 | 99 | 33.2 | 3162 | 65.1 |
| 21 | 22.063 | 4.0255 | 260 | 87.2 | 4855 | 100 |
| 22 | 23.167 | 3.8362 | 88 | 29.5 | 1405 | 28.9 |
| 23 | 23.827 | 3.7313 | 63 | 21.1 | 758 | 15.6 |
| 24 | 24.434 | 3.64 | 56 | 18.8 | 1208 | 24.9 |
| 25 | 25.083 | 3.5473 | 110 | 36.9 | 2278 | 46.9 |
| 26 | 25.892 | 3.4382 | 74 | 24.8 | 1205 | 24.8 |
| 27 | 26.681 | 3.3383 | 88 | 29.5 | 1870 | 38.5 |
| 28 | 27.033 | 3.2956 | 62 | 20.8 | 2071 | 42.7 |
| 29 | 27.515 | 3.239 | 79 | 26.5 | 864 | 17.8 |
| 30 | 28.392 | 3.1409 | 40 | 13.4 | 1538 | 31.7 |
| 31 | 28.673 | 3.1108 | 47 | 15.8 | 1540 | 31.7 |
| 32 | 29.164 | 3.0596 | 45 | 15.1 | 340 | 7 |
| 33 | 29.722 | 3.0033 | 43 | 14.4 | 509 | 10.5 |
| 34 | 29.91 | 2.9849 | 56 | 18.8 | 1380 | 28.4 |
| 35 | 30.476 | 2.9307 | 49 | 16.4 | 1746 | 36 |
| 36 | 35.607 | 2.5193 | 39 | 13.1 | 473 | 9.7 |

In certain embodiments of the present invention, the crystal form A of hydrobromide has an X-ray powder diffraction pattern substantially as shown in FIG. 11; and has a DSC pattern substantially as shown in FIG. 12.

In certain embodiments of the present invention, the crystal form is crystal form B of hydrobromide of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form B of hydrobromide has an X-ray powder diffraction pattern comprising diffraction peaks at 2θ (±0.2°) of 6.9±0.2°, 18.5±0.2°, 19.4±0.2°, 22.8±0.2°, 24.9±0.2° and 25.3±0.2°; preferably, the X-ray powder diffraction pattern further comprises diffraction peaks at 2θ (±0.2°) of 7.1 ±0.2° and 19.7±0.2°; more preferably, the X-ray powder diffraction pattern further comprises diffraction peaks at 2θ (±0.2°) of 15.3±0.2° and 22.6±0.2°.

In certain embodiments of the present invention, by using Cu-Kα radiation, the X-ray characteristic diffraction peaks of the crystal form B of hydrobromide represented by 2θ angles and interplanar spacing d values are as shown in Table 7.

Table 7

| No. | XRPD data of crystal form B of hydrobromide | | | | | |
|---|---|---|---|---|---|---|
| | 2θ (±0.2°) | d value | Peak height | Relative intensity (I%) | Area | Relative intensity (I%) |
| 1 | 5.54 | 15.9377 | 366 | 53.8 | 3007 | 20.5 |
| 2 | 6.879 | 12.8384 | 272 | 40 | 8857 | 60.3 |

(continued)

| No. | XRPD data of crystal form B of hydrobromide | | | | | |
|---|---|---|---|---|---|---|
| | 2θ (±0.2°) | d value | Peak height | Relative intensity (I%) | Area | Relative intensity (I%) |
| 3 | 7.079 | 12.4761 | 450 | 66.2 | 5287 | 36 |
| 4 | 8.461 | 10.4419 | 123 | 18.1 | 1241 | 8.4 |
| 5 | 9.368 | 9.4325 | 33 | 4.9 | 185 | 1.3 |
| 6 | 10.531 | 8.3933 | 35 | 5.1 | 314 | 2.1 |
| 7 | 11.172 | 7.9131 | 121 | 17.8 | 1726 | 11.7 |
| 8 | 12.576 | 7.033 | 301 | 44.3 | 3193 | 21.7 |
| 9 | 13.574 | 6.518 | 46 | 6.8 | 319 | 2.2 |
| 10 | 14.664 | 6.0359 | 140 | 20.6 | 2874 | 19.6 |
| 11 | 15.331 | 5.7745 | 297 | 43.7 | 4988 | 33.9 |
| 12 | 15.841 | 5.5897 | 100 | 14.7 | 555 | 3.8 |
| 13 | 16.836 | 5.2617 | 150 | 22.1 | 1692 | 11.5 |
| 14 | 17.145 | 5.1676 | 50 | 7.4 | 909 | 6.2 |
| 15 | 17.575 | 5.0422 | 45 | 6.6 | 477 | 3.2 |
| 16 | 18.251 | 4.8569 | 261 | 38.4 | 3104 | 21.1 |
| 17 | 18.497 | 4.7928 | 348 | 51.2 | 5732 | 39 |
| 18 | 19.408 | 4.5697 | 354 | 52.1 | 5903 | 40.2 |
| 19 | 19.688 | 4.5054 | 296 | 43.5 | 5012 | 34.1 |
| 20 | 20.325 | 4.3656 | 153 | 22.5 | 1629 | 11.1 |
| 21 | 20.854 | 4.256 | 233 | 34.3 | 4537 | 30.9 |
| 22 | 21.171 | 4.1931 | 178 | 26.2 | 2840 | 19.3 |
| 23 | 21.819 | 4.0701 | 138 | 20.3 | 1092 | 7.4 |
| 24 | 22.308 | 3.9819 | 65 | 9.6 | 533 | 3.6 |
| 25 | 22.57 | 3.9362 | 178 | 26.2 | 4592 | 31.2 |
| 26 | 22.795 | 3.8979 | 217 | 31.9 | 5312 | 36.1 |
| 27 | 23.256 | 3.8216 | 78 | 11.5 | 716 | 4.9 |
| 28 | 23.76 | 3.7417 | 52 | 7.6 | 477 | 3.2 |
| 29 | 24.861 | 3.5784 | 283 | 41.6 | 5976 | 40.7 |
| 30 | 25.288 | 3.519 | 680 | 100 | 14697 | 100 |
| 31 | 25.78 | 3.4529 | 94 | 13.8 | 399 | 2.7 |
| 32 | 26.545 | 3.3552 | 59 | 8.7 | 340 | 2.3 |
| 33 | 26.807 | 3.3229 | 262 | 38.5 | 4081 | 27.8 |
| 34 | 27.336 | 3.2598 | 191 | 28.1 | 3965 | 27 |
| 35 | 28.161 | 3.1661 | 85 | 12.5 | 1139 | 7.7 |
| 36 | 28.831 | 3.0941 | 52 | 7.6 | 678 | 4.6 |
| 37 | 29.506 | 3.0248 | 116 | 17.1 | 1587 | 10.8 |
| 38 | 30.476 | 2.9307 | 47 | 6.9 | 1684 | 11.5 |
| 39 | 30.94 | 2.8878 | 55 | 8.1 | 1675 | 11.4 |
| 40 | 31.326 | 2.8531 | 51 | 7.5 | 1683 | 11.5 |

(continued)

| No. | XRPD data of crystal form B of hydrobromide | | | | | |
|---|---|---|---|---|---|---|
| | 2θ (±0.2°) | d value | Peak height | Relative intensity (I%) | Area | Relative intensity (I%) |
| 41 | 31.631 | 2.8263 | 40 | 5.9 | 82 | 0.6 |
| 42 | 32.848 | 2.7243 | 71 | 10.4 | 884 | 6 |
| 43 | 33.48 | 2.6743 | 50 | 7.4 | 721 | 4.9 |
| 44 | 34.25 | 2.6159 | 51 | 7.5 | 578 | 3.9 |
| 45 | 34.879 | 2.5702 | 52 | 7.6 | 993 | 6.8 |
| 46 | 36.213 | 2.4785 | 44 | 6.5 | 404 | 2.7 |
| 47 | 37.123 | 2.4198 | 37 | 5.4 | 292 | 2 |
| 48 | 37.778 | 2.3793 | 42 | 6.2 | 398 | 2.7 |

In certain embodiments of the present invention, the crystal form B of hydrobromide has an X-ray powder diffraction pattern substantially as shown in FIG. 13; and has a DSC pattern substantially as shown in FIG. 14.

In certain embodiments of the present invention, the crystal form is crystal form A of citrate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of citrate has an X-ray powder diffraction pattern comprising diffraction peaks at 2θ (±0.2°) of 12.2±0.2°, 16.7±0.2°, 18.7+0.2°, 19.4±0.2°, 21.9±0.2° and 22.3 ±0.2°; preferably, the X-ray powder diffraction pattern further comprises diffraction peaks at 2θ (±0.2°) of 16.2±0.2° and 23.5±0.2°; more preferably, the X-ray powder diffraction pattern further comprises diffraction peaks at 2θ (±0.2°) of 11.7 ±0.2° and 15.6±0.2°.

In certain embodiments of the present invention, by using Cu-Kα radiation, the X-ray characteristic diffraction peaks of the crystal form A of citrate represented by 2θ angles and interplanar spacing d values are as shown in Table 8.

Table 8

| No. | XRPD data of crystal form A of citrate | | | | | |
|---|---|---|---|---|---|---|
| | 2θ (±0.2°) | d value | Peak height | Relative intensity (I%) | Area | Relative intensity (I%) |
| 1 | 6.131 | 14.403 | 129 | 17 | 2066 | 17.6 |
| 2 | 8.175 | 10.8058 | 73 | 9.6 | 779 | 6.6 |
| 3 | 8.682 | 10.1768 | 94 | 12.4 | 1065 | 9.1 |
| 4 | 11.684 | 7.5676 | 218 | 28.7 | 4770 | 40.6 |
| 5 | 12.189 | 7.255 | 340 | 44.8 | 6360 | 54.1 |
| 6 | 13.874 | 6.3779 | 297 | 39.1 | 3710 | 31.6 |
| 7 | 14.541 | 6.0865 | 99 | 13 | 941 | 8 |
| 8 | 15.557 | 5.6914 | 437 | 57.6 | 4485 | 38.2 |
| 9 | 16.183 | 5.4724 | 206 | 27.1 | 4918 | 41.9 |
| 10 | 16.652 | 5.3194 | 356 | 46.9 | 7708 | 65.6 |
| 11 | 17.685 | 5.011 | 114 | 15 | 3714 | 31.6 |
| 12 | 18.171 | 4.878 | 79 | 10.4 | 2002 | 17 |
| 13 | 18.739 | 4.7314 | 452 | 59.6 | 7182 | 61.1 |
| 14 | 19.369 | 4.5789 | 376 | 49.5 | 7036 | 59.9 |
| 15 | 20.073 | 4.42 | 166 | 21.9 | 1623 | 13.8 |
| 16 | 20.503 | 4.3282 | 106 | 14 | 1917 | 16.3 |
| 17 | 20.825 | 4.2621 | 107 | 14.1 | 1575 | 13.4 |
| 18 | 21.921 | 4.0512 | 759 | 100 | 11749 | 100 |

(continued)

| No. | XRPD data of crystal form A of citrate | | | | | |
|---|---|---|---|---|---|---|
| | 2θ (±0.2°) | d value | Peak height | Relative intensity (I%) | Area | Relative intensity (I%) |
| 19 | 22.264 | 3.9896 | 250 | 32.9 | 7969 | 67.8 |
| 20 | 22.815 | 3.8944 | 42 | 5.5 | 220 | 1.9 |
| 21 | 23.482 | 3.7854 | 152 | 20 | 5162 | 43.9 |
| 22 | 23.867 | 3.7252 | 121 | 15.9 | 4112 | 35 |
| 23 | 25.145 | 3.5387 | 129 | 17 | 1268 | 10.8 |
| 24 | 25.611 | 3.4754 | 104 | 13.7 | 1036 | 8.8 |
| 25 | 26.299 | 3.3859 | 180 | 23.7 | 2872 | 24.4 |
| 26 | 27.071 | 3.2912 | 87 | 11.5 | 1694 | 14.4 |
| 27 | 27.462 | 3.2451 | 83 | 10.9 | 498 | 4.2 |
| 28 | 28.688 | 3.1092 | 39 | 5.1 | 626 | 5.3 |
| 29 | 29.054 | 3.0708 | 50 | 6.6 | 606 | 5.2 |
| 30 | 29.63 | 3.0125 | 51 | 6.7 | 1044 | 8.9 |
| 31 | 30.003 | 2.9758 | 53 | 7 | 1050 | 8.9 |
| 32 | 30.66 | 2.9136 | 69 | 9.1 | 1228 | 10.5 |
| 33 | 31.164 | 2.8676 | 104 | 13.7 | 3282 | 27.9 |
| 34 | 33.306 | 2.6879 | 44 | 5.8 | 387 | 3.3 |
| 35 | 35.913 | 2.4985 | 45 | 5.9 | 1161 | 9.9 |
| 36 | 36.412 | 2.4654 | 49 | 6.5 | 700 | 6 |
| 37 | 37.45 | 2.3995 | 107 | 14.1 | 2608 | 22.2 |
| 38 | 38.938 | 2.3111 | 41 | 5.4 | 690 | 5.9 |

In certain embodiments of the present invention, the crystal form A of citrate has an X-ray powder diffraction pattern substantially as shown in FIG. 15; and has a DSC pattern substantially as shown in FIG. 16.

In certain embodiments of the present invention, the crystal form is crystal form A of oxalate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of oxalate has an X-ray powder diffraction pattern comprising diffraction peaks at 2θ (±0.2°) of 4.3±0.2°, 17.5±0.2°, 17.8±0.2°, 18.2±0.2°, 20.7±0.2° and 25.8 ±0.2°; preferably, the X-ray powder diffraction pattern further comprises diffraction peaks at 2θ (±0.2°) of 8.4±0.2° and 16.8±0.2°; more preferably, the X-ray powder diffraction pattern further comprises diffraction peaks at 2θ (±0.2°) of 21.0 ±0.2° and 27.4±0.2°.

In certain embodiments of the present invention, by using Cu-Kα radiation, the X-ray characteristic diffraction peaks of the crystal form A of oxalate represented by 2θ angles and interplanar spacing d values are as shown in Table 9.

Table 9

| No. | XRPD data of crystal form A of oxalate | | | | | |
|---|---|---|---|---|---|---|
| | 2θ (±0.2°) | d value | Peak height | Relative intensity (I%) | Area | Relative intensity (I%) |
| 1 | 4.302 | 20.5203 | 568 | 80 | 9826 | 65.7 |
| 2 | 8.42 | 10.4919 | 710 | 100 | 8030 | 53.7 |
| 3 | 10.325 | 8.5601 | 453 | 63.8 | 4154 | 27.8 |
| 4 | 11.137 | 7.9378 | 240 | 33.8 | 2701 | 18.1 |
| 5 | 12.718 | 6.9549 | 195 | 27.5 | 1997 | 13.3 |
| 6 | 13.305 | 6.649 | 268 | 37.7 | 4885 | 32.6 |

(continued)

| No. | XRPD data of crystal form A of oxalate | | | | | |
|---|---|---|---|---|---|---|
| | 2θ (±0.2°) | d value | Peak height | Relative intensity (I%) | Area | Relative intensity (I%) |
| 7 | 15.171 | 5.8352 | 364 | 51.3 | 6633 | 44.3 |
| 8 | 16.243 | 5.4524 | 207 | 29.2 | 3613 | 24.1 |
| 9 | 16.79 | 5.2762 | 514 | 72.4 | 7782 | 52 |
| 10 | 17.525 | 5.0564 | 298 | 42 | 8714 | 58.2 |
| 11 | 17.788 | 4.9823 | 668 | 94.1 | 12227 | 81.7 |
| 12 | 18.212 | 4.8671 | 313 | 44.1 | 10035 | 67.1 |
| 13 | 18.575 | 4.7729 | 184 | 25.9 | 2175 | 14.5 |
| 14 | 20.34 | 4.3624 | 249 | 35.1 | 4574 | 30.6 |
| 15 | 20.726 | 4.2821 | 619 | 87.2 | 14963 | 100 |
| 16 | 21.011 | 4.2247 | 438 | 61.7 | 7332 | 49 |
| 17 | 22.426 | 3.9611 | 62 | 8.7 | 662 | 4.4 |
| 18 | 23.541 | 3.776 | 143 | 20.1 | 1483 | 9.9 |
| 19 | 24.415 | 3.6428 | 229 | 32.3 | 4712 | 31.5 |
| 20 | 24.778 | 3.5902 | 250 | 35.2 | 4334 | 29 |
| 21 | 25.757 | 3.456 | 697 | 98.2 | 9261 | 61.9 |
| 22 | 26.746 | 3.3304 | 492 | 69.3 | 6076 | 40.6 |
| 23 | 27.435 | 3.2483 | 558 | 78.6 | 7614 | 50.9 |
| 24 | 28.655 | 3.1127 | 110 | 15.5 | 1642 | 11 |
| 25 | 29.85 | 2.9908 | 141 | 19.9 | 1804 | 12.1 |
| 26 | 30.357 | 2.9419 | 191 | 26.9 | 2330 | 15.6 |
| 27 | 31.427 | 2.8442 | 52 | 7.3 | 1081 | 7.2 |
| 28 | 32.708 | 2.7356 | 55 | 7.7 | 1249 | 8.3 |
| 29 | 33.269 | 2.6908 | 54 | 7.6 | 1014 | 6.8 |
| 30 | 33.903 | 2.6419 | 38 | 5.4 | 576 | 3.8 |
| 31 | 35.257 | 2.5435 | 45 | 6.3 | 723 | 4.8 |
| 32 | 35.952 | 2.4959 | 42 | 5.9 | 822 | 5.5 |
| 33 | 37.026 | 2.4259 | 85 | 12 | 1608 | 10.7 |
| 34 | 38.161 | 2.3564 | 38 | 5.4 | 220 | 1.5 |
| 35 | 39.216 | 2.2954 | 39 | 5.5 | 880 | 5.9 |

In certain embodiments of the present invention, the crystal form A of oxalate has an X-ray powder diffraction pattern substantially as shown in FIG. 17; and has a DSC pattern substantially as shown in FIG. 18.

In certain embodiments of the present invention, the crystal form is crystal form A of maleate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of maleate has an X-ray powder diffraction pattern comprising diffraction peaks at 2θ (±0.2°) of 7.1±0.2°, 8.1±0.2°, 17.0±0.2°, 19.0±0.2°, 25.7±0.2° and 26.9±0.2°; preferably, the X-ray powder diffraction pattern further comprises diffraction peaks at 20 (±0.2°) of 9.0 ±0.2° and 22.9±0.2°; more preferably, the X-ray powder diffraction pattern further comprises diffraction peaks at 2θ (±0.2') of 13.4±0.2° and 20.6±0.2°.

In certain embodiments of the present invention, by using Cu-Kα radiation, the X-ray characteristic diffraction peaks of the crystal form A of maleate represented by 20 angles and interplanar spacing d values are as shown in Table 10.

Table 10

| No. | XRPD data of crystal form A of maleate | | | | | |
|---|---|---|---|---|---|---|
| | 2θ (±0.2°) | d value | Peak height | Relative intensity (I%) | Area | Relative intensity (I%) |
| 1 | 4.505 | 19.5968 | 286 | 15.9 | 1215 | 8.3 |
| 2 | 7.082 | 12.4724 | 1277 | 70.8 | 9091 | 61.8 |
| 3 | 8.072 | 10.9447 | 1803 | 100 | 13079 | 88.9 |
| 4 | 8.945 | 9.8782 | 530 | 29.4 | 3781 | 25.7 |
| 5 | 9.49 | 9.3119 | 171 | 9.5 | 1134 | 7.7 |
| 6 | 11.178 | 7.9088 | 49 | 2.7 | 406 | 2.8 |
| 7 | 13.019 | 6.7943 | 130 | 7.2 | 1034 | 7 |
| 8 | 13.387 | 6.6087 | 263 | 14.6 | 3258 | 22.1 |
| 9 | 14.6 | 6.0622 | 157 | 8.7 | 1715 | 11.7 |
| 10 | 14.968 | 5.9138 | 296 | 16.4 | 1712 | 11.6 |
| 11 | 15.898 | 5.5699 | 270 | 15 | 2681 | 18.2 |
| 12 | 16.991 | 5.2139 | 1016 | 56.4 | 8348 | 56.8 |
| 13 | 17.44 | 5.0809 | 301 | 16.7 | 2418 | 16.4 |
| 14 | 17.867 | 4.9603 | 288 | 16 | 1689 | 11.5 |
| 15 | 18.533 | 4.7834 | 67 | 3.7 | 919 | 6.2 |
| 16 | 18.984 | 4.6708 | 764 | 42.4 | 9328 | 63.4 |
| 17 | 19.673 | 4.5089 | 201 | 11.1 | 1818 | 12.4 |
| 18 | 20.184 | 4.3958 | 53 | 2.9 | 442 | 3 |
| 19 | 20.599 | 4.3081 | 347 | 19.2 | 3184 | 21.6 |
| 20 | 21.177 | 4.1919 | 61 | 3.4 | 372 | 2.5 |
| 21 | 22.006 | 4.0359 | 36 | 2 | 225 | 1.5 |
| 22 | 22.369 | 3.9712 | 60 | 3.3 | 503 | 3.4 |
| 23 | 22.896 | 3.8809 | 396 | 22 | 4284 | 29.1 |
| 24 | 24.274 | 3.6637 | 121 | 6.7 | 1514 | 10.3 |
| 25 | 24.782 | 3.5897 | 129 | 7.2 | 1003 | 6.8 |
| 26 | 25.652 | 3.4699 | 1378 | 76.4 | 14710 | 100 |
| 27 | 26.094 | 3.4121 | 56 | 3.1 | 520 | 3.5 |
| 28 | 26.504 | 3.3602 | 226 | 12.5 | 2550 | 17.3 |
| 29 | 26.872 | 3.3151 | 441 | 24.5 | 4532 | 30.8 |
| 30 | 27.341 | 3.2592 | 74 | 4.1 | 446 | 3 |
| 31 | 27.741 | 3.2131 | 154 | 8.5 | 2367 | 16.1 |
| 32 | 28.005 | 3.1835 | 125 | 6.9 | 2116 | 14.4 |
| 33 | 28.794 | 3.098 | 126 | 7 | 3103 | 21.1 |
| 34 | 29.121 | 3.0639 | 91 | 5 | 1302 | 8.9 |
| 35 | 29.89 | 2.9868 | 142 | 7.9 | 2904 | 19.7 |
| 36 | 30.131 | 2.9635 | 146 | 8.1 | 2515 | 17.1 |
| 37 | 31.347 | 2.8512 | 53 | 2.9 | 785 | 5.3 |
| 38 | 31.974 | 2.7967 | 69 | 3.8 | 914 | 6.2 |

(continued)

| No. | XRPD data of crystal form A of maleate | | | | | |
|---|---|---|---|---|---|---|
| | 2θ (±0.2°) | d value | Peak height | Relative intensity (I%) | Area | Relative intensity (I%) |
| 39 | 32.437 | 2.7579 | 52 | 2.9 | 769 | 5.2 |
| 40 | 32.85 | 2.7241 | 46 | 2.6 | 366 | 2.5 |
| 41 | 33.768 | 2.6522 | 62 | 3.4 | 344 | 2.3 |
| 42 | 34.735 | 2.5805 | 38 | 2.1 | 381 | 2.6 |
| 43 | 35.199 | 2.5475 | 55 | 3.1 | 448 | 3 |
| 44 | 36.804 | 2.4401 | 59 | 3.3 | 1450 | 9.9 |
| 45 | 39.601 | 2.2739 | 46 | 2.6 | 224 | 1.5 |

In certain embodiments of the present invention, the crystal form A of maleate has an X-ray powder diffraction pattern substantially as shown in FIG. 19; and has a DSC pattern substantially as shown in FIG. 20.

In certain embodiments of the present invention, the crystal form is crystal form A of salicylate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of salicylate has an X-ray powder diffraction pattern comprising diffraction peaks at 2θ (±0.2°) of 8.3±0.2°, 16.5±0.2°, 18.5±0.2°, 20.4±0.2°, 22.3 ±0.2° and 24.4±0.2°; preferably, the X-ray powder diffraction pattern further comprises diffraction peaks at 20 (±0.2°) of 22.0±0.2° and 28.0±0.2°; more preferably, the X-ray powder diffraction pattern further comprises diffraction peaks at 2θ (±0.2°) of 16.3±0.2° and 21.0±0.2°.

In certain embodiments of the present invention, by using Cu-Kα radiation, the X-ray characteristic diffraction peaks of the crystal form A of salicylate represented by 20 angles and interplanar spacing d values are as shown in Table 11.

Table 11

| No. | XRPD data of crystal form A of salicylate | | | | | |
|---|---|---|---|---|---|---|
| | 2θ (±0.2°) | d value | Peak height | Relative intensity (I%) | Area | Relative intensity (I%) |
| 1 | 4.937 | 17.8841 | 100 | 3 | 328 | 1 |
| 2 | 8.319 | 10.6199 | 959 | 28.9 | 7334 | 23.1 |
| 3 | 9.74 | 9.073 | 226 | 6.8 | 1788 | 5.6 |
| 4 | 10.789 | 8.1936 | 147 | 4.4 | 1077 | 3.4 |
| 5 | 11.275 | 7.8413 | 87 | 2.6 | 1009 | 3.2 |
| 6 | 11.991 | 7.3746 | 178 | 5.4 | 1282 | 4 |
| 7 | 13.304 | 6.6497 | 395 | 11.9 | 4221 | 13.3 |
| 8 | 13.878 | 6.3759 | 52 | 1.6 | 595 | 1.9 |
| 9 | 14.191 | 6.2357 | 107 | 3.2 | 1551 | 4.9 |
| 10 | 14.584 | 6.0685 | 462 | 13.9 | 4682 | 14.8 |
| 11 | 14.95 | 5.921 | 467 | 14.1 | 4108 | 13 |
| 12 | 15.759 | 5.6188 | 332 | 10 | 2558 | 8.1 |
| 13 | 16.284 | 5.4386 | 515 | 15.5 | 6053 | 19.1 |
| 14 | 16.531 | 5.3582 | 726 | 21.9 | 7966 | 25.1 |
| 15 | 17.378 | 5.0987 | 55 | 1.7 | 332 | 1 |
| 16 | 18.234 | 4.8613 | 248 | 7.5 | 2232 | 7 |
| 17 | 18.518 | 4.7874 | 1268 | 38.2 | 15225 | 48 |
| 18 | 19.226 | 4.6126 | 61 | 1.8 | 186 | 0.6 |

(continued)

| No. | XRPD data of crystal form A of salicylate | | | | | |
|---|---|---|---|---|---|---|
| | 2θ (±0.2°) | d value | Peak height | Relative intensity (I%) | Area | Relative intensity (I%) |
| 19 | 19.552 | 4.5366 | 55 | 1.7 | 120 | 0.4 |
| 20 | 19.956 | 4.4455 | 405 | 12.2 | 4760 | 15 |
| 21 | 20.443 | 4.3408 | 1493 | 45 | 14117 | 44.5 |
| 22 | 20.99 | 4.2289 | 669 | 20.2 | 6122 | 19.3 |
| 23 | 21.523 | 4.1253 | 42 | 1.3 | 192 | 0.6 |
| 24 | 21.959 | 4.0443 | 404 | 12.2 | 6431 | 20.3 |
| 25 | 22.326 | 3.9787 | 157 | 4.7 | 6694 | 21.1 |
| 26 | 23.298 | 3.8148 | 90 | 2.7 | 855 | 2.7 |
| 27 | 23.971 | 3.7093 | 425 | 12.8 | 3814 | 12 |
| 28 | 24.353 | 3.652 | 3318 | 100 | 31706 | 100 |
| 29 | 24.961 | 3.5644 | 206 | 6.2 | 1357 | 4.3 |
| 30 | 25.529 | 3.4863 | 240 | 7.2 | 2151 | 6.8 |
| 31 | 25.755 | 3.4562 | 143 | 4.3 | 1749 | 5.5 |
| 32 | 26.216 | 3.3965 | 137 | 4.1 | 1259 | 4 |
| 33 | 26.706 | 3.3352 | 69 | 2.1 | 1212 | 3.8 |
| 34 | 26.926 | 3.3085 | 61 | 1.8 | 1299 | 4.1 |
| 35 | 27.293 | 3.2649 | 54 | 1.6 | 528 | 1.7 |
| 36 | 27.964 | 3.188 | 578 | 17.4 | 6270 | 19.8 |
| 37 | 28.429 | 3.1369 | 297 | 9 | 2537 | 8 |
| 38 | 28.955 | 3.0811 | 54 | 1.6 | 230 | 0.7 |
| 39 | 29.283 | 3.0474 | 95 | 2.9 | 1327 | 4.2 |
| 40 | 30.618 | 2.9174 | 208 | 6.3 | 2235 | 7 |
| 41 | 31.205 | 2.8639 | 73 | 2.2 | 1726 | 5.4 |
| 42 | 32.124 | 2.784 | 35 | 1.1 | 146 | 0.5 |
| 43 | 32.764 | 2.7311 | 90 | 2.7 | 1409 | 4.4 |
| 44 | 33.029 | 2.7098 | 91 | 2.7 | 1959 | 6.2 |
| 45 | 33.928 | 2.64 | 53 | 1.6 | 310 | 1 |
| 46 | 34.386 | 2.6059 | 74 | 2.2 | 1417 | 4.5 |
| 47 | 34.795 | 2.5762 | 49 | 1.5 | 555 | 1.8 |
| 48 | 35.808 | 2.5056 | 135 | 4.1 | 1860 | 5.9 |
| 49 | 36.781 | 2.4415 | 61 | 1.8 | 822 | 2.6 |
| 50 | 37.065 | 2.4235 | 68 | 2 | 922 | 2.9 |
| 51 | 37.657 | 2.3867 | 85 | 2.6 | 684 | 2.2 |
| 52 | 38.241 | 2.3516 | 46 | 1.4 | 975 | 3.1 |
| 53 | 38.52 | 2.3352 | 46 | 1.4 | 1446 | 4.6 |
| 54 | 38.832 | 2.3171 | 66 | 2 | 1455 | 4.6 |
| 55 | 39.153 | 2.2989 | 40 | 1.2 | 410 | 1.3 |

In certain embodiments of the present invention, the crystal form A of salicylate has an X-ray powder diffraction

pattern substantially as shown in FIG. 21; and has a DSC pattern substantially as shown in FIG. 22.

In certain embodiments of the present invention, the crystal form is crystal form A of p-hydroxybenzoate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A of p-hydroxybenzoate has an X-ray powder diffraction pattern comprising diffraction peaks at 2θ (±0.2°) of 5.9±0.2°, 17.2±0.2°, 17.5±0.2°, 18.9±0.2°, 22.3 ±0.2° and 25.2±0.2°; preferably, the X-ray powder diffraction pattern further comprises diffraction peaks at 2θ (±0.2°) of 16.1±0.2° and 22.0±0.2°; more preferably, the X-ray powder diffraction pattern further comprises diffraction peaks at 2θ (±0.2°) of 23.7±0.2° and 25.6±0.2°.

In certain embodiments of the present invention, by using Cu-Kα radiation, the X-ray characteristic diffraction peaks of the crystal form A of p-hydroxybenzoate represented by 2θ angles and interplanar spacing d values are as shown in Table 12.

Table 12

| No. | XRPD data of crystal form A of p-hydroxybenzoate | | | | | |
|---|---|---|---|---|---|---|
| | 2θ (±0.2°) | d value | Peak height | Relative intensity (I%) | Area | Relative intensity (I%) |
| 1 | 5.925 | 14.9038 | 1866 | 100 | 24628 | 100 |
| 2 | 8.381 | 10.5416 | 113 | 6.1 | 3076 | 12.5 |
| 3 | 8.621 | 10.2481 | 365 | 19.6 | 4202 | 17.1 |
| 4 | 11.05 | 8.0001 | 132 | 7.1 | 984 | 4 |
| 5 | 11.668 | 7.5783 | 296 | 15.9 | 3318 | 13.5 |
| 6 | 12.84 | 6.8886 | 153 | 8.2 | 1248 | 5.1 |
| 7 | 13.873 | 6.3783 | 371 | 19.9 | 4088 | 16.6 |
| 8 | 15.231 | 5.8125 | 352 | 18.9 | 3845 | 15.6 |
| 9 | 15.765 | 5.6168 | 113 | 6.1 | 1005 | 4.1 |
| 10 | 16.123 | 5.4928 | 630 | 33.8 | 5720 | 23.2 |
| 11 | 16.588 | 5.3399 | 73 | 3.9 | 770 | 3.1 |
| 12 | 17.179 | 5.1573 | 154 | 8.3 | 6605 | 26.8 |
| 13 | 17.502 | 5.0629 | 808 | 43.3 | 12043 | 48.9 |
| 14 | 17.89 | 4.9541 | 158 | 8.5 | 1734 | 7 |
| 15 | 18.924 | 4.6856 | 569 | 30.5 | 5745 | 23.3 |
| 16 | 19.369 | 4.579 | 137 | 7.3 | 1709 | 6.9 |
| 17 | 19.982 | 4.4397 | 126 | 6.8 | 994 | 4 |
| 18 | 20.449 | 4.3394 | 52 | 2.8 | 576 | 2.3 |
| 19 | 20.864 | 4.254 | 272 | 14.6 | 3416 | 13.9 |
| 20 | 21.295 | 4.169 | 90 | 4.8 | 302 | 1.2 |
| 21 | 22.004 | 4.0362 | 369 | 19.8 | 5167 | 21 |
| 22 | 22.324 | 3.979 | 263 | 14.1 | 6126 | 24.9 |
| 23 | 22.59 | 3.9327 | 146 | 7.8 | 3248 | 13.2 |
| 24 | 23.705 | 3.7503 | 275 | 14.7 | 4389 | 17.8 |
| 25 | 24.417 | 3.6426 | 176 | 9.4 | 1461 | 5.9 |
| 26 | 25.206 | 3.5302 | 736 | 39.4 | 9330 | 37.9 |
| 27 | 25.57 | 3.4808 | 265 | 14.2 | 4745 | 19.3 |
| 28 | 25.874 | 3.4405 | 191 | 10.2 | 2547 | 10.3 |
| 29 | 26.645 | 3.3427 | 221 | 11.8 | 2249 | 9.1 |
| 30 | 27.539 | 3.2362 | 110 | 5.9 | 1018 | 4.1 |

(continued)

| No. | XRPD data of crystal form A of p-hydroxybenzoate | | | | | |
|---|---|---|---|---|---|---|
| | 2θ (±0.2°) | d value | Peak height | Relative intensity (I%) | Area | Relative intensity (I%) |
| 31 | 27.759 | 3.2111 | 147 | 7.9 | 2293 | 9.3 |
| 32 | 28.554 | 3.1235 | 233 | 12.5 | 3335 | 13.5 |
| 33 | 29.324 | 3.0432 | 45 | 2.4 | 200 | 0.8 |
| 34 | 30.011 | 2.9751 | 136 | 7.3 | 3022 | 12.3 |
| 35 | 30.316 | 2.9459 | 118 | 6.3 | 3125 | 12.7 |
| 36 | 31.002 | 2.8822 | 95 | 5.1 | 1486 | 6 |
| 37 | 31.24 | 2.8607 | 56 | 3 | 1171 | 4.8 |
| 38 | 31.955 | 2.7984 | 87 | 4.7 | 810 | 3.3 |
| 39 | 33.29 | 2.6891 | 92 | 4.9 | 1198 | 4.9 |
| 40 | 34.51 | 2.5968 | 66 | 3.5 | 464 | 1.9 |
| 41 | 35.95 | 2.496 | 66 | 3.5 | 923 | 3.7 |
| 42 | 36.981 | 2.4288 | 45 | 2.4 | 361 | 1.5 |
| 43 | 38.137 | 2.3577 | 34 | 1.8 | 536 | 2.2 |
| 44 | 38.991 | 2.3081 | 38 | 2 | 440 | 1.8 |
| 45 | 39.172 | 2.2978 | 40 | 2.1 | 439 | 1.8 |

In certain embodiments of the present invention, the crystal form A of p-hydroxybenzoate has an X-ray powder diffraction pattern substantially as shown in FIG. 23; and has a DSC pattern substantially as shown in FIG. 24.

In certain embodiments of the present invention, in the X-ray powder diffraction patterns of the crystal form A of phosphate, the crystal form B of phosphate, the crystal form A of hydrochloride, the crystal form B of hydrochloride, the crystal form A of sulphate, the crystal form A of hydrobromide, the crystal form B of hydrobromide, the crystal form A of citrate, the crystal form A of oxalate, the crystal form A of maleate, the crystal form A of salicylate, and the crystal form A of p-hydroxybenzoate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, the positions of the diffraction peaks with the top ten relative peak intensities have a 2θ deviation of ±0.2° to ±0.5°, preferably ±0.2° to ±0.3°, and most preferably ±0.2° from the corresponding positions of the diffraction peaks in FIG. 1, FIG. 3, FIG. 5, FIG. 7, FIG. 9, FIG. 11, FIG. 13, FIG. 15, FIG. 17, FIG. 19, FIG. 21, and FIG. 23, respectively.

In certain embodiments of the present invention, the crystal form is crystal form A of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form A has an X-ray powder diffraction pattern comprising diffraction peaks at 2θ (±0.2°) of 13.0±0.2°, 14.0±0.2°, 15.9±0.2°, 19.8+0.2°, 20.2±0.2° and 22.3±0.2°; preferably, the X-ray powder diffraction pattern further comprises diffraction peaks at 2θ (±0.2°) of 12.4±0.2° and 24.6±0.2°; more preferably, the X-ray powder diffraction pattern further comprises diffraction peaks at 2θ (±0.2°) of 5.3±0.2° and 21.3±0.2°.

In certain embodiments of the present invention, by using Cu-Kα radiation, the X-ray characteristic diffraction peaks of the crystal form A represented by 2θ angles and interplanar spacing d values are as shown in Table 13.

Table 13

| No. | XRPD data of crystal form A of free base | | | | | |
|---|---|---|---|---|---|---|
| | 2θ (+0.2°) | d value | Peak height | Relative intensity (I%) | Area | Relative intensity (I%) |
| 1 | 5.26 | 16.7858 | 809 | 31.3 | 4460 | 26.8 |
| 2 | 8.337 | 10.5969 | 113 | 4.4 | 772 | 4.6 |
| 3 | 12.409 | 7.1269 | 860 | 33.3 | 5172 | 31.1 |
| 4 | 12.956 | 6.8276 | 2583 | 100 | 16627 | 100 |
| 5 | 14.008 | 6.3171 | 1284 | 49.7 | 8309 | 50 |

(continued)

| No. | XRPD data of crystal form A of free base | | | | | |
|---|---|---|---|---|---|---|
| | 2θ (+0.2°) | d value | Peak height | Relative intensity (I%) | Area | Relative intensity (I%) |
| 6 | 14.613 | 6.0566 | 55 | 2.1 | 289 | 1.7 |
| 7 | 15.914 | 5.5645 | 1521 | 58.9 | 9424 | 56.7 |
| 8 | 16.232 | 5.4562 | 124 | 4.8 | 991 | 6 |
| 9 | 16.79 | 5.276 | 146 | 5.7 | 1330 | 8 |
| 10 | 17.202 | 5.1506 | 143 | 5.5 | 946 | 5.7 |
| 11 | 17.48 | 5.0693 | 32 | 1.2 | 172 | 1 |
| 12 | 18.112 | 4.8937 | 92 | 3.6 | 763 | 4.6 |
| 13 | 19.794 | 4.4816 | 1025 | 39.7 | 8927 | 53.7 |
| 14 | 20.219 | 4.3883 | 861 | 33.3 | 9216 | 55.4 |
| 15 | 20.592 | 4.3096 | 48 | 1.9 | 242 | 1.5 |
| 16 | 20.86 | 4.2548 | 136 | 5.3 | 1113 | 6.7 |
| 17 | 21.273 | 4.1732 | 544 | 21.1 | 4080 | 24.5 |
| 18 | 22.266 | 3.9892 | 1159 | 44.9 | 8716 | 52.4 |
| 19 | 23.282 | 3.8175 | 234 | 9.1 | 1728 | 10.4 |
| 20 | 24.06 | 3.6957 | 405 | 15.7 | 2845 | 17.1 |
| 21 | 24.642 | 3.6097 | 821 | 31.8 | 7980 | 48 |
| 22 | 25.091 | 3.5461 | 104 | 4 | 736 | 4.4 |
| 23 | 25.928 | 3.4335 | 62 | 2.4 | 483 | 2.9 |
| 24 | 26.687 | 3.3375 | 351 | 13.6 | 3136 | 18.9 |
| 25 | 27.544 | 3.2356 | 270 | 10.5 | 2364 | 14.2 |
| 26 | 28.245 | 3.1569 | 163 | 6.3 | 1423 | 8.6 |
| 27 | 28.757 | 3.1019 | 33 | 1.3 | 397 | 2.4 |
| 28 | 29.044 | 3.0718 | 99 | 3.8 | 792 | 4.8 |
| 29 | 29.879 | 2.9879 | 37 | 1.4 | 227 | 1.4 |
| 30 | 30.212 | 2.9557 | 241 | 9.3 | 4031 | 24.2 |
| 31 | 30.777 | 2.9027 | 37 | 1.4 | 203 | 1.2 |
| 32 | 31.306 | 2.8549 | 60 | 2.3 | 485 | 2.9 |
| 33 | 33.277 | 2.6901 | 110 | 4.3 | 1433 | 8.6 |
| 34 | 34.251 | 2.6159 | 87 | 3.4 | 604 | 3.6 |
| 35 | 34.617 | 2.589 | 75 | 2.9 | 2015 | 12.1 |
| 36 | 34.944 | 2.5656 | 33 | 1.3 | 642 | 3.9 |
| 37 | 35.208 | 2.5469 | 35 | 1.4 | 578 | 3.5 |
| 38 | 36.141 | 2.4833 | 130 | 5 | 1496 | 9 |
| 39 | 36.699 | 2.4468 | 63 | 2.4 | 520 | 3.1 |
| 40 | 37.736 | 2.3819 | 46 | 1.8 | 830 | 5 |
| 41 | 37.984 | 2.3669 | 82 | 3.2 | 1170 | 7 |
| 42 | 39.276 | 2.292 | 39 | 1.5 | 590 | 3.5 |
| 43 | 40.109 | 2.2463 | 33 | 1.3 | 515 | 3.1 |

(continued)

| No. | XRPD data of crystal form A of free base | | | | | |
|---|---|---|---|---|---|---|
| | 2θ (+0.2°) | d value | Peak height | Relative intensity (I%) | Area | Relative intensity (I%) |
| 44 | 42.067 | 2.1461 | 25 | 1 | 190 | 1.1 |
| 45 | 43.231 | 2.091 | 29 | 1.1 | 859 | 5.2 |
| 46 | 43.399 | 2.0833 | 74 | 2.9 | 962 | 5.8 |
| 47 | 45.499 | 1.9919 | 34 | 1.3 | 365 | 2.2 |
| 48 | 47.052 | 1.9297 | 26 | 1 | 325 | 2 |
| 49 | 47.314 | 1.9197 | 36 | 1.4 | 402 | 2.4 |

In certain embodiments of the present invention, the crystal form A has an X-ray powder diffraction pattern substantially as shown in FIG. 25; and has a DSC pattern as shown in FIG. 26.

In certain embodiments of the present invention, the crystal form is crystal form B of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form B has an X-ray powder diffraction pattern comprising diffraction peaks at 20 (±0.2°) of 11.3±0.2°, 11.6±0.2°, 17.4±0.2°, 21.9±0.2°, 22.2±0.2° and 23.3±0.2°; preferably, the X-ray powder diffraction pattern further comprises diffraction peaks at 2θ (±0.2°) of 12.0±0.2° and 15.5±0.2°; more preferably, the X-ray powder diffraction pattern further comprises diffraction peaks at 20 (±0.2°) of 15.9±0.2° and 27.2±0.2°.

In certain embodiments of the present invention, by using Cu-Kα radiation, the X-ray characteristic diffraction peaks of the crystal form B represented by 2θ angles and interplanar spacing d values are as shown in Table 14.

Table 14

| No. | XRPD data of crystal form B of free base | | | | | |
|---|---|---|---|---|---|---|
| | 2θ (+0.2°) | d value | Peak height | Relative intensity (I%) | Area | Relative intensity (I%) |
| 1 | 5.36 | 16.474 | 272 | 10 | 3524 | 11 |
| 2 | 6.293 | 14.0337 | 135 | 5 | 868 | 2.7 |
| 3 | 7.791 | 11.3376 | 156 | 5.7 | 1724 | 5.4 |
| 4 | 9.78 | 9.0367 | 142 | 5.2 | 1239 | 3.9 |
| 5 | 11.342 | 7.7951 | 299 | 11 | 6131 | 19.1 |
| 6 | 11.644 | 7.5939 | 2721 | 100 | 32026 | 100 |
| 7 | 12.007 | 7.3648 | 142 | 5.2 | 5722 | 17.9 |
| 8 | 12.515 | 7.0669 | 181 | 6.7 | 1170 | 3.7 |
| 9 | 13.862 | 6.3831 | 53 | 1.9 | 238 | 0.7 |
| 10 | 14.44 | 6.1287 | 332 | 12.2 | 3391 | 10.6 |
| 11 | 15.518 | 5.7057 | 492 | 18.1 | 5558 | 17.4 |
| 12 | 15.941 | 5.555 | 344 | 12.6 | 4654 | 14.5 |
| 13 | 16.348 | 5.4176 | 72 | 2.6 | 503 | 1.6 |
| 14 | 16.932 | 5.232 | 156 | 5.7 | 1523 | 4.8 |
| 15 | 17.421 | 5.0863 | 212 | 7.8 | 6236 | 19.5 |
| 16 | 17.786 | 4.9827 | 98 | 3.6 | 1806 | 5.6 |
| 17 | 18.192 | 4.8723 | 61 | 2.2 | 373 | 1.2 |
| 18 | 18.739 | 4.7314 | 241 | 8.9 | 2188 | 6.8 |
| 19 | 20.055 | 4.4238 | 265 | 9.7 | 3105 | 9.7 |
| 20 | 20.399 | 4.35 | 290 | 10.7 | 4096 | 12.8 |
| 21 | 21.454 | 4.1384 | 282 | 10.4 | 3783 | 11.8 |

(continued)

| No. | XRPD data of crystal form B of free base | | | | | |
|---|---|---|---|---|---|---|
| | 2θ (+0.2°) | d value | Peak height | Relative intensity (I%) | Area | Relative intensity (I%) |
| 22 | 21.941 | 4.0477 | 352 | 12.9 | 7876 | 24.6 |
| 23 | 22.206 | 3.9999 | 414 | 15.2 | 6390 | 20 |
| 24 | 22.833 | 3.8915 | 275 | 10.1 | 2898 | 9 |
| 25 | 23.301 | 3.8144 | 527 | 19.4 | 6133 | 19.2 |
| 26 | 24.092 | 3.6909 | 155 | 5.7 | 1485 | 4.6 |
| 27 | 25.169 | 3.5354 | 105 | 3.9 | 2534 | 7.9 |
| 28 | 25.589 | 3.4783 | 208 | 7.6 | 2934 | 9.2 |
| 29 | 26.243 | 3.3931 | 216 | 7.9 | 2798 | 8.7 |
| 30 | 27.231 | 3.2721 | 293 | 10.8 | 4840 | 15.1 |
| 31 | 27.516 | 3.2389 | 129 | 4.7 | 2461 | 7.7 |
| 32 | 28.431 | 3.1367 | 130 | 4.8 | 1365 | 4.3 |
| 33 | 28.975 | 3.079 | 49 | 1.8 | 428 | 1.3 |
| 34 | 29.48 | 3.0274 | 74 | 2.7 | 1535 | 4.8 |
| 35 | 29.79 | 2.9967 | 158 | 5.8 | 3555 | 11.1 |
| 36 | 31.344 | 2.8515 | 42 | 1.5 | 277 | 0.9 |
| 37 | 32.743 | 2.7328 | 45 | 1.7 | 796 | 2.5 |
| 38 | 33.233 | 2.6936 | 33 | 1.2 | 808 | 2.5 |
| 39 | 34.306 | 2.6118 | 72 | 2.6 | 821 | 2.6 |
| 40 | 34.996 | 2.5618 | 39 | 1.4 | 751 | 2.3 |
| 41 | 35.34 | 2.5377 | 58 | 2.1 | 898 | 2.8 |
| 42 | 36.842 | 2.4376 | 68 | 2.5 | 845 | 2.6 |
| 43 | 37.975 | 2.3674 | 62 | 2.3 | 948 | 3 |
| 44 | 39.154 | 2.2988 | 43 | 1.6 | 357 | 1.1 |

In certain embodiments of the present invention, the crystal form B has an X-ray powder diffraction pattern substantially as shown in FIG. 27; and has a DSC pattern substantially as shown in FIG. 28.

In certain embodiments of the present invention, the crystal form is crystal form C of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form C has an X-ray powder diffraction pattern comprising diffraction peaks at 2θ (±0.2°) of 11.6±0.2°, 18.5±0.2°, 19.3±0.2°, 21.5±0.2°, 23.2±0.2° and 23.4±0.2°; preferably, the X-ray powder diffraction pattern further comprises diffraction peaks at 2θ (±0.2°) of 7.2±0.2° and 18.8±0.2°; more preferably, the X-ray powder diffraction pattern further comprises diffraction peaks at 2θ (±0.2°) of 8.6±0.2° and 22.3±0.2°.

In certain embodiments of the present invention, by using Cu-Kα radiation, the X-ray characteristic diffraction peaks of the crystal form C represented by 2θ angles and interplanar spacing d values are as shown in Table 15.

Table 15

| No. | XRPD data of crystal form C of free base | | | | | |
|---|---|---|---|---|---|---|
| | 2θ (±0.2°) | d value | Peak height | Relative intensity (I%) | Area | Relative intensity (I%) |
| 1 | 6.228 | 14.1806 | 452 | 41.1 | 3804 | 23.9 |
| 2 | 7.222 | 12.2302 | 788 | 71.6 | 6559 | 41.2 |
| 3 | 8.639 | 10.2275 | 572 | 52 | 5788 | 36.4 |
| 4 | 11.581 | 7.6346 | 1082 | 98.3 | 8543 | 53.7 |

(continued)

| No. | XRPD data of crystal form C of free base | | | | | |
|---|---|---|---|---|---|---|
| | 2θ (±0.2°) | d value | Peak height | Relative intensity (I%) | Area | Relative intensity (I%) |
| 5 | 12.086 | 7.3167 | 179 | 16.3 | 2413 | 15.2 |
| 6 | 12.314 | 7.1816 | 211 | 19.2 | 1944 | 12.2 |
| 7 | 12.728 | 6.9491 | 85 | 7.7 | 515 | 3.2 |
| 8 | 13.22 | 6.6915 | 333 | 30.2 | 2611 | 16.4 |
| 9 | 14.355 | 6.1649 | 350 | 31.8 | 3227 | 20.3 |
| 10 | 15.693 | 5.6422 | 51 | 4.6 | 487 | 3.1 |
| 11 | 16.036 | 5.5224 | 39 | 3.5 | 206 | 1.3 |
| 12 | 16.588 | 5.3398 | 127 | 11.5 | 1414 | 8.9 |
| 13 | 17.159 | 5.1635 | 456 | 41.4 | 3920 | 24.6 |
| 14 | 17.888 | 4.9547 | 519 | 47.1 | 4308 | 27.1 |
| 15 | 18.476 | 4.7981 | 1101 | 100 | 11375 | 71.5 |
| 16 | 18.798 | 4.7166 | 553 | 50.2 | 6160 | 38.7 |
| 17 | 19.344 | 4.5848 | 797 | 72.4 | 10713 | 67.3 |
| 18 | 19.815 | 4.4768 | 475 | 43.1 | 5233 | 32.9 |
| 19 | 20.519 | 4.3248 | 98 | 8.9 | 909 | 5.7 |
| 20 | 20.867 | 4.2536 | 117 | 10.6 | 817 | 5.1 |
| 21 | 21.153 | 4.1965 | 579 | 52.6 | 5598 | 35.2 |
| 22 | 21.513 | 4.1271 | 779 | 70.8 | 7056 | 44.3 |
| 23 | 21.984 | 4.0398 | 102 | 9.3 | 1136 | 7.1 |
| 24 | 22.323 | 3.9792 | 563 | 51.1 | 5346 | 33.6 |
| 25 | 22.894 | 3.8813 | 135 | 12.3 | 3719 | 23.4 |
| 26 | 23.18 | 3.834 | 413 | 37.5 | 10264 | 64.5 |
| 27 | 23.401 | 3.7983 | 826 | 75 | 15915 | 100 |
| 28 | 23.722 | 3.7476 | 267 | 24.3 | 3727 | 23.4 |
| 29 | 24.152 | 3.6819 | 139 | 12.6 | 1694 | 10.6 |
| 30 | 24.434 | 3.64 | 130 | 11.8 | 1818 | 11.4 |
| 31 | 24.618 | 3.6132 | 172 | 15.6 | 2563 | 16.1 |
| 32 | 25.652 | 3.4699 | 138 | 12.5 | 1796 | 11.3 |
| 33 | 26.101 | 3.4112 | 180 | 16.3 | 1834 | 11.5 |
| 34 | 26.968 | 3.3034 | 51 | 4.6 | 369 | 2.3 |
| 35 | 27.66 | 3.2224 | 115 | 10.4 | 1045 | 6.6 |
| 36 | 28.025 | 3.1812 | 361 | 32.8 | 4288 | 26.9 |
| 37 | 28.677 | 3.1104 | 96 | 8.7 | 1345 | 8.5 |
| 38 | 29.176 | 3.0583 | 43 | 3.9 | 562 | 3.5 |
| 39 | 29.908 | 2.9851 | 116 | 10.5 | 1590 | 10 |
| 40 | 30.332 | 2.9443 | 63 | 5.7 | 758 | 4.8 |
| 41 | 30.906 | 2.8909 | 33 | 3 | 67 | 0.4 |
| 42 | 31.713 | 2.8191 | 33 | 3 | 154 | 1 |

(continued)

| No. | XRPD data of crystal form C of free base | | | | | |
|---|---|---|---|---|---|---|
| | 2θ (±0.2°) | d value | Peak height | Relative intensity (I%) | Area | Relative intensity (I%) |
| 43 | 32.705 | 2.7359 | 98 | 8.9 | 1402 | 8.8 |
| 44 | 33.074 | 2.7062 | 84 | 7.6 | 1979 | 12.4 |
| 45 | 33.661 | 2.6604 | 52 | 4.7 | 335 | 2.1 |
| 46 | 34.737 | 2.5804 | 70 | 6.4 | 1114 | 7 |
| 47 | 35.259 | 2.5434 | 84 | 7.6 | 695 | 4.4 |
| 48 | 36.435 | 2.4639 | 54 | 4.9 | 1164 | 7.3 |
| 49 | 37.294 | 2.4091 | 34 | 3.1 | 352 | 2.2 |
| 50 | 37.574 | 2.3918 | 53 | 4.8 | 531 | 3.3 |
| 51 | 38.12 | 2.3588 | 78 | 7.1 | 1915 | 12 |
| 52 | 38.545 | 2.3338 | 79 | 7.2 | 1567 | 9.8 |

In certain embodiments of the present invention, the crystal form C has an X-ray powder diffraction pattern substantially as shown in FIG. 29; and has a DSC pattern substantially as shown in FIG. 30.

In certain embodiments of the present invention, the crystal form is crystal form D of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form D has an X-ray powder diffraction pattern comprising diffraction peaks at 20 (±0.2°) of 12.2±0.2°, 16.1±0.2°, 19.7±0.2°, 21.2±0.2°, 21.9±0.2° and 24.0±0.2°; preferably, the X-ray powder diffraction pattern further comprises diffraction peaks at 2θ (±0.2') of 15.9±0.2° and 21.4±0.2°; more preferably, the X-ray powder diffraction pattern further comprises diffraction peaks at 2θ (±0.2°) of 24.5±0.2° and 29.0±0.2°.

In certain embodiments of the present invention, by using Cu-Kα radiation, the X-ray characteristic diffraction peaks of the crystal form D represented by 20 angles and interplanar spacing d values are as shown in Table 16.

Table 16

| No. | XRPD data of crystal form D of free base | | | | | |
|---|---|---|---|---|---|---|
| | 2θ (+0.2°) | d value | Peak height | Relative intensity (I%) | Area | Relative intensity (I%) |
| 1 | 8.025 | 11.008 | 245 | 30.1 | 2470 | 23.1 |
| 2 | 12.155 | 7.2752 | 815 | 100 | 8046 | 75.2 |
| 3 | 13.249 | 6.6771 | 192 | 23.6 | 1692 | 15.8 |
| 4 | 15.895 | 5.5709 | 204 | 25 | 4703 | 44 |
| 5 | 16.109 | 5.4975 | 365 | 44.8 | 5955 | 55.7 |
| 6 | 17.009 | 5.2085 | 221 | 27.1 | 2025 | 18.9 |
| 7 | 18.642 | 4.7557 | 207 | 25.4 | 1929 | 18 |
| 8 | 19.191 | 4.621 | 77 | 9.4 | 728 | 6.8 |
| 9 | 19.657 | 4.5125 | 619 | 76 | 6785 | 63.4 |
| 10 | 20.059 | 4.4229 | 116 | 14.2 | 2158 | 20.2 |
| 11 | 21.175 | 4.1923 | 688 | 84.4 | 9689 | 90.6 |
| 12 | 21.448 | 4.1395 | 195 | 23.9 | 5436 | 50.8 |
| 13 | 21.895 | 4.056 | 706 | 86.6 | 10695 | 100 |
| 14 | 23.374 | 3.8027 | 76 | 9.3 | 598 | 5.6 |
| 15 | 24 | 3.7049 | 554 | 68 | 5968 | 55.8 |
| 16 | 24.467 | 3.6351 | 243 | 29.8 | 3300 | 30.9 |
| 17 | 25.037 | 3.5537 | 161 | 19.8 | 1619 | 15.1 |

(continued)

| No. | XRPD data of crystal form D of free base | | | | | |
|---|---|---|---|---|---|---|
| | 2θ (+0.2°) | d value | Peak height | Relative intensity (I%) | Area | Relative intensity (I%) |
| 18 | 25.519 | 3.4876 | 179 | 22 | 2115 | 19.8 |
| 19 | 26.378 | 3.376 | 30 | 3.7 | 238 | 2.2 |
| 20 | 26.83 | 3.3201 | 93 | 11.4 | 973 | 9.1 |
| 21 | 27.37 | 3.2558 | 54 | 6.6 | 898 | 8.4 |
| 22 | 27.565 | 3.2332 | 76 | 9.3 | 824 | 7.7 |
| 23 | 27.976 | 3.1867 | 88 | 10.8 | 1059 | 9.9 |
| 24 | 28.72 | 3.1058 | 86 | 10.6 | 1000 | 9.4 |
| 25 | 29.046 | 3.0717 | 142 | 17.4 | 3001 | 28.1 |
| 26 | 29.415 | 3.0339 | 62 | 7.6 | 1398 | 13.1 |
| 27 | 30.331 | 2.9444 | 117 | 14.4 | 1350 | 12.6 |
| 28 | 31.229 | 2.8617 | 87 | 10.7 | 693 | 6.5 |
| 29 | 32.206 | 2.7771 | 32 | 3.9 | 443 | 4.1 |
| 30 | 32.574 | 2.7466 | 85 | 10.4 | 1392 | 13 |
| 31 | 35.12 | 2.5531 | 106 | 13 | 2649 | 24.8 |
| 32 | 35.702 | 2.5128 | 51 | 6.3 | 859 | 8 |
| 33 | 36.327 | 2.471 | 57 | 7 | 830 | 7.8 |
| 34 | 37.586 | 2.3911 | 30 | 3.7 | 493 | 4.6 |
| 35 | 39.644 | 2.2716 | 34 | 4.2 | 520 | 4.9 |
| 36 | 44.399 | 2.0387 | 42 | 5.2 | 398 | 3.7 |

In certain embodiments of the present invention, the crystal form D has an X-ray powder diffraction pattern substantially as shown in FIG. 31; and has a DSC pattern substantially as shown in FIG. 32.

In certain embodiments of the present invention, the crystal form is crystal form E of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form E has an X-ray powder diffraction pattern comprising diffraction peaks at 2θ (±0.2°) of 12.3±0.2°, 16.6±0.2°, 20.0±0.2°, 21.2+0.2°, 21.6±0.2° and 24.4±0.2°; preferably, the X-ray powder diffraction pattern further comprises diffraction peaks at 2θ (±0.2°) of 8.1±0.2° and 24.1±0.2°; more preferably, the X-ray powder diffraction pattern further comprises diffraction peaks at 2θ (±0.2°) of 16.0±0.2° and 19.8±0.2°.

In certain embodiments of the present invention, by using Cu-Kα radiation, the X-ray characteristic diffraction peaks of the crystal form E represented by 2θ angles and interplanar spacing d values are as shown in Table 17.

Table 17

| No. | XRPD data of crystal form E of free base | | | | | |
|---|---|---|---|---|---|---|
| | 2θ (+0.2°) | d value | Peak height | Relative intensity (I%) | Area | Relative intensity (I%) |
| 1 | 5.178 | 17.0533 | 74 | 8.1 | 296 | 2.7 |
| 2 | 8.074 | 10.9409 | 337 | 37.1 | 3605 | 32.7 |
| 3 | 11.971 | 7.3872 | 182 | 20 | 2300 | 20.9 |
| 4 | 12.331 | 7.1719 | 815 | 89.7 | 9683 | 87.9 |
| 5 | 13.386 | 6.6092 | 152 | 16.7 | 1396 | 12.7 |
| 6 | 15.435 | 5.736 | 273 | 30 | 2731 | 24.8 |
| 7 | 16.044 | 5.5197 | 285 | 31.4 | 3201 | 29.1 |
| 8 | 16.649 | 5.3202 | 446 | 49.1 | 5029 | 45.7 |

(continued)

| No. | XRPD data of crystal form E of free base | | | | | |
|---|---|---|---|---|---|---|
| | 2θ (+0.2°) | d value | Peak height | Relative intensity (I%) | Area | Relative intensity (I%) |
| 9 | 17.786 | 4.9827 | 40 | 4.4 | 317 | 2.9 |
| 10 | 18.78 | 4.7213 | 208 | 22.9 | 1895 | 17.2 |
| 11 | 19.13 | 4.6355 | 31 | 3.4 | 175 | 1.6 |
| 12 | 19.753 | 4.4907 | 168 | 18.5 | 2997 | 27.2 |
| 13 | 20.019 | 4.4318 | 376 | 41.4 | 5763 | 52.3 |
| 14 | 21.17 | 4.1933 | 909 | 100 | 10575 | 96 |
| 15 | 21.638 | 4.1035 | 575 | 63.3 | 11012 | 100 |
| 16 | 22.957 | 3.8707 | 61 | 6.7 | 507 | 4.6 |
| 17 | 24.093 | 3.6908 | 138 | 15.2 | 3660 | 33.2 |
| 18 | 24.352 | 3.6521 | 294 | 32.3 | 4709 | 42.8 |
| 19 | 25.186 | 3.533 | 157 | 17.3 | 1861 | 16.9 |
| 20 | 25.636 | 3.4721 | 175 | 19.3 | 1931 | 17.5 |
| 21 | 26.012 | 3.4226 | 49 | 5.4 | 437 | 4 |
| 22 | 27.004 | 3.2991 | 77 | 8.5 | 1601 | 14.5 |
| 23 | 27.642 | 3.2244 | 111 | 12.2 | 1760 | 16 |
| 24 | 28.592 | 3.1194 | 147 | 16.2 | 1735 | 15.8 |
| 25 | 29.344 | 3.0411 | 127 | 14 | 3006 | 27.3 |
| 26 | 29.907 | 2.9852 | 29 | 3.2 | 762 | 6.9 |
| 27 | 30.462 | 2.9321 | 88 | 9.7 | 664 | 6 |
| 28 | 30.881 | 2.8932 | 53 | 5.8 | 487 | 4.4 |
| 29 | 31.41 | 2.8457 | 49 | 5.4 | 608 | 5.5 |
| 30 | 31.657 | 2.824 | 38 | 4.2 | 637 | 5.8 |
| 31 | 32.305 | 2.7689 | 60 | 6.6 | 798 | 7.2 |
| 32 | 32.601 | 2.7444 | 32 | 3.5 | 773 | 7 |
| 33 | 33.987 | 2.6355 | 61 | 6.7 | 1442 | 13.1 |
| 34 | 35.811 | 2.5054 | 48 | 5.3 | 319 | 2.9 |
| 35 | 36.216 | 2.4783 | 59 | 6.5 | 1186 | 10.8 |
| 36 | 36.698 | 2.4468 | 64 | 7 | 909 | 8.3 |
| 37 | 37.513 | 2.3955 | 39 | 4.3 | 449 | 4.1 |
| 38 | 38.303 | 2.348 | 41 | 4.5 | 766 | 7 |

In certain embodiments of the present invention, the crystal form E has an X-ray powder diffraction pattern substantially as shown in FIG. 33; and has a DSC pattern substantially as shown in FIG. 34.

In certain embodiments of the present invention, the crystal form is crystal form F of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form F has an X-ray powder diffraction pattern comprising diffraction peaks at 20 (±0.2°) of 14.2±0.2°, 17.2±0.2°, 19.7±0.2°, 21.2±0.2°, 25.3±0.2° and 26.2±0.2°; preferably, the X-ray powder diffraction pattern further comprises diffraction peaks at 2θ (±0.2°) of 6.6±0.2° and 23.3±0.2°; more preferably, the X-ray powder diffraction pattern further comprises diffraction peaks at 2θ (±0.2°) of 15.1±0.2° and 18.1±0.2°.

In certain embodiments of the present invention, by using Cu-Kα radiation, the X-ray characteristic diffraction peaks of the crystal form F represented by 20 angles and interplanar spacing d values are as shown in Table 18.

Table 18

| No. | XRPD data of crystal form F of free base | | | | | |
|---|---|---|---|---|---|---|
| | 2θ (+0.2°) | d value | Peak height | Relative intensity (I%) | Area | Relative intensity (I%) |
| 1 | 5.403 | 16.3427 | 174 | 34.1 | 827 | 14.5 |
| 2 | 6.553 | 13.4765 | 439 | 85.9 | 3751 | 65.9 |
| 3 | 8.784 | 10.0583 | 347 | 67.9 | 3122 | 54.8 |
| 4 | 11.604 | 7.6198 | 153 | 29.9 | 1279 | 22.5 |
| 5 | 12.547 | 7.0492 | 101 | 19.8 | 342 | 6 |
| 6 | 13.082 | 6.762 | 190 | 37.2 | 1325 | 23.3 |
| 7 | 13.81 | 6.4071 | 123 | 24.1 | 1127 | 19.8 |
| 8 | 14.197 | 6.2334 | 365 | 71.4 | 5133 | 90.2 |
| 9 | 15.052 | 5.8811 | 511 | 100 | 3649 | 64.1 |
| 10 | 15.373 | 5.7589 | 273 | 53.4 | 2657 | 46.7 |
| 11 | 16.046 | 5.5191 | 214 | 41.9 | 1935 | 34 |
| 12 | 16.815 | 5.2682 | 297 | 58.1 | 3406 | 59.8 |
| 13 | 17.156 | 5.1643 | 311 | 60.9 | 4293 | 75.4 |
| 14 | 18.111 | 4.8941 | 244 | 47.7 | 3742 | 65.7 |
| 15 | 18.518 | 4.7874 | 137 | 26.8 | 1481 | 26 |
| 16 | 19.328 | 4.5886 | 106 | 20.7 | 2261 | 39.7 |
| 17 | 19.653 | 4.5133 | 382 | 74.8 | 5690 | 100 |
| 18 | 20.3 | 4.371 | 177 | 34.6 | 1338 | 23.5 |
| 19 | 21.229 | 4.1817 | 166 | 32.5 | 3797 | 66.7 |
| 20 | 21.998 | 4.0373 | 52 | 10.2 | 363 | 6.4 |
| 21 | 22.384 | 3.9685 | 88 | 17.2 | 873 | 15.3 |
| 22 | 23.261 | 3.8209 | 328 | 64.2 | 3767 | 66.2 |
| 23 | 23.987 | 3.7069 | 109 | 21.3 | 1360 | 23.9 |
| 24 | 25.326 | 3.5138 | 296 | 57.9 | 5692 | 100 |
| 25 | 26.22 | 3.396 | 257 | 50.3 | 4073 | 71.6 |
| 26 | 26.832 | 3.3199 | 105 | 20.5 | 1105 | 19.4 |
| 27 | 27.607 | 3.2284 | 44 | 8.6 | 964 | 16.9 |
| 28 | 27.884 | 3.1969 | 58 | 11.4 | 965 | 17 |
| 29 | 28.523 | 3.1268 | 44 | 8.6 | 426 | 7.5 |
| 30 | 28.754 | 3.1022 | 63 | 12.3 | 1140 | 20 |
| 31 | 29.141 | 3.0619 | 74 | 14.5 | 2548 | 44.8 |
| 32 | 29.343 | 3.0413 | 87 | 17 | 2058 | 36.2 |
| 33 | 30.353 | 2.9423 | 43 | 8.4 | 631 | 11.1 |
| 34 | 30.922 | 2.8895 | 79 | 15.5 | 2048 | 36 |
| 35 | 32.147 | 2.7821 | 34 | 6.7 | 237 | 4.2 |
| 36 | 36.903 | 2.4337 | 35 | 6.8 | 311 | 5.5 |
| 37 | 38.293 | 2.3485 | 48 | 9.4 | 1157 | 20.3 |

In certain embodiments of the present invention, the crystal form F has an X-ray powder diffraction pattern

substantially as shown in FIG. 35; and has a DSC pattern substantially as shown in FIG. 36.

In certain embodiments of the present invention, the crystal form is crystal form G of N-((2R,35)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein the crystal form G has an X-ray powder diffraction pattern comprising diffraction peaks at 20 (±0.2°) of 10.4±0.2°, 16.3±0.2°, 18.8+0.2°, 21.3±0.2°, 22.2±0.2° and 23.9±0.2°; preferably, the X-ray powder diffraction pattern further comprises diffraction peaks at 2θ (±0.2°) of 9.4±0.2° and 25.7±0.2°; more preferably, the X-ray powder diffraction pattern further comprises diffraction peaks at 2θ (±0.2°) of 16.6±0.2° and 23.2±0.2°.

In certain embodiments of the present invention, by using Cu-Kα radiation, the X-ray characteristic diffraction peaks of the crystal form G represented by 20 angles and interplanar spacing d values are as shown in Table 19.

Table 19

| No. | XRPD data of crystal form G of free base | | | | | |
|---|---|---|---|---|---|---|
| | 2θ (+0.2°) | d value | Peak height | Relative intensity (I%) | Area | Relative intensity (I%) |
| 1 | 7.384 | 11.9614 | 124 | 5.5 | 838 | 4.7 |
| 2 | 9.368 | 9.4328 | 546 | 24.1 | 4444 | 24.7 |
| 3 | 10.408 | 8.4925 | 2268 | 100 | 17991 | 100 |
| 4 | 11.591 | 7.6282 | 66 | 2.9 | 356 | 2 |
| 5 | 12.979 | 6.8153 | 93 | 4.1 | 617 | 3.4 |
| 6 | 13.898 | 6.3669 | 40 | 1.8 | 262 | 1.5 |
| 7 | 15.454 | 5.729 | 87 | 3.8 | 1421 | 7.9 |
| 8 | 15.868 | 5.5806 | 78 | 3.4 | 1077 | 6 |
| 9 | 16.326 | 5.4248 | 653 | 28.8 | 7735 | 43 |
| 10 | 16.588 | 5.34 | 304 | 13.4 | 4099 | 22.8 |
| 11 | 17.401 | 5.0921 | 143 | 6.3 | 1107 | 6.2 |
| 12 | 18.099 | 4.8972 | 30 | 1.3 | 144 | 0.8 |
| 13 | 18.796 | 4.7172 | 952 | 42 | 10415 | 57.9 |
| 14 | 19.671 | 4.5093 | 192 | 8.5 | 1487 | 8.3 |
| 15 | 20.258 | 4.38 | 369 | 16.3 | 3198 | 17.8 |
| 16 | 20.645 | 4.2987 | 144 | 6.3 | 2041 | 11.3 |
| 17 | 21.295 | 4.169 | 469 | 20.7 | 10632 | 59.1 |
| 18 | 22.185 | 4.0036 | 1232 | 54.3 | 9954 | 55.3 |
| 19 | 23.22 | 3.8275 | 482 | 21.3 | 3487 | 19.4 |
| 20 | 23.867 | 3.7252 | 724 | 31.9 | 6079 | 33.8 |
| 21 | 24.479 | 3.6335 | 401 | 17.7 | 3158 | 17.6 |
| 22 | 25.673 | 3.4671 | 472 | 20.8 | 5769 | 32.1 |
| 23 | 26.097 | 3.4117 | 158 | 7 | 2527 | 14 |
| 24 | 26.51 | 3.3595 | 53 | 2.3 | 295 | 1.6 |
| 25 | 27.173 | 3.279 | 75 | 3.3 | 322 | 1.8 |
| 26 | 27.562 | 3.2336 | 178 | 7.8 | 1815 | 10.1 |
| 27 | 27.884 | 3.197 | 194 | 8.6 | 2483 | 13.8 |
| 28 | 28.694 | 3.1086 | 72 | 3.2 | 618 | 3.4 |
| 29 | 29.08 | 3.0682 | 98 | 4.3 | 1155 | 6.4 |
| 30 | 29.545 | 3.0209 | 58 | 2.6 | 462 | 2.6 |
| 31 | 30.114 | 2.9652 | 119 | 5.2 | 1928 | 10.7 |

(continued)

| No. | XRPD data of crystal form G of free base | | | | | |
|---|---|---|---|---|---|---|
| | 2θ (+0.2°) | d value | Peak height | Relative intensity (I%) | Area | Relative intensity (I%) |
| 32 | 30.457 | 2.9325 | 54 | 2.4 | 846 | 4.7 |
| 33 | 31.061 | 2.8768 | 145 | 6.4 | 1568 | 8.7 |
| 34 | 31.446 | 2.8425 | 109 | 4.8 | 1476 | 8.2 |
| 35 | 31.79 | 2.8125 | 87 | 3.8 | 775 | 4.3 |
| 36 | 32.747 | 2.7325 | 38 | 1.7 | 636 | 3.5 |
| 37 | 33.391 | 2.6812 | 80 | 3.5 | 618 | 3.4 |
| 38 | 33.917 | 2.6408 | 96 | 4.2 | 1034 | 5.7 |
| 39 | 34.918 | 2.5674 | 81 | 3.6 | 1128 | 6.3 |
| 40 | 37.41 | 2.4019 | 35 | 1.5 | 440 | 2.4 |
| 41 | 38.89 | 2.3138 | 29 | 1.3 | 251 | 1.4 |

In certain embodiments of the present invention, the crystal form G has an X-ray powder diffraction pattern substantially as shown in FIG. 37; and has a DSC pattern substantially as shown in FIG. 38.

In certain embodiments of the present invention, in the X-ray powder diffraction patterns of the crystal form A, the crystal form B, the crystal form C, the crystal form D, the crystal form E, the crystal form F, and the crystal form G of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, the positions of the diffraction peaks with the top ten relative peak intensities have a 2θ deviation of ±0.2° to ±0.5°, preferably ±0.2° to ±0.3°, and most preferably ±0.2° from the corresponding positions of the diffraction peaks in FIG. 25, FIG. 27, FIG. 29, FIG. 31, FIG. 33, FIG. 35, and FIG. 37, respectively. The present invention also provides a method for preparing the above-mentioned acid addition salt, wherein the method involves method I or method II:

method I comprises the following steps:

1) dissolving the compound in an organic solvent;
2) adding a counterion acid, wherein the amount of the counterion acid is preferably 1.2 equivalents; the counterion acid can be dissolved in an organic solvent;
3) stirring and mixing, and evaporating the solvent;
4) performing separation to obtain the acid addition salt;

method II comprises the following steps:

1) suspending the compound in an organic solvent;
2) adding a counterion acid, wherein the amount of the counterion acid is preferably 1.2 equivalents; the counterion acid can be dissolved in an organic solvent;
3) stirring until dissolution, and then further stirring to allow precipitation;
4) performing separation to obtain the acid addition salt;

wherein: the solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, ethanol, acetone or ethyl acetate;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic

acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid or L-malic acid.

The present invention also provides a method for preparing the above-mentioned crystal form of the acid addition salt, wherein the method involves method I or method II:

method I comprises the following steps:

1) weighing an appropriate amount of the compound and dissolving the compound in a good solvent;
2) weighing an appropriate amount of a counterion acid and dissolving the counterion acid in an organic solvent, wherein the amount of the counterion acid is preferably 1.2 equivalents;
3) combining the two solutions, and stirring to allow precipitation, or dropwise adding a poor solvent and then stirring to allow precipitation;
4) subjecting the mixture to rapid centrifugation or to standing followed by blow-drying to obtain the crystal form,

wherein: the good solvent is selected from methanol, ethanol, acetone, ethyl acetate, 2-butanone, 3-pentanone or 1,4-dioxane, preferably acetone, 2-butanone, 3-pentanone or ethyl acetate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, ethanol, acetone or ethyl acetate; the good solvent and the organic solution are mutually miscible when used;
the poor solvent is selected from heptane, water, methyl tert-butyl ether, isopropyl ether, etc.;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid or L-malic acid;
method II comprises the following steps:

1) weighing an appropriate amount of the compound and dissolving the compound in a solvent;
2) mixing with a counterion acid to obtain the crystal form, wherein the amount of the counterion acid is preferably 1.2 equivalents;

the solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, ethanol, acetone or ethyl acetate;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric

acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid or L-malic acid.

In certain embodiments of the present invention, the method for preparing the crystal form A of phosphate comprises the following steps:

1) dissolving the compound in an ester solvent;
2) adding a solution of phosphoric acid in an alcohol to the solution obtained from step 1);
3) stirring the mixture to obtain the crystal form A of phosphate,

wherein the ester solvent is preferably methyl formate, ethyl formate, methyl acetate or ethyl acetate, preferably ethyl acetate;
the alcohol is preferably methanol, ethanol, n-propanol, isopropanol or n-butanol, preferably methanol.

The present invention also provides a method for preparing the above-mentioned crystal form, wherein the method involves method I or method II:

method I comprises the following steps:

1) weighing an appropriate amount of the compound, and dissolving the compound in a good solvent with heating;
2) quickly placing the resulting solution at room temperature, and stirring same until a solid precipitates;
3) quickly centrifuging the resulting suspension, removing the supernatant, and drying the residual solid in a vacuum drying oven at 40°C to a constant weight to obtain a target crystal form,

wherein: the good solvent is selected from methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butyl alcohol, 2-butanone, 3-pentanone, N-methylpyrrolidone, or dimethyl sulphoxide, preferably tetrahydrofuran, dichloromethane, 2-methyl-tetrahydrofuran and 3-pentanone;
method II comprises the following steps:

1) weighing an appropriate amount of a free base, and dissolving the free base in a good solvent;
2) adding an anti-solvent to the resulting solution at a given temperature, preferably at 0°C-25°C, and stirring until a solid precipitates;
3) quickly centrifuging the resulting suspension, removing the supernatant, and drying the residual solid in a vacuum drying oven at 40°C to a constant weight to obtain a target product,

wherein: the good solvent is selected from acetone, 88% acetone, tetrahydrofuran, 2-methyltetrahydrofuran, dichloromethane, 1,4-dioxane, toluene, isopropanol, n-butanol, isobutanol, N,N-dimethylformamide, N,N-dimethylacetamide, n-propanol, tert-butyl alcohol, 2-butanone, 3-pentanone, N-methylpyrrolidone, ethyl formate, or dimethyl sulphoxide, preferably acetone, dichloromethane, tetrahydrofuran, 2-methyltetrahydrofuran, ethyl formate, butanone, 3-pentanone, or 1,4-dioxane.

The poor solvent is selected from heptane, water, methyl tert-butyl ether, or isopropyl ether.
The present invention also provides a pharmaceutical composition comprising a therapeutically effective dose of the above-mentioned acid addition salt or the crystal form thereof, and one or more pharmaceutically acceptable carriers, diluents or excipients.
The present invention also provides the use of the above-mentioned acid addition salt, the above-mentioned crystal form, or the above-mentioned pharmaceutical composition in the preparation of an EGFR inhibitor drug.
In certain embodiments of the present invention, the EGFR is a mutated EGFR, preferably comprising one or more mutations of Dell9, L858R, T790M or C797S, and more preferably comprising mutations L858R/T790M, Dell9/T790M, Dell9/C797S, L858R/C797S, Del19/T790M/C797S or L858R/T790M/C797S.
The present invention also provides the use of the above-mentioned acid addition salt, the above-mentioned crystal form, or the above-mentioned pharmaceutical composition in the preparation of a medicament for treating cancer, wherein preferably, the cancer is selected from ovarian cancer, cervical cancer, colorectal cancer, breast cancer,

pancreatic cancer, glioma, glioblastoma, melanoma, prostate cancer, leukaemia, lymphoma, non-Hodgkin's lymphoma, gastric cancer, lung cancer, hepatocellular carcinoma, gastric cancer, gastrointestinal stromal tumour, thyroid cancer, bile duct cancer, endometrial cancer, renal cancer, anaplastic large cell lymphoma, multiple myeloma, melanoma or mesothelioma; more preferably, the cancer is non-small cell lung cancer; further preferably, the cancer is a non-small cell lung cancer with EGFR mutations of L858R/T790M, Del19/T790M, Del19/C797S, L858R/C797S, Del19/T790M/C797S or L858R/T790M/C797S.

**Brief Description of the Drawings**

FIG. 1 shows an XRPD diagram of crystal form A of phosphate.
FIG. 2 shows a DSC diagram of crystal form A of phosphate.
FIG. 3 shows an XRPD diagram of crystal form B of phosphate.
FIG. 4 shows a DSC diagram of crystal form B of phosphate.
FIG. 5 shows an XRPD diagram of crystal form A of hydrochloride.
FIG. 6 shows a DSC diagram of crystal form A of hydrochloride.
FIG. 7 shows an XRPD diagram of crystal form B of hydrochloride.
FIG. 8 shows a DSC diagram of crystal form B of hydrochloride.
FIG. 9 shows an XRPD diagram of crystal form A of sulphate.
FIG. 10 shows a DSC diagram of crystal form A of sulphate.
FIG. 11 shows an XRPD diagram of crystal form A of hydrobromide.
FIG. 12 shows a DSC diagram of crystal form A of hydrobromide.
FIG. 13 shows an XRPD diagram of crystal form B of hydrobromide.
FIG. 14 shows a DSC diagram of crystal form B of hydrobromide.
FIG. 15 shows an XRPD diagram of crystal form A of citrate.
FIG. 16 shows a DSC diagram of crystal form A of citrate.
FIG. 17 shows an XRPD diagram of crystal form A of oxalate.
FIG. 18 shows a DSC diagram of crystal form A of oxalate.
FIG. 19 shows an XRPD diagram of crystal form A of maleate.
FIG. 20 shows a DSC diagram of crystal form A of maleate.
FIG. 21 shows an XRPD diagram of crystal form A of salicylate.
FIG. 22 shows a DSC diagram of crystal form A of salicylate.
FIG. 23 shows an XRPD diagram of crystal form A of p-hydroxybenzoate.
FIG. 24 shows a DSC diagram of crystal form A of p-hydroxybenzoate.
FIG. 25 shows an XRPD diagram of crystal form A of free base.
FIG. 26 shows a DSC diagram of crystal form A of free base.
FIG. 27 shows an XRPD diagram of crystal form B of free base.
FIG. 28 shows a DSC diagram of crystal form B of free base.
FIG. 29 shows an XRPD diagram of crystal form C of free base.
FIG. 30 shows a DSC diagram of crystal form C of free base.
FIG. 31 shows an XRPD diagram of crystal form D of free base.
FIG. 32 shows a DSC diagram of crystal form D of free base.
FIG. 33 shows an XRPD diagram of crystal form E of free base.
FIG. 34 shows a DSC diagram of crystal form E of free base.
FIG. 35 shows an XRPD diagram of crystal form F of free base.
FIG. 36 shows a DSC diagram of crystal form F of free base.
FIG. 37 shows an XRPD diagram of crystal form G of free base.
FIG. 38 shows a DSC diagram of crystal form G of free base.

**Detailed Description of Embodiments**

The present invention will be further described below in conjunction with examples, but these examples are not meant to limit the scope of the present invention.

**I. Compound Examples**

The structure of the compound of the present invention is determined by nuclear magnetic resonance (NMR)

or/and liquid chromatography-mass spectrometry (LC-MS). NMR chemical shift (δ) is given in parts per million (ppm). NMR is determined with Bruker AVANCE-400 nuclear magnetic resonance instrument; the solvents for determination are deuterated dimethyl sulphoxide (DMSO-$d_6$), deuterated methanol ($CD_3OD$) and deuterated chloroform ($CDCl_3$); and the internal standard is tetramethylsilane (TMS).

Liquid chromatography-mass spectrometry LC-MS is determined by Agilent 1200 Infinity Series mass spectrometer. For determination by HPLC, Agilent 1200DAD high-pressure liquid chromatograph (Sunfire C18 150 × 4.6 mm chromatographic column) and Waters 2695-2996 high-pressure liquid chromatograph (Gimini $C_{18}$ 150 × 4.6 mm chromatographic column) are used.

Yantai Huanghai HSGF254 or Qingdao GF254 silica gel plate is used as a thin layer chromatography silica plate; the specification used for the TLC is 0.15-0.20 mm; and the specification when separating and purifying a product by thin layer chromatography is 0.4-0.5 mm. Yantai Huanghai silica gel of 200-300 mesh silica gel is generally used as a carrier in column chromatography.

The starting materials in the examples of the present invention are known and can be purchased on the market, or can be synthesized using or according to methods known in the art.

Unless otherwise specified, all reactions in the present invention are carried out with continuous magnetic stirring in a dry nitrogen or argon atmosphere, using dried solvents and with the reaction temperature expressed in degrees Celsius.

Intermediate 1

3-((methanesulphonyl)methyl)azetidine

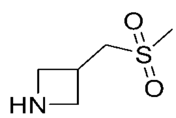

Step 1 Synthesis of tert-butyl 3-((methylthio)methyl)azetidine-1-carboxylate

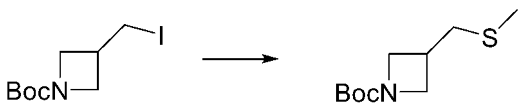

Tert-butyl 3-(iodomethyl)azetidine-1-carboxylate (2 g, 6.73 mmol) and sodium methyl mercaptide (970 mg, 13.50 mmol) were dissolved in ACN (15 mL) and $H_2O$ (5 mL), and the mixture was warmed to 60°C and reacted for 12 hours. The reaction solution was cooled to room temperature, concentrated under reduced pressure, and then separated by column chromatography to obtain the target compound tert-butyl 3-((methylthio)methyl)azetidine-1-carboxylate (1.30 g, 88.9%). MS m/z (ESI):162.0 $[M+H-56]^+$.

Step 2 Synthesis of tert-butyl 3-((methanesulphonyl)methyl)azetidine-1-carboxylate

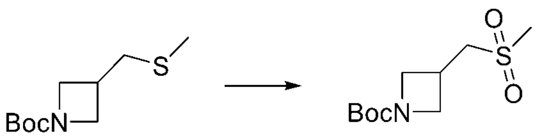

Tert-butyl 3-((methylthio)methyl)azetidine-1-carboxylate (1.30 g, 5.99 mmol) was dissolved in dichloromethane (15 mL), m-chloroperoxybenzoic acid (2 g, 12 mmol) was added and the mixture was stirred overnight. The reaction solution was washed with saturated sodium thiosulphate solution and saturated sodium chloride solution, and the organic phase was dried over anhydrous sodium sulphate, concentrated under reduced pressure and separated by column chromatography to obtain the target compound tert-butyl 3-((methanesulphonyl)methyl)azetidine-1-carboxylate (1.30 g,

87.1%). MS m/z (ESI):194.0 [M+H-56]+.

Step 3 Synthesis of 3-((methanesulphonyl)methyl)azetidine trifluoroacetate

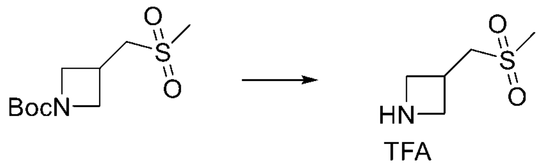

Tert-butyl 3-((methanesulphonyl)methyl)azetidine-1-carboxylate (1.30 g, 5.22 mmol) was dissolved in dichloromethane (15 mL), TFA (3 mL) was added, and the mixture was stirred for 4 hours. The reaction solution was concentrated under reduced pressure to obtain the crude target compound 3-((methanesulphonyl)methyl)azetidine trifluoroacetate (740 mg). MS m/z (ESI): 150.0 [M+H]+.

Intermediate 2

(2R,3S)-2-methyl-3-((methanesulphonyl)methyl)azetidine

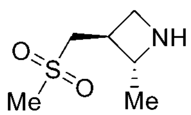

Step 1 Synthesis of (2R,3S)-1-diphenylmethyl-2-methylazetidin-3-yl methanesulphonate

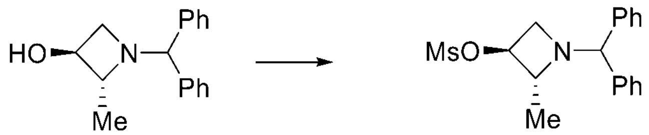

(2R,3S)-1-Diphenylmethyl-2-methylazetidin-3-ol (10 g, 39.50 mmol) was dissolved in dichloromethane (100 mL), triethylamine (4.80 g, 47.30 mmol) was added, the reaction solution was cooled to 0°C, methanesulphonyl chloride (5 g, 43.40 mmol) was slowly added dropwise, and the mixture was allowed to react at room temperature overnight. The reaction was quenched by adding water, the reaction solution was subjected to phase separation with dichloromethane and water, and the organic phase was dried over anhydrous sodium sulphate, and concentrated under reduced pressure to obtain the crude target compound (2R, 3S)-1-diphenylmethyl-2-methylazetidin-3-yl methanesulphonate (12.80 g). MS m/z (ESI): 332.2 [M+H]+.

Step 2 Synthesis of methyl (S)-2-((2R,3S)-1-diphenylmethyl-2-methylazetidin-3-yl)-2-(methanesulphonyl)acetate

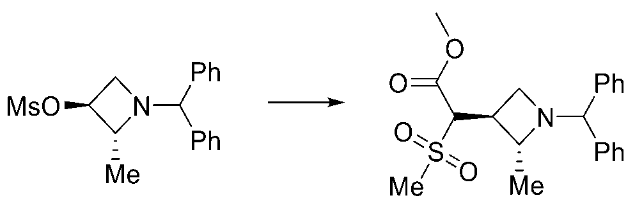

(2R,3S)-1-Diphenylmethyl-2-methylazetidin-3-yl methanesulphonate (12.80 g, 38.62 mmol) and methyl

2-(methylsulphonyl)acetate (7.70 g, 50.60 mmol) were dissolved in DMF (100 mL), sodium hydride (2.20 g, 60% in mineral oil, 55.50 mmol) was added in batches, and the mixture was reacted at room temperature for 15 minutes, and then warmed to 80°C and reacted overnight. The reaction solution was cooled to room temperature and quenched with a saturated ammonium chloride solution, the reaction solution was subjected to phase separation with ethyl acetate and water, and the organic phase was dried over anhydrous sodium sulphate, concentrated and separated by column chromatography to obtain the target compound methyl (S)-2-((2R,3S)-1-diphenylmethyl-2-methylazetidin-3-yl)-2-(methanesulphonyl)acetate (11.60 g, 77.5%). MS m/z (ESI): 388.2 $[M+H]^+$.

Step 3 Synthesis of (2R,3S)-1-diphenylmethyl-2-methyl-3-((methanesulphonyl)methyl)azetidine

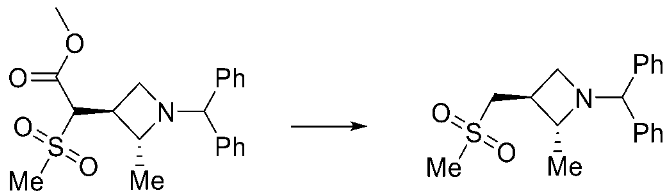

Methyl (S)-2-((2R,3S)-1-diphenylmethyl-2-methylazetidin-3-yl)-2-(methanesulphonyl)acetate (11.60 g, 29.94 mmol) was dissolved in DMA (120 mL), lithium chloride (10.50 g, 247.50 mmol) was added, and the mixture was warmed to 150°C and reacted for 2 hours. The mixture was cooled to room temperature, the reaction solution was subjected to phase separation with ethyl acetate and water, and the organic phase was dried over anhydrous sodium sulphate, concentrated under reduced pressure and separated by column chromatography to obtain the target compound (2R,3S)-1-diphenylmethyl-2-methyl-3-((methanesulphonyl)methyl) azetidine (9.20 g, 93.3%). MS m/z (ESI): 330.0 $[M+H]^+$.

Step 4 Synthesis of (2R,3S)-2-methyl-3-((methanesulphonyl)methyl)azetidine

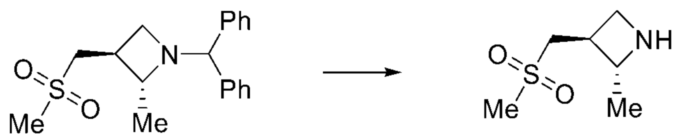

(2R,3S)-1-Diphenylmethyl-2-methyl-3-((methanesulphonyl)methyl)azetidine (9.20 g, 27.92 mmol) was dissolved in methanol (120 mL), TFA (5 mL) and palladium hydroxide (2.80 g) were added, and the reaction system was evacuated and filled with hydrogen. The operations were repeated three times, and the reaction solution was reacted overnight under hydrogen atmosphere. The reaction solution was filtered, and the filtrate was concentrated under reduced pressure to obtain the crude target compound (2R,3S)-2-methyl-3-((methanesulphonyl)methyl)azetidine (4 g). MS m/z (ESI): 164.0 $[M+H]^+$.

Intermediate 3

8-Bromo-3-chloro-6-fluoro-5-isopropylisoquinoline

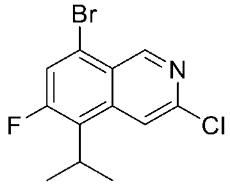

Step 1 Synthesis of 3-chloro-6-fluoroisoquinoline

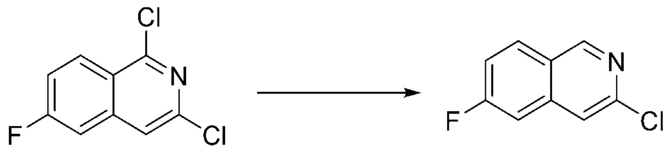

1,3-Dichloro-6-fluoroisoquinoline (7.50 g, 34.72 mmol) was dissolved in glacial acetic acid (40 mL) and hydriodic acid (20 mL, 45% aqueous), red phosphorus (2.69 g, 86.8 mmol) was added, and the mixture was warmed to 100°C and reacted for 4 hours. The reaction solution was cooled to room temperature, concentrated, diluted with dichloromethane (100 mL), and washed with an aqueous saturated sodium carbonate solution, the organic phase was separated, dried over anhydrous sodium sulphate, and filtered, and the organic solvent was concentrated under reduced pressure and separated by column chromatography to obtain the target compound 3-chloro-6-fluoroisoquinoline (4.90 g, 77.8%). MS m/z (ESI): 182.0 $[M+H]^+$.

Step 2 Synthesis of 5-bromo-3-chloro-6-fluoroisoquinoline

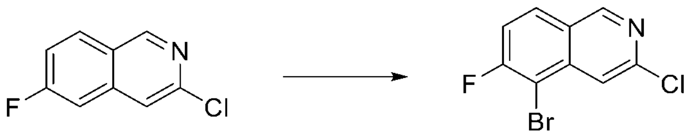

3-Chloro-6-fluoroisoquinoline (3.20 g, 17.62 mmol) was dissolved in concentrated sulphuric acid (20 mL), NBS (3.45 g, 19.38 mmol) was added, and the mixture was stirred at room temperature overnight. The reaction solution was slowly added to ice water, the reaction solution was subjected to phase separation with ethyl acetate and water, and the organic phase was separated and dried over anhydrous sodium sulphate. After filtration, the organic solvent was concentrated under reduced pressure and separated by column chromatography to obtain the target compound 5-bromo-3-chloro-6-fluoroisoquinoline (4.0 g, 87.1%). MS m/z (ESI): 260.0 $[M+H]^+$.

Step 3 Synthesis of 3-chloro-6-fluoro-5-(prop-1-en-2-yl)isoquinoline

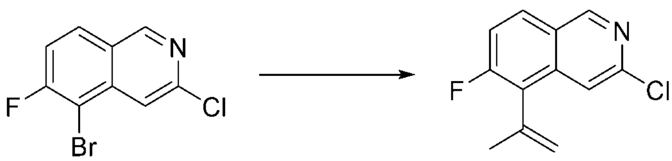

5-Bromo-3-chloro-6-fluoroisoquinoline (4.0 g, 15.36 mmol) and 4,4,5,5-tetramethyl-2-(prop-1-en-2-yl)-1,3,2-dioxaborolane (2.71 g, 16.72 mmol) were dissolved in 1,4-dioxane (20 mL) and water (3 mL), [1,1'-bis(diphenylphosphine) ferrocene]palladium dichloride (562 mg, 0.77 mmol) and caesium carbonate (10.01 g, 30.71 mmol) were added, and the mixture was warmed to 80°C and reacted for 2 hours. The reaction solution was cooled to room temperature, concentrated under reduced pressure, and then separated by column chromatography to obtain the target compound 3-chloro-6-fluoro-5-(prop-1-en-2-yl)isoquinoline (3.0 g, 88.1%). MS m/z (ESI): 222.0 $[M+H]^+$.

Step 4 Synthesis of 3-chloro-6-fluoro-5-isopropylisoquinoline

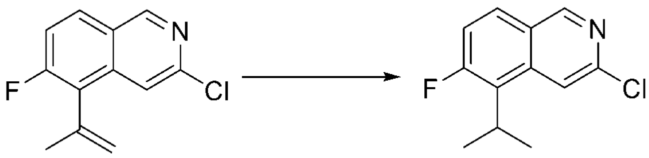

3-Chloro-6-fluoro-5-(prop-1-en-2-yl)isoquinoline (3.0 g, 13.53 mmol) was dissolved in ethyl acetate (30 mL), platinum dioxide (615 mg, 2.71 mmol) was added, and the mixture was stirred at room temperature for 4 hours under hydrogen atmosphere. The reaction solution was filtered, and the filtrate was concentrated under reduced pressure and separated by column chromatography to obtain the target compound 3-chloro-6-fluoro-5-isopropylisoquinoline (2.10 g, 69.4%). MS m/z (ESI): 224.1 $[M+H]^+$.

Step 5 Synthesis of 8-bromo-3-chloro-6-fluoro-5-isopropylisoquinoline

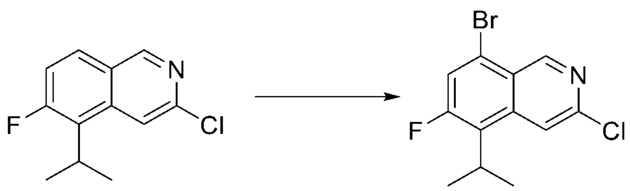

In an ice bath, to a solution of 3-chloro-6-fluoro-5-isopropylisoquinoline (800 mg, 3.58 mmol) in concentrated sulphuric acid solution (5 mL) was slowly added dibromohydantoin (1.12 g, 3.93 mmol) in batches, and the mixture was stirred in the ice bath for additional 0.5 hours. The reaction solution was carefully added to ice water and the reaction solution was subjected to phase separation with dichloromethane and water. The organic phase was dried over anhydrous sodium sulphate. After filtration, the organic solvent was concentrated under reduced pressure and separated by column chromatography to obtain the target compound 8-bromo-3-chloro-6-fluoro-5-isopropylisoquinoline (320 mg, 29.6%). MS m/z (ESI): 302.0 $[M+H]^+$.

Intermediate 4

N-methyl-N-((2R,3S)-2-methylazetidin-3-yl)methanesulphonamide trifluoroacetate

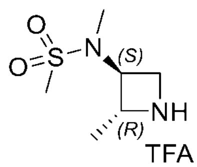

Step 1 Synthesis of (2R,3S)-1-diphenylmethyl-2-methylazetidin-3-yl methanesulphonate

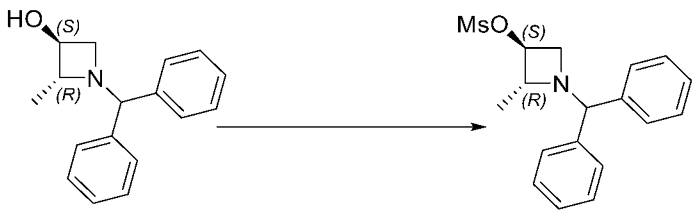

(2R,3S)-1-Diphenylmethyl-2-methylazetidin-3-ol (100 g, 397 mmol), triethylamine (110 mL, 793 mmol) and anhydrous dichloromethane (1000 mL) were added to a 2 L reaction bottle. The mixture was cooled to 5°C under nitrogen atmosphere, methanesulphonic anhydride (138 g, 795 mmol) was added in batches, and the mixture was warmed to room

temperature and stirred for 2 h. The reaction solution was subjected to phase separation with water and dichloromethane, and the organic phase was separated and washed sequentially with 5% sodium carbonate solution and a saturated sodium chloride solution. The organic phase was separated and dried over anhydrous sodium sulphate, and filtered, and the filtrate was concentrated under reduced pressure to obtain the crude target product (2R,3S)-1-diphenylmethyl-2-methylazetidin-3-yl methanesulphonate (138 g). MS m/z (ESI): 332.1[M+H]$^+$.

Step 2 Synthesis of N-((2R,3S)-1-diphenylmethyl-2-methylazetidin-3-yl)-N-methylmethanesulphonamide

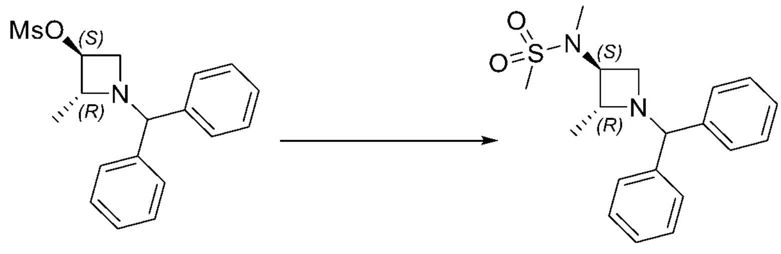

At room temperature, (2R,3S)-1-diphenylmethyl-2-methylazetidin-3-yl methanesulphonate (138 g, 0.42 mol) was dissolved in MeCN (950 mL), $Cs_2CO_3$ (273 g, 0.84 mol) and N-methylmethanesulphonamide (82 g, 0.75 mol) were added, and the mixture was stirred overnight at 80°C under nitrogen atmosphere. The reaction solution was cooled to room temperature, water (0.5 L) was added to the reaction solution, and the mixture was extracted with ethyl acetate (1 L x 2). The organic phases were combined and washed with an aqueous saturated sodium chloride solution, and separated to obtain the organic phases, which were dried over anhydrous sodium sulphate and filtered, and the filtrate was concentrated under reduced pressure to remove the organic solvent and separated by column chromatography to obtain the target product N-((2R,3S)-1-diphenylmethyl-2-methylazetidin-3-yl)-N-methylmethanesulphonamide (110 g, 76.7 %). MS m/z (ESI): 345.1 [M+H]$^+$.

Step 3 Synthesis of N-methyl-N-((2R,3S)-2-methylazetidin-3-yl)methanesulphonamide trifluoroacetate

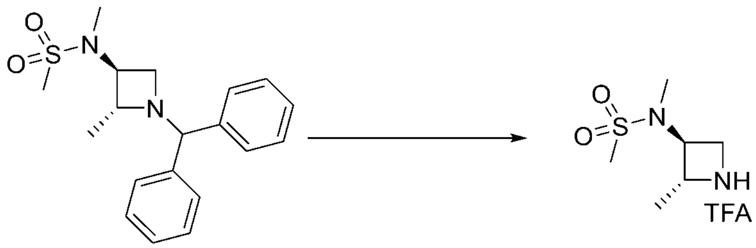

N-((2R,3S)-1-diphenylmethyl-2-methylazetidin-3-yl)-N-methylmethanesulphonamide (85 g, 0.247 mol) was dissolved in a mixed solution of MeOH (850 mL) and TFA (52 mL), 10% $Pd(OH)_2/C$ (28 g) was added, and the mixture was stirred overnight at room temperature under H2 atmosphere. The reaction solution was filtered over celite, and the filtrate was concentrated under reduced pressure to obtain the crude target product N-methyl-N-((2R,3S)-2-methylazetidin-3-yl)methanesulphonamide trifluoroacetate (66 g).

$^1$H NMR(400 MHz, DMSO-$d_6$) $\delta$ 8.88 (s, 1H),4.62-4.56 (m, 1H), 4.31-4.25 (m, 1H), 4.10-4.06 (m, 1H), 3.95-3.91 (m, 1H), 2.95 (s, 3H), 2.83 (s, 3H), 1.40 (d, $J$ = 6.0 Hz, 3H);
MS m/z (ESI) :179.1 [M+H]$^+$.

Intermediate 5

2-(3-Chloro-1-methyl-1H-pyrazol-4-yl)pyrimidin-4-amine

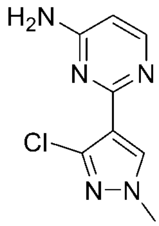

Step 1 Synthesis of 4-bromo-3-chloro-1-methyl-1H-pyrazole

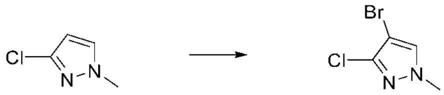

3-Chloro-1-methyl-1H-pyrazole (500 mg, 4.29 mmol) was dissolved in dichloromethane (10 mL) and cooled to 0°C, NBS (764 mg, 4.29 mmol) was added in batches, and the mixture was warmed to room temperature and stirred for 2 hours. The reaction solution was concentrated under reduced pressure, the reaction solution was subjected to phase separation with dichloromethane and water, the organic phase was dried over anhydrous sodium sulphate and filtered, and the organic solvent was concentrated under reduced pressure and separated by column chromatography to obtain the target compound 4-bromo-3-chloro-1-methyl-1H-pyrazole (790 mg, 94.2%).

Step 2 Synthesis of 3-chloro-1-methyl-4-(4,4,5,5-tetramethyl-1,3,2-dioxaborolan-2-yl)-1H-pyrazole

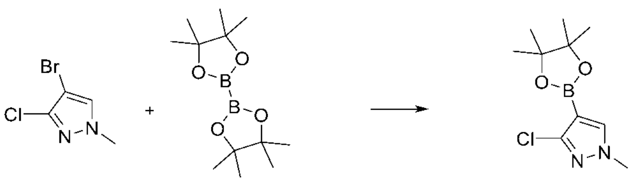

4-Bromo-3-chloro-1-methyl-1H-pyrazole (790 mg, 4.04 mmol) and 4,4,4',4',5,5,5',5'-octamethyl-2,2'-di(1,3,2-dioxaborolane) (1.23 g, 4.85 mmol) were dissolved in dioxane (15 mL), potassium phosphate (793 mg, 8.08 mmol) and [1,1'-bis(diphenylphosphino)ferrocene]palladium dichloride (300 mg, 0.41 mmol) were added, and the mixture was warmed to 95°C and stirred for 12 hours. The reaction solution was cooled to room temperature, and concentrated under reduced pressure, the reaction solution was subjected to phase separation with dichloromethane and water, the organic phase was dried over anhydrous sodium sulphate and filtered, and the organic solvent was concentrated under reduced pressure and separated by column chromatography to obtain the target compound 3-chloro-1-methyl-4-(4,4,5,5-tetramethyl-1,3,2-dioxaborolan-2-yl)-1H-pyrazole (590 mg, 60.2%). MS m/z (ESI): 243.1 $[M+H]^+$.

Step 3 Synthesis of 2-(3-chloro-1-methyl-1H-pyrazol-4-yl)pyrimidin-4-amine

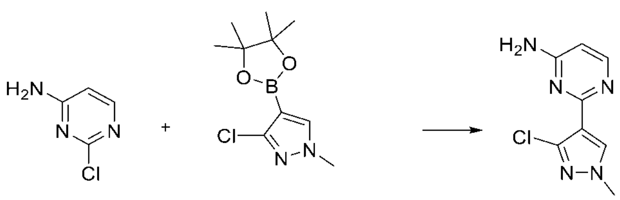

2-Chloropyrimidin-4-amine (259 mg, 2 mmol), 3-chloro-1-methyl-4-(4,4,5,5-tetramethyl-1,3,2-dioxaborolan-2-yl)-1H-pyrazole (534 mg, 2.20 mmol) and potassium carbonate (829 mg, 6 mmol) were mixed with dioxane (5 mL) and water (1 mL), [1,1'-bis(diphenylphosphino)ferrocene]palladium dichloride (146 mg, 0.20 mmol) was added, and the

mixture was warmed to 95°C and stirred for 12 hours. The reaction solution was cooled to room temperature, and concentrated under reduced pressure, the reaction solution was subjected to phase separation with dichloromethane and water, the organic phase was dried over anhydrous sodium sulphate and filtered, and the organic solvent was concentrated under reduced pressure and separated by column chromatography to obtain the target compound 2-(3-chloro-1-methyl-1H-pyrazol-4-yl)pyrimidin-4-amine (143 mg, 34.2%). MS m/z (ESI): 210.0 $[M+H]^+$.

Example 1

Preparation of N-(2-(3-chloro-1-methyl-1H-pyrazol-4-yl)pyrimidin-4-yl)-6-fluoro-5-isopropyl-8-((2R,3S)-2-methyl-3-((methanesulphonyl)methyl)azetidin-1-yl)isoquinolin-3-amine

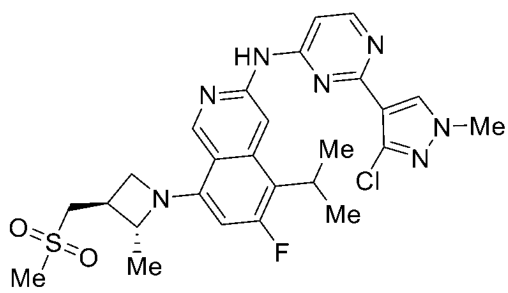

Step 1 Synthesis of 3-chloro-6-fluoro-5-isopropyl-8-((2R,3 S)-2-methyl-3-((methanesulphonyl)methyl)azetidin-1-yl)isoquinoline

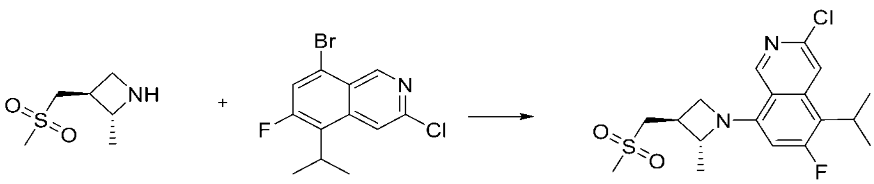

8-Bromo-3-chloro-6-fluoro-5-isopropylisoquinoline (500 mg, 1.65 mmol) and (2R,3S)-2-methyl-3-((methanesulphonyl)methyl)azetidine (297 mg, 1.82 mmol) were dissolved in dioxane (10 mL), caesium carbonate (1.15 g, 3.54 mmol) and Xantphos Pd G4 (164 mg, 0.17 mmol) were added, and the mixture was warmed to 100°C and stirred for 12 hours. The reaction solution was cooled to room temperature, and concentrated under reduced pressure, the reaction solution was subjected to phase separation with dichloromethane and water, the organic phase was dried over anhydrous sodium sulphate, and the organic solvent was concentrated under reduced pressure and separated by column chromatography to obtain the target compound 3-chloro-6-fluoro-5-isopropyl-8-((2R,3S)-2-methyl-3-((methanesulphonyl)methyl)azetidin-1-yl)isoquinoline (418 mg, 65.8%). MS m/z (ESI): 385.1 $[M+H]^+$.

Step 2 Synthesis of N-(2-(3-chloro-1-methyl-1H-pyrazol-4-yl)pyrimidin-4-yl)-6-fluoro-5-isopropyl-8-((2R,3S)-2-methyl-3-((methanesulphonyl)methyl)azetidin-1-yl)isoquinolin-3-amine

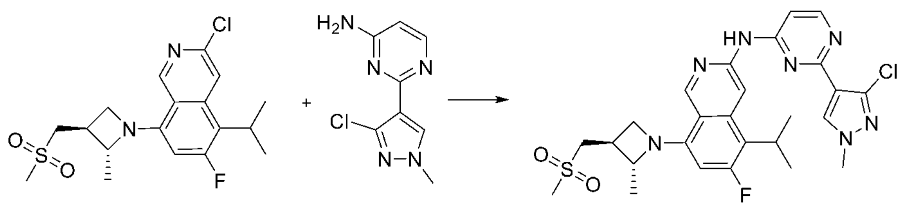

3-Chloro-6-fluoro-5-isopropyl-8-((2R,3S)-2-methyl-3-((methanesulphonyl)methyl)azetidin-1-yl)isoquinoline

(100 mg, 0.26 mmol) and 2-(3-chloro-1-methyl-1H-pyrazol-4-yl)pyrimidin-4-amine (54 mg, 0.26 mmol) were dissolved in dioxane (5 mL), caesium carbonate (274 mg, 0.84 mmol) and BrettPhos Pd G4 (24 mg, 26 μmol) were added, and the mixture was warmed to 100°C and stirred for 12 hours. The reaction solution was cooled to room temperature, and concentrated under reduced pressure, the reaction solution was subjected to phase separation with dichloromethane and water, the organic phase was dried over anhydrous sodium sulphate, and the organic solvent was concentrated under reduced pressure and separated by column chromatography to obtain the target compound N-(2-(3-chloro-1-methyl-1H-pyrazol-4-yl)pyrimidin-4-yl)-6-fluoro-5-isopropyl-8-((2R,3S)-2-methyl-3-((methanesulphonyl)methyl)azetidin-1-yl)isoquinolin-3-amine (61 mg, 42%). MS m/z (ESI): 558.2 $[M+H]^+$.

$^1H$ NMR (400 MHz, DMSO-$d_6$) δ 10.14 (s, 1H), 8.96 (s, 1H), 8.53 (s, 1H), 8.35 (d, $J$ = 5.8 Hz, 1H), 8.24 (s, 1H), 7.19 (d, $J$ = 5.8 Hz, 1H), 6.27 (d, $J$ = 14.4 Hz, 1H), 4.70-4.62 (m, 1H), 4.21-4.14 (m, 1H), 3.83 (s, 3H), 3.72-3.64 (m, 1H), 3.57-3.41 (m, 3H), 2.93 (s, 3H), 2.87-2.77 (m, 1H), 1.39-1.26 (m, 9H);

Example 2

Preparation of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide

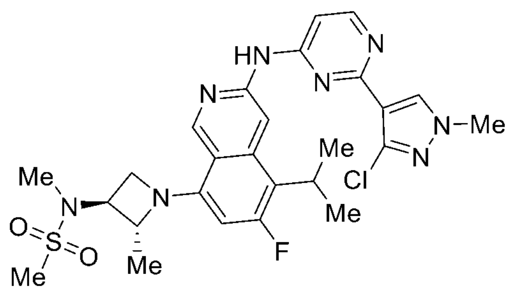

Step 1 Synthesis of N-((2R,3S)-1-(3-chloro-6-fluoro-5-isopropylisoquinolin-8-yl)-2-methylazetidin-3-yl)-N-methylmethanesulphonamide

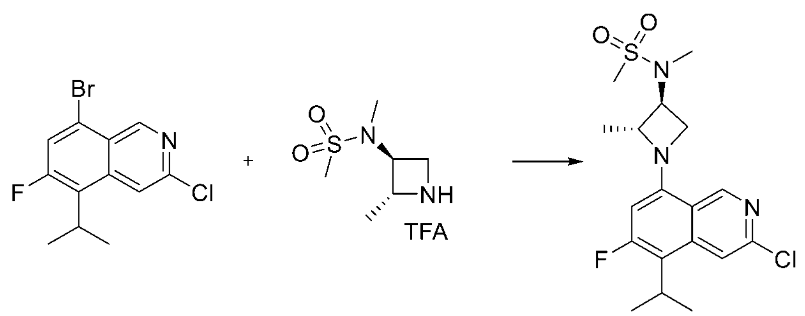

8-Bromo-3-chloro-6-fluoro-5-isopropylisoquinoline (500 mg, 1.65 mmol) and N-methyl-N-((2R,3S)-2-methylazetidin-3-yl)methanesulphonamide trifluoroacetate (470 mg, 1.70 mmol) were mixed in dioxane (10 mL), caesium carbonate (1.15 g, 3.54 mmol) and Xantphos Pd G4 (164 mg, 0.17 mmol) were added, and the mixture was warmed to 100°C and stirred for 5 hours. The reaction solution was cooled to room temperature, and concentrated under reduced pressure, the residue was subjected to phase separation with dichloromethane and water, the organic phase was dried over anhydrous sodium sulphate and filtered, and the filtrate was concentrated under reduced pressure and separated by column chromatography to obtain the target compound N-((2R,3S)-1-(3-chloro-6-fluoro-5-isopropylisoquinolin-8-yl)-2-methylazetidin-3-yl)-N-methylmethanesulphonamide (330 mg, 50.0%). MS m/z (ESI): 400.1 $[M+H]^+$.

$^1H$ NMR(400 MHz, DMSO-$d_6$) δ 9.08 (s, 1H), 8.07 (s, 1H), 6.68-6.65 (m, 1H), 4.76-4.69 (m, 1H), 4.59-4.55 (m, 1H), 4.26-4.22 (m, 1H), 3.98-3.96 (m, 1H), 3.67-3.59 (m, 1H), 2.95 (s, 3H), 2.83 (s, 3H), 1.40 (d, $J$ = 6.0 Hz, 3H), 1.37-1.31 (m, 6H) ;

Step 2 Synthesis of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide

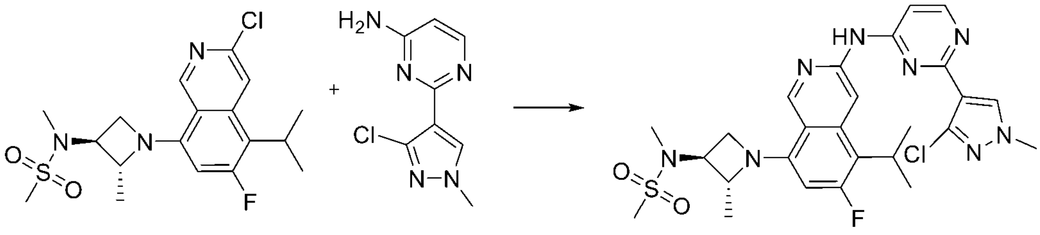

N-((2R,3S)-1-(3-Chloro-6-fluoro-5-isopropylisoquinolin-8-yl)-2-methylazetidin-3-yl)-N-methylmethanesulphonamide (100 mg, 0.25 mmol) and 2-(3-chloro-1-methyl-1H-pyrazol-4-yl)pyrimidin-4-amine (55 mg, 0.25 mmol) were dissolved in dioxane (5 mL), caesium carbonate (274 mg, 0.84 mmol) and BrettPhos Pd G4 (24 mg, 26 μmol) were added, and the mixture was warmed to 100°C and stirred for 12 hours. The reaction solution was cooled to room temperature, and concentrated under reduced pressure, the residue was subjected to phase separation with dichloromethane and water, the organic phase was dried over anhydrous sodium sulphate and filtered, and the filtrate was concentrated under reduced pressure and separated by column chromatography to obtain the target compound N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide (73 mg, 50.9%). MS m/z (ESI): 573.2 $[M+H]^+$.

$^1$H NMR (400 MHz, DMSO-$d_6$) δ 10.21 (s, 1H), 9.05 (s, 1H), 8.60 (s, 1H), 8.42 (d, *J* = 5.8 Hz, 1H), 8.30 (s, 1H), 7.28 (d, *J* = 6.0 Hz, 1H), 6.47-6.43 (m, 1H), 4.71 -4.69(m, 1H), 4.53-4.49 (m, 1H), 4.23-4.18 (m, 1H), 3.94-3.91 (m, 1H), 3.89 (s, 3H), 3.63 -3.58 (m, 1H), 2.95 (s, 3H), 2.84 (s, 3H), 1.40 (d, *J* = 6.0 Hz, 6H), 1.36-1.32 (m, 3H).

Example 3

Preparation of N-(1-(3-((2-(3-chloro-1-methyl-1H-pyrazol-4-yl)pyrimidin-4-yl)amino)-6-fluoro-5-isopropylisoquinolin-8-yl)azetidin-3-yl)-N-methylmethanesulphonamide

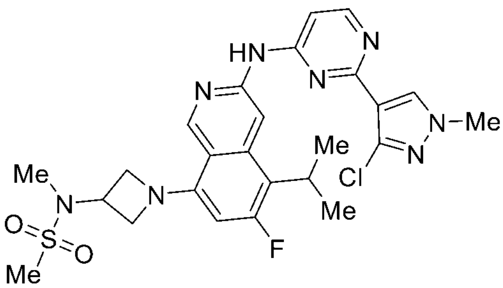

N-(1-(3-((2-(3-chloro-1-methyl-1H-pyrazol-4-yl)pyrimidin-4-yl)amino)-6-fluoro-5-isopropylisoquinolin-8-yl)azetidin-3-yl)-N-methylmethanesulphonamide was prepared with reference to example 1.

$^1$H NMR (400 MHz, DMSO-$d_6$) δ 10.18 (s,1H), 9.06 (s, 1H), 8.56 (s, 1H), 8.42 (d, *J* = 5.8 Hz, 1H), 8.30 (s, 1H), 7.31 (d, *J* = 5.8 Hz, 1H), 6.28-6.25 (m, 1H), 4.70 -4.68(m, 1H), 4.46-4.43 (m, 2H), 4.32-4.29 (m, 2H), 3.89 (s, 3H), 3.59-3.56 (m, 1H), 2.95-2.93 (m, 6H), 1.38-1.36 (m, 6H);
MS m/z (ESI): 559.2 $[M+H]^+$.

Example 4

Preparation of N-(2-(1,3-dimethyl-1H-pyrazol-4-yl)pyrimidin-4-yl)-6-fluoro-5-isopropyl-8-((2R,3S)-2-methyl-3-((methanesulphonyl)methyl)azetidin-1-yl)isoquinolin-3-amine

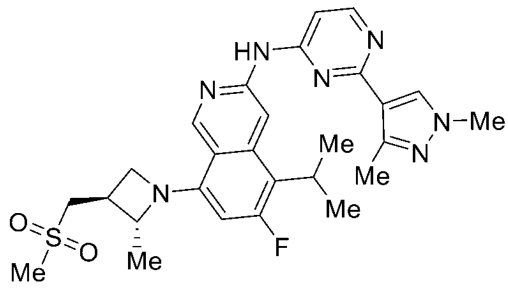

N-(2-(1,3-dimethyl-1H-pyrazol-4-yl)pyrimidin-4-yl)-6-fluoro-5-isopropyl-8-((2R,3S)-2-methyl-3-((methanesulphonyl)methyl)azetidin-1-yl)isoquinolin-3-amine was prepared with reference to example 1.

$^1$H NMR (400 MHz, DMSO-$d_6$) $\delta$ 10.04 (s, 1H), 8.96 (s, 1H), 8.47 (s, 1H), 8.31 (d, $J$ = 5.8 Hz, 1H), 8.07 (s, 1H), 7.17-7.09 (m, 1H), 6.31-6.23 (m, 1H), 4.66 (t, $J$ = 7.6 Hz, 1H), 4.18 (t, $J$ = 6.2 Hz, 1H), 3.77 (s, 3H), 3.68 (t, $J$ = 7.2 Hz, 1H), 3.56-3.44 (m, 3H), 2.93 (s, 3H), 2.86-2.77 (m, 1H), 2.44 (s, 3H), 1.39-1.31 (m, 9H);
MS m/z (ESI): 538.2 [M+H]$^+$.

Example 5

Preparation of N-((2R,3S)-1-(3-((2-(1,3-dimethyl-1H-pyrazol-4-yl)pyrimidin-4-yl)amino)-6-fluoro-5-isopropylisoquinolin-8-yl)-2-methylazetidin-3-yl)-N-methylmethanesulphonamide

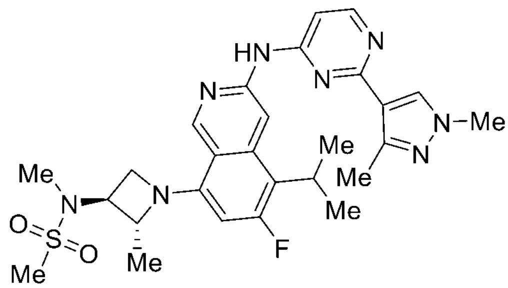

N-((2R,3S)-1-(3-((2-(1,3-dimethyl-1H-pyrazol-4-yl)pyrimidin-4-yl)amino)-6-fluoro-5-isopropylisoquinolin-8-yl)-2-methylazetidin-3-yl)-N-methylmethanesulphonamide was prepared with reference to example 2.

$^1$H NMR (400 MHz, DMSO-$d_6$) $\delta$ 10.12 (s, 1H), 9.05 (s, 1H), 8.55 (s, 1H), 8.38 (d, $J$= 5.8 Hz, 1H), 8.14 (s, 1H), 7.22 (d, $J$ = 5.6 Hz, 1H), 6.47 (d, $J$ = 14.3 Hz, 1H), 4.75-4.67 (m, 1H), 4.57-4.48 (m, 1H), 4.27-4.19 (m, 1H), 3.97-3.91 (m, 1H), 3.84 (s, 3H), 3.64-3.52 (m, 1H), 2.95 (s, 3H), 2.84 (s, 3H), 2.51 (s, 3H), 1.46-1.37 (m, 9H);
MS m/z (ESI): 553.2 [M+H]$^+$.

Example 6

Preparation of N-(1-(3-((2-(1,3-dimethyl-1H-pyrazol-4-yl)pyrimidin-4-yl)amino)-6-fluoro-5-isopropylisoquinolin-8-yl) azetidin-3-yl)-N-methylmethanesulphonamide

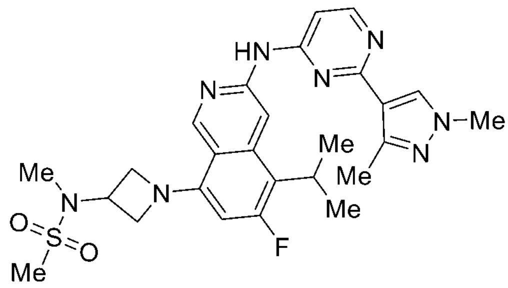

N-(1-(3-((2-(1,3-dimethyl-1H-pyrazol-4-yl)pyrimidin-4-yl)amino)-6-fluoro-5-isopropylisoquinolin-8-yl)azetidin-3-yl)-N-methylmethanesulphonamide was prepared with reference to example 1.

$^1$H NMR (400 MHz, DMSO-$d_6$) $\delta$ 10.00 (s, 1H), 9.00 (s, 1H), 8.43 (s, 1H), 8.32 (d, $J$ = 5.8 Hz, 1H), 8.07 (s, 1H), 7.21-7.17 (m, 1H), 6.23-6.15 (m, 1H), 4.67-4.60 (m, 1H), 4.39 (t, $J$ = 8.2 Hz, 2H), 4.29-4.22 (m, 2H), 3.77 (s, 3H), 3.55-3.47 (m, 1H), 2.91-2.84 (m, 6H), 2.44 (s, 3H), 1.34 (d, $J$ = 6.6 Hz, 6H);
MS m/z (ESI): 539.2 $[M+H]^+$.

Example 7

Preparation of N-(1-(3-((2-(3-chloro-1-methyl-1H-pyrazol-4-yl)pyrimidin-4-yl)amino)-5-(dimethylamino)-6-fluoroisoquinolin-8-yl)azetidin-3-yl)-N-methylmethanesulphonamide

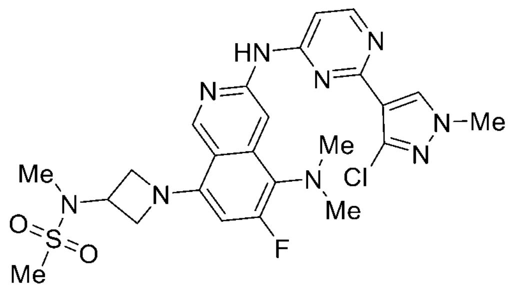

N-(1-(3-((2-(3-chloro-1-methyl-1H-pyrazol-4-yl)pyrimidin-4-yl)amino)-5-(dimethylamino)-6-fluoroisoquinolin-8-yl)azetidin-3-yl)-N-methylmethanesulphonamide was prepared with reference to example 1.
MS m/z (ESI): 560.2 $[M+H]^+$.

Example 8

Preparation of (R)-N-(1-(3-((2-(3-chloro-1-methyl-1H-pyrazol-4-yl)pyrimidin-4-yl)amino)-6-fluoro-5-(1-methoxyethyl)isoquinolin-8-yl)azetidin-3-yl)-N-methylmethanesulphonamide

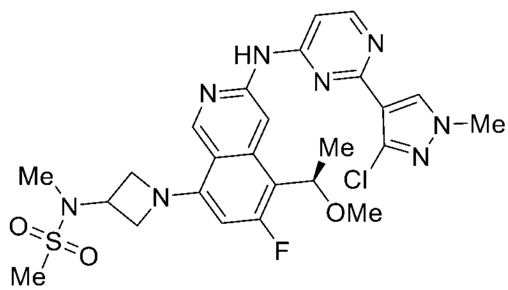

(R)-N-(1-(3-((2-(3-chloro-1-methyl-1H-pyrazol-4-yl)pyrimidin-4-yl)amino)-6-fluoro-5-(1-methoxyethyl)isoquinolin-8-yl)azetidin-3-yl)-N-methylmethanesulphonamide was prepared with reference to example 1.

MS m/z (ESI): 575.2 [M+H]+.

Example 9

Preparation of (S)-N-(1-(3-((2-(3-chloro-1-methyl-1H-pyrazol-4-yl)pyrimidin-4-yl)amino)-6-fluoro-5-(1-methoxyethyl) isoquinolin-8-yl)azetidin-3-yl)-N-methylmethanesulphonamide

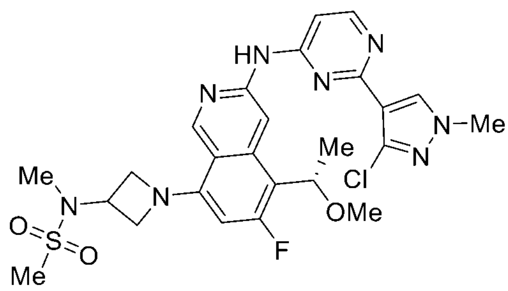

(S)-N-(1-(3-((2-(3-chloro-1-methyl-1H-pyrazol-4-yl)pyrimidin-4-yl)amino)-6-fluoro-5-(1-methoxyethyl)isoquinolin-8-yl)azetidin-3-yl)-N-methylmethanesulphonamide was prepared with reference to example 1.

MS m/z (ESI): 575.2 [M+H]+.

Example 10

Preparation of N3-(2-(3-chloro-1-methyl-1H-pyrazol-4-yl)pyrimidin-4-yl)-6-fluoro-N5,N5-dimethyl-8-((2R,3S)-2-methyl-3-((methanesulphonyl)methyl)azetidin-1-yl)isoquinolin-3,5-diamine

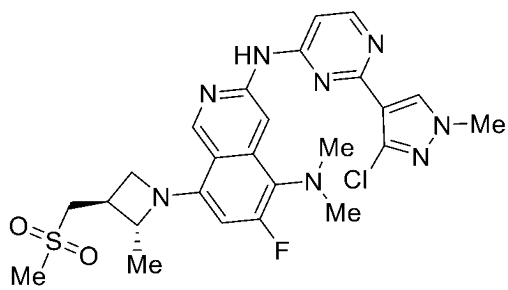

N3-(2-(3-chloro-1-methyl-1H-pyrazol-4-yl)pyrimidin-4-yl)-6-fluoro-N5,N5-dimethyl-8-((2R,3S)-2-methyl-3-((methanesulphonyl)methyl)azetidin-1-yl)isoquinolin-3,5-diamine was prepared with reference to example 1.

MS m/z (ESI): 559.2 [M+H]+.

Example 11

Preparation of N-(2-(3-chloro-1-methyl-1H-pyrazol-4-yl)pyrimidin-4-yl)-6-fluoro-5-((R)-1-methoxyethyl)-8-((2R,3S)-2-methyl-3-((methanesulphonyl)methyl)azetidin-1-yl)isoquinolin-3-amine

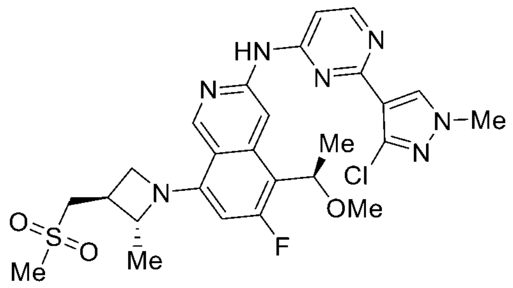

N-(2-(3-chloro-1-methyl-1H-pyrazol-4-yl)pyrimidin-4-yl)-6-fluoro-5-((R)-1-methoxyethyl)-8-((2R,3S)-2-methyl-3-((methanesulphonyl)methyl)azetidin-1-yl)isoquinolin-3-amine was prepared with reference to example 1.
MS m/z (ESI): 574.2 $[M+H]^+$.

Example 12

Preparation of N-(2-(3-chloro-1-methyl-1H-pyrazol-4-yl)pyrimidin-4-yl)-6-fluoro-5-((S)-1-methoxyethyl)-8-((2R,3S)-2-methyl-3-((methanesulphonyl)methyl)azetidin-1-yl)isoquinolin-3-amine

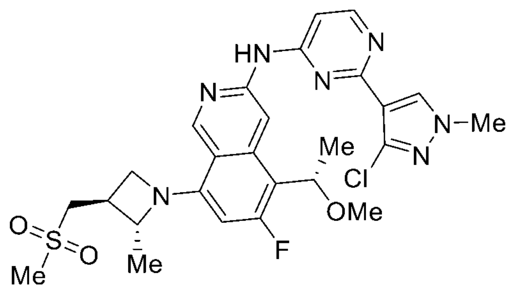

N-(2-(3-chloro-1-methyl-1H-pyrazol-4-yl)pyrimidin-4-yl)-6-fluoro-5-((S)-1-methoxyethyl)-8-((2R,3S)-2-methyl-3-((methanesulphonyl)methyl)azetidin-1-yl)isoquinolin-3-amine was prepared with reference to example 1.
MS m/z (ESI): 574.2 $[M+H]^+$.

Example 13

Preparation of N-(1-(3-((2-(3-chloro-1-methyl-1H-pyrazol-4-yl)pyrimidin-4-yl)amino)-6-fluoro-5-isopropylisoquinolin-8-yl)azetidin-3-yl)methanesulphonamide

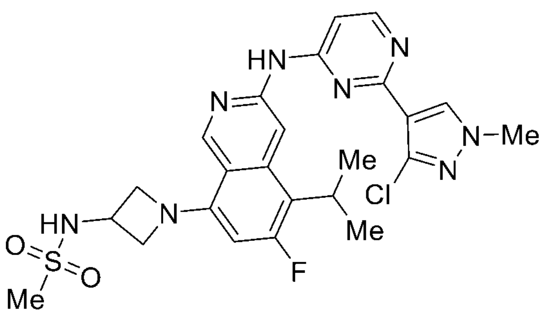

N-(1-(3-((2-(3-chloro-1-methyl-1H-pyrazol-4-yl)pyrimidin-4-yl)amino)-6-fluoro-5-isopropylisoquinolin-8-yl)azetidin-3-yl)methanesulphonamide was prepared with reference to example 2.

$^1$H NMR (400 MHz, DMSO-$d_6$) $\delta$ 10.24 (s, 1H), 9.08 (s, 1H), 8.63 (s, 1H), 8.48 (d, $J$ = 5.2 Hz, 1H), 8.36 (s, 1H), 7.92 (d, $J$ = 7.2 Hz, 1H), 7.35 (br s, 1H), 6.36 (d, $J$ = 8.0 Hz, 1H), 4.69-4.65 (m, 2H), 4.44-4.41 (m, 1H), 4.08-4.04 (m, 2H), 3.95 (s,

3H), 3.58-3.52 (m, 1H), 3.04 (s, 3H), 1.44-1.33 (m, 6H);
MS m/z (ESI): 545.2 $[M+H]^+$.

Example 14

Preparation of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)methanesulphonamide

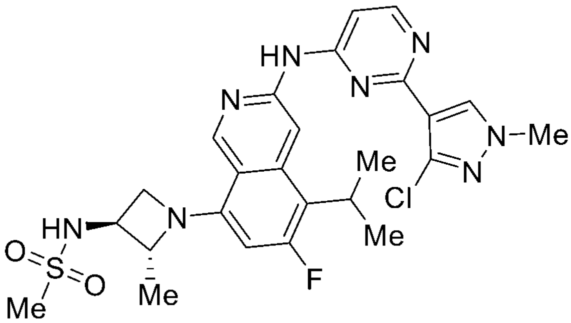

N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)methanesulphonamide was prepared with reference to example 2.

$^1$H NMR (400 MHz, DMSO-$d_6$) $\delta$ 10.16 (s, 1H), 8.96 (s, 1H), 8.54 (s, 1H), 8.35 (d, $J$ = 7.2 Hz, 1H), 8.24 (s, 1H), 7.71 (d, $J$ = 7.6 Hz, 1H), 7.20 (br s, 1H), 6.36 (br s, 1H), 4.82-4.77 (m, 1H), 4.16-4.10 (m, 1H), 3.88-3.83 (m, 4H), 3.62-3.54 (m, 2H), 2.90 (s, 3H), 1.37-1.16 (m, 9H);
MS m/z (ESI): 559.2 $[M+H]^+$.

## II. Biological assay and evaluation of compounds

The present invention will be further described and explained below in conjunction with test examples, but these examples are not meant to limit the scope of the present invention.

### Test example 1. Determination of the inhibitory effect of the compounds of the present invention on the activity of EGFR del19/T790M/C797S and EGFR L858R/T790M/C797S mutant kinases

Experimental objective: The objective of this test example is to test the inhibitory activity of the compounds on the activity of EGFR **del19/T790M/C797S** and EGFR L858R/T790M/C797S mutant kinases.

Experimental instruments: The centrifuge (5810R) was purchased from Eppendorf, the pipette was purchased from Eppendorf or Rainin, and the microplate reader was purchased from BioTek (United States) with a model of SynergyH1 multifunctional microplate reader.

Experimental methods: This experiment used Cisbio's HTRF kinase assay method (Cisbio#62TK0PEB), wherein a catalytic reaction occurred between the substrate polypeptide TK and ATP in the presence of the tyrosine kinase with EGFR del19/T790M/C797S or EGFR L858R/T790M/C797S mutation, the substance was phosphorylated, and the activity of the kinase was characterized by measuring the content of the phosphorylated substrate generated during the reaction, and the half inhibitory concentration $IC_{50}$ of the compound on the activity of EGFR del19/T790M/C797S or EGFR L858R/T790M/C797S mutant kinase was obtained.

Specific experimental operations were as follows: the kinase reaction was carried out in a white 384-well plate (Perkin Elmer#6008280), and 1-5 $\mu$L of compounds at different concentrations which were diluted with 1% DMSO-containing ddH$_2$O were added; to the positive control wells were added 1-5 $\mu$L of 1% DMSO-containing ddH$_2$O, followed by 1-5 $\mu$L of 0.5-5 nM 4$\times$EGFR del19/T790M/C797S or EGFR L858R/T790M/C797S mutant kinase solution which was diluted with Dilution buffer (5 $\times$kinase buffer, $MgCl_2$ 6.65 Mm, $MnCl_2$ 1.33 mM, DTT 1.33 mM); to the negative control wells were added 1-5 $\mu$L of Dilution buffer; to all the wells were added 1-5 $\mu$L of 4 $\mu$M 4$\times$ substrate TK solution which was prepared with 10$\times$ Dilution buffer, and finally added 1-5 $\mu$L of 24 $\mu$M 4$\times$ ATP solution which was diluted with Dilution buffer to start the reaction; after reacting at room temperature for 120 minutes, 10 $\mu$L of detection solution (TK antibody 16 nM, XL665 0.5 $\mu$M) was added to each well, same was reacted at room temperature in the dark for 20 minutes, and then the chemiluminescence value was detected using a BioTek Synergy H1 microplate reader.

Experimental data processing method: The percent inhibition data for compound-treated wells was calculated according to the positive control wells (DMSO control wells) and negative control wells (no kinase added) on the plate {% inhibition rate = 100 - [(test compound value-negative control value)] / (positive control value - negative control value) × 100}. GraphPad prism was used to fit different concentrations and corresponding percent inhibition rate data to a 4-parameter nonlinear logistic equation to calculate $IC_{50}$ values.

Experimental results and conclusions: The compounds in this example of the present invention have good inhibitory effects on EGFR L858R/T790M/C797S and EGFR del19/T790M/C797S drug-resistant mutant kinases.

Test example 2: Cell proliferation inhibition experiment

Experimental objective: The objective of this test example is to test the inhibitory activity of compounds on the proliferation of different EGFR C797S drug-resistant mutant cell lines Ba/F3 EGFR Del19/T790M/C797S, Ba/F3 EGFR L858R/T790M/C797S, and Ba/F3 EGFR Del19/C797S.
Experimental instruments: Centrifuge (Eppendorf 5810R); Microplate reader (BioTek Synergy H1)
Pipette (Eppendorf or Rainin); Carbon dioxide incubator (Thermo 311)
Cell counter (Life Countess II)
Experimental reagents and consumables:

Ba/F3 EGFR Del19/T790M/C797S cells were purchased from KYinno Biotechnology Co., Ltd.;
Ba/F3 EGFR Del19/C797S cells were purchased from CoBioer Biosciences Co., Ltd.;
Ba/F3 EGFR L858R/T790M/C797S cells were purchased from CoBioer Biosciences Co., Ltd.;
Cell Titer-Glo was purchased from Promega, with a catalogue number of G7573;
RPMI 1640 was purchased from Gibco, with a catalogue number of 22400089;
FBS was purchased from Gibco, with a catalogue number of 10091148;
cell culture plates were purchased from Corning, with a catalogue number of 3610.

Experimental methods: The inhibitory activity of the compound on the proliferation of different EGFR C797S drug-resistant mutant cell lines was detected using the Cell Titer-Glo method. The different cell lines were cultured in RPMI 1640 complete culture medium containing 10% FBS under conditions of 37°C and 5% $CO_2$, the cells were collected by centrifugation when growing to a certain density, and adjusted to appropriate cell density after counting, the cells were spread on a white 96-well plate at 90 μL/well, and cultured in a 37°C, 5% $CO_2$ incubator overnight, prepared compound solutions at different concentrations were added at 10 μL/well, corresponding solvent controls were set, and all wells were cultured in a 37°C, 5% $CO_2$ incubator for 72 hours, 50 μL of CellTiter-Glo solution was added to each well, shook and mixed evenly, and incubated in the dark for 10 minutes, and a BioTek Synergy H1 microplate reader was used for reading.
Experimental data processing method: The luminescence signal value was used to calculate the inhibition rate. Graphpad Prism software was used to fit the concentration and inhibition rate to a nonlinear regression curve, so as to obtain the $IC_{50}$ value, as shown in the Table below for details.

Table 20

| Example No. | Ba/F3 EGFR Del19_T790M_C797S $IC_{50}$ (nM) |
|---|---|
| 1 | 7.1 |
| 2 | 4.5 |
| 4 | 6.8 |
| 5 | 4.5 |

Table 21

| Example | Ba/F3 EGFR L858R_T790M_C797S $IC_{50}$ (nM) |
|---|---|
| 1 | 14.0 |
| 2 | 15.0 |
| 14 | 8.8 |

Table 22

| Example No. | Ba/F3 EGFR Dell9/C797S IC50 (nM) |
|---|---|
| 1 | 11.0 |
| 2 | 6.2 |
| 3 | 14.0 |
| 4 | 18 |
| 5 | 7.8 |
| 6 | 22 |
| 13 | 19 |
| 14 | 2.6 |

Experimental conclusion: According to the above-mentioned schemes, it can be concluded that the compounds shown in the present invention show a significant inhibitory activity against the proliferation of different EGFR C797S drug-resistant mutant cell lines in the proliferation inhibitory activity test.

**Test example 3: Rat pharmacokinetic evaluation test**

1. Study objective: SD rats were used as test animals to study the pharmacokinetic behaviour of the compounds of the present invention in rats (plasma) after oral administration at a dose of 5 mg/kg.
2. Experimental scheme:
2.1 Experimental drugs: The example compounds of the present invention, made in house.
2.2 Experimental animals: 3 SD rats/group, male, from Shanghai Jiesijie Experimental Animal Co., Ltd., animal production license number (SCXK (Shanghai) 2013-0006 N0.311620400001794).
2.3 Preparation formulation:

Formulation of drug for oral administration: 20% solutol HS15 in 0.5% MC (methylcellulose)
20 g of a Solutol HS15 solid and 0.5 g of a MC solid powder were weighed and dissolved in 80 mL of purified water, and the mixture was vortexed, mixed until uniform, and subjected to ultrasound treatment to obtain a clear solution of 20% solutol HS15 in 0.5% MC. The example compound was weighed into a 20 mL glass vial, the solution was added thereto and the mixture was subjected to ultrasound treatment for 10 minutes to obtain a clear solution at a concentration of 0.5 mg/mL.

2.4 Administration: 3 male SD rats were administered p.o. respectively after the rats were fasted overnight; and the p.o. dose was 5 mg/kg and the administration volume was 10 mL/kg.
2.5 Sample collection:
Blood collection: Before and after administration to rats, 0.2 mL of blood was collected from the jugular vein at 0.25, 0.5, 1, 2, 4, 6, 8 and 24 h, and placed in an EDTA-$K_2$ anticoagulation tube, which was centrifuged at 6000 rpm at 4°C for 6 min to separate plasma which was stored at -80°C; and the animals were fed 4 h after administration.
2.6 Sample treatment:

1) Acetonitrile (160 μL) was added to plasma sample (40 μL) for precipitation, and the mixture was centrifuged at 3500 × g for 5-20 min.
2) The treated supernatant solution was taken and subjected to LC/MS/MS to analyse the concentrations of the test compound. LC/MS/MS analysis instrument: AB Sciex API 4000 Qtrap.

2.7 Liquid phase analysis:
• Liquid phase conditions: Shimadzu LC-20AD pump
• Chromatographic column: Agilent ZORBAX XDB-C18 (50 × 2.1 mm, 3.5 μm), mobile phase: solution A: 0.1% formic acid in water, solution B: acetonitrile
• Flow rate: 0.4 mL/min
• Elution time: 0-4.0 min, with eluents as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis: The main pharmacokinetic parameters were calculated using WinNonlin 6.1. The results of pharmacokinetic experiments in rats are as shown in Table 23 below:

Table 23

| Example | Cmax (ng/mL) | $AUC_{0-t}$ (hr*ng/mL) | $T_{1/2}$ (h) |
|---|---|---|---|
| 1 | 178 | 2102 | 2.3 |
| 2 | 142 | 1771 | 3.9 |
| 5 | 157 | 2005 | 3.9 |

3.4 Experimental conclusion: The data in the table show that in the rat pharmacokinetic evaluation experiment, the example compounds of the present invention show high exposure after oral administration.

**Test example 4: *In vivo* pharmacodynamic study of the compound in a nude mouse subcutaneous transplanted tumour model of mouse primary B cell line Ba/$F_3$ EGFR Del19/C797S**

1.1 Experimental objective: The objective of this test example is to evaluate the *in vivo* efficacy of the compound in a nude mouse subcutaneous transplanted tumour model of mouse primary B cell line Ba/$F_3$ EGFR Del19/C797S.
1.2. Experimental instruments and reagents
1.2.1 Instruments

1. Refrigerator (BCD-268TN, Haier)
2. Biological safety cabinet (BSC-1300II A2, Shanghai Boxun Industry & Commerce Co., Ltd. Medical Equipment Factory)
3. Ultra-clean workbench (CJ-2F, Suzhou Fengshi Experimental Animal Equipment Co., Ltd.)
4. Electric pipette-aid (Easypet 3, Eppendorf)
5. Thermostat water bath (HWS-12, Shanghai Yiheng Science)
6. $CO_2$ incubator (Thermo-311, Thermo)
7. Centrifuge (Centrifuge 5720R, Eppendorf)
8. Full-automatic cell counter (Countess II, Life Technologies);
9. Vernier caliper (CD-6"AX, Mitutoyo)
10. Cell culture flask (T25/T75/T225, Corning)
11. Electronic balance (CPA2202S, Sartorius)
12. Electronic balance (BSA2202S-CW, Sartorius)
13. Ultrasonic cleaner (115F0032, Shanghai Kudos)
14. Water purifier (Pacific TII, Thermo) 15. Magnetic stirrer (08-2G, Chijiu)

1.2.2 Reagents

1. RPMI-1640 medium (22400-089, Gibco)
2. Fetal bovine serum (FBS) (10099-141C, Gibco)
3. Phosphate buffer solution (PBS) (10010-023, Gibco)
4. Kolliphor HS15 (42966-1KG, Sigma-Aldrich)

5. Methylcellulose M450 (69016482, Sinopharm Chemical Reagent Co., Ltd.)

1.3. Experimental operations and data processing

1.3.1 Animals: BALB/c nude mice, 6-8 weeks old, ♀, purchased from the Laboratory Animal Business Department of Shanghai Institute of Planned Parenthood Research

1.3.2 Cell culture and cell suspension preparation

a. A Ba/$F_3$ EGFR Del19/C797S cell line was taken out from the cell bank, and the cells were recovered with RPMI-1640 medium (RPMI-1640 + 10% FBS). The recovered cells were cultured in a $CO_2$ incubator (incubator temperature: 37°C, $CO_2$ concentration: 5%).
b. The cells were passaged every three days, and after passage, the cells were placed in the $CO_2$ incubator to continue the culture. This process was repeated until the cell number met the requirements for *in vivo* efficacy.
c. The cells in the exponential growth phase were collected, counted using a full-automatic cell counter, resuspended to $2 \times 10^7$ cells/mL with PBS according to the counting results, and then placed in an ice box until use.

1.3.3 Cell inoculation

a. Before inoculation, the nude mice were marked with disposable universal ear tags for mice and rats.
b. At the time of inoculation, the cell suspension was mixed uniformly, 0.1-1 mL of the cell suspension was aspirated using a 1 mL syringe with air bubbles expelled, and the syringe was then placed on an ice bag for later use.
c. The nude mouse was secured with the left hand, the right dorsal area near the right shoulder (inoculation site) of the nude mouse was disinfected with a 75% alcohol cotton ball, and inoculation was initiated after 30 seconds.
d. The experimental nude mice were inoculated sequentially (0.1 mL of cell suspension per mouse).

1.3.4 Tumour volume measurement, grouping and administration in tumour-bearing mice

a. Tumour was measured on days 9-12 after inoculation depending on the tumour growth, and the tumour size was calculated.

Tumour volume calculation: tumour volume ($mm^3$) = length (mm) $\times$ width (mm) $\times$ width (mm)/2

b. According to the body weight of tumour-bearing mice and the size of the tumour, the mice were grouped by random grouping.
c. According to the grouping results, the test drug was started to be administered (administration method: oral administration; administration volume: 10 mL/kg; administration frequency: once/day or twice/day; administration cycle: 14 days; vehicle: 10% Solutol HS15/0.5% MC).
d. Tumour measurement and weighing were performed twice a week after starting administration of test drugs.
e. The animals were euthanized at the end of the experiment.
f. The data was processed with software such as Excel. Calculation of tumour growth inhibition (TGI) (%) of compound: when the tumour did not regress, TGI (%) = [(1 - (average tumour volume at the end of administration in a treatment group - average tumour volume at the beginning of administration in the treatment group)) / (average tumour volume at the end of administration in the solvent control group - average tumour volume at the beginning of administration in the solvent control group)] $\times$ 100%. When the tumour regressed, TGI (%) = [1 - (average tumour volume at the end of administration in a treatment group - average tumour volume at the beginning of administration in the treatment group) / average tumour volume at the beginning of administration in the treatment group] $\times$ 100%.

1.4 Experimental results and conclusion:

The key example compounds of the present invention exhibit excellent tumour inhibition effects in the model. When administered orally at a dose of 50 mpk QD, the compounds exhibited an excellent tumour inhibition effect, with tumour growth inhibition (TGI) (%) > 80%, and the tumour growth inhibition (TGI) (%) of the key preferred compounds > 100%. When administered orally at a dose of 75 mpk QD, the compounds exhibited an excellent tumour inhibition effect, with tumour growth inhibition (TGI) (%) > 100%, and the tumour growth inhibition (TGI) (%) of the key preferred compounds > 150%. When administered orally at a dose of 120 mpk QD, the compounds exhibited an excellent tumour inhibition effect, with tumour growth inhibition (TGI) (%) > 150%, and the tumour growth inhibition (TGI) (%) of

the key preferred compounds > 190%, and there was no significant weight loss.

**III. Study on crystal form of salt of Example 2**

1.1 Experimental instruments

1.1.1 Some parameters of physical and chemical testing instruments

| | | |
|---|---|---|
| XRPD | Instrument model | BRUKER D8 ADVANCE |
| | Diffraction line | CuK (40 kV, 25 mA) |
| | Scan rate | 0.02°/S (20 value) |
| | Scan range | 4° - 40°(20 value) |
| DSC | Instrument model | NETZSCH DSC 214 polyma |
| | Purge gas | Nitrogen |
| | Purge rate | 40 mL/min |
| | Heating rate | 10°C/min |
| | Temperature range | 25°C-350°C |
| | Pan type | Aluminium pan |
| TGA | Instrument model | NETZSCH TG 209 Tarsus |
| | Purge gas | Nitrogen |
| | Purge rate | 40 mL/min |
| | Heating rate | 10°C/min |
| | Temperature range | Room temperature-400°C |
| | Pan type | $Al_2O_3$ |

1.2 Instrument and liquid phase analysis conditions

1.2.1 Instruments and equipment

| Instrument name | Model |
|---|---|
| Analytical balance | 2.1 g/XPR2 |
| Water purifier | Milli-Q Plus, Millipore |
| High performance liquid chromatograph | Agilent1260 |
| Pump | Agilent G1311B |
| Sample injector | G1329B |
| Column oven | G1316A |
| Detector | G1315D |

1.2.2 Chromatographic conditions

| | | | |
|---|---|---|---|
| Mobile phase | A: 0.1% aqueous solution of phosphoric acid; B: acetonitrile | | |
| Chromatographic column | ZOBAX Bonus RP (4.6*150 mm, 3.5 μm) | | |
| Flow rate | 1.0 mL/min | | |
| Wavelength | 254 nm | | |
| Injection volume | 5 μL | | |
| Column temperature | 35°C | | |
| Gradient | Time (min) | A% | B% |
| | 0.00 | 90.0 | 10.0 |
| | 5.00 | 20.0 | 80.0 |
| | 7.00 | 10.0 | 90.0 |
| | 7.01 | 90.0 | 10.0 |
| | 10.00 | 90.0 | 10.0 |

1.2.3 Chromatographic conditions

| | | | |
|---|---|---|---|
| Mobile phase | A: 0.1% aqueous solution of phosphoric acid; B: acetonitrile | | |
| Chromatographic column | Waters Atlantis ™ T3 (4.6* 150 mm, 3 μm) | | |
| Flow rate | 1.0 mL/min | | |
| Wavelength | 254 nm | | |
| Injection volume | 5 μL | | |
| Column temperature | 35°C | | |
| Gradient | Time (min) | A% | B% |
| | 0.00 | 90.0 | 10.0 |
| | 8.00 | 40.0 | 60.0 |
| | 12.00 | 10.0 | 90.0 |
| | 12.01 | 90.0 | 10.0 |
| | 15.00 | 90.0 | 10.0 |

**1. Preparation of crystal forms of salts of compounds**

1. Crystal form A of phosphate
20 mg of the amorphous free base was weighed, 0.2 mL of ethyl acetate was added, and the mixture was dissolved to give a clear solution at room temperature. 0.042 mL of 1 M solution of phosphoric acid in methanol was added to the system, which was clear after the addition of acid. The system was stirred for a given time, and then a large amount of yellow solid precipitated. The mixture was reacted at room temperature for 2 hours, and centrifuged to obtain a solid, which was then dried to finally obtain the crystal form A of phosphate.
2. Crystal form B of phosphate
20 mg of the amorphous free base was weighed, 0.1 mL of acetone was added, and the mixture was dissolved to give a clear solution at room temperature. Then 0.3 mL of methanol was added. 0.042 mL of 1 M solution of phosphoric acid in methanol was added to the system, which was turbid after the addition of acid. The system was stirred for a given time, and then a yellow solid precipitated. The mixture was reacted at room temperature for 3 hours, and centrifuged to obtain a solid, which was then dried to finally obtain the crystal form B of phosphate.
3. Crystal form A of hydrochloride

20 mg of the amorphous free base was weighed, 0.1 mL of ethanol was added, and the mixture was stirred at 50°C for the amorphous form to undergo crystal form transformation to a suspension. 0.042 mL of 1 M solution of hydrogen chloride in methanol was added to the system, which became a red solution after the addition of acid. The system was cooled to room temperature, stirred for a given time, and then a large amount of solid precipitated. The mixture was centrifuged to obtain a solid, which was then dried to finally obtain the crystal form A of hydrochloride.

4. Crystal form B of hydrochloride

20 mg of the amorphous free base was weighed, 0.2 mL of ethyl acetate was added, and the mixture was dissolved to give a clear solution at room temperature. 0.042 mL of 1 M solution of hydrogen chloride in methanol was added to the system, which became a red solution after the addition of acid. A given amount of crystal seed A of crystal form A of hydrochloride was added to the system until the system became turbid. The system was stirred for a given time, and then a large amount of solid precipitated. The mixture was reacted at room temperature for another 48 hours, and centrifuged to obtain a solid, which was then dried to finally obtain the crystal form B of hydrochloride.

5. Crystal form A of sulphate

20 mg of the amorphous free base was weighed, 0.1 mL of ethanol was added, and the mixture was stirred at 50°C for the amorphous form to undergo crystal form transformation to a suspension. 0.042 mL of 1 M solution of hydrogen sulphide in methanol was added to the system, which became a red solution after the addition of acid. The system was cooled to room temperature and stirred for a given time, and then a large amount of solid precipitated. The mixture was centrifuged to obtain a solid, which was then dried to finally obtain the crystal form A of sulphate.

6. Crystal form A of hydrobromide

20 mg of the amorphous free base was weighed, 0.1 mL of ethanol was added, and the mixture was stirred at 50°C for the amorphous form to undergo crystal form transformation to a suspension. 0.042 mL of 1 M solution of hydrogen bromide in methanol was added to the system, which became a red solution after the addition of acid. The system was cooled to room temperature and stirred for a given time, and then a large amount of solid precipitated. The mixture was centrifuged to obtain a solid, which was then dried to finally obtain the crystal form A of hydrobromide.

7. Crystal form B of hydrobromide

20 mg of the amorphous free base was weighed, 0.2 mL of ethyl acetate was added, and the mixture was dissolved to give a clear solution at room temperature. 0.042 mL of 1 M solution of hydrogen bromide in methanol was added to the system, which was clear after the addition of acid. The system was stirred for a given time, and then a large amount of orange-yellow solid precipitated. The mixture was reacted at room temperature for 2 hours, and centrifuged to obtain a solid, which was then dried to finally obtain the crystal form B of hydrobromide.

8. Crystal form A of citrate

20 mg of the amorphous free base and 8.079 mg of citric acid monohydrate were weighed and mixed, 0.3 mL of ethyl acetate was added, and the mixture was reacted at 40°C. After a given time, a beige solid precipitated. The mixture was cooled to room temperature, stirred for 2 hours, and then centrifuged to obtain a solid, which was then dried to finally obtain the crystal form A of citrate.

9. Crystal form A of oxalate

20 mg of the amorphous free base and 4.8 mg of oxalic acid dihydrate were weighed and mixed, 0.3 mL of ethyl acetate was added, and the mixture was reacted at 40°C. After a given time, a gel-like solid precipitated. The mixture was cooled to room temperature, stirred for 2 hours, and then centrifuged to obtain a solid, which was then dried to finally obtain the crystal form A of oxalate.

10. Crystal form A of maleate

20 mg of the amorphous free base and 4.56 mg of maleic acid were weighed and mixed, 0.3 mL of ethyl acetate was added, and the mixture was reacted at 40°C. After a given time, a light yellow solid precipitated. The mixture was cooled to room temperature, stirred for 2 hours, and then centrifuged to obtain a solid, which was then dried to finally obtain the crystal form A of maleate.

11. Crystal form A of salicylate

20 mg of the amorphous free base and 5.3 mg of salicylic acid were weighed and mixed, 0.3 mL of ethyl acetate was added, and the mixture was reacted at 40°C. After a given time, a yellow solid precipitated. The mixture was cooled to room temperature, stirred for 2 hours, and then centrifuged to obtain a solid, which was then dried to finally obtain the crystal form A of salicylate.

12. Crystal form A of p-hydroxybenzoate

20 mg of the amorphous free base and 5.3 mg of p-hydroxybenzoic acid were weighed and mixed, 0.3 mL of ethyl acetate was added, and the mixture was reacted at 40°C for 1 hour. No solid precipitated. The system was cooled to room temperature and stirred for a given time, and then a light yellow solid precipitated. The mixture was centrifuged to obtain a solid, which was then dried to finally obtain the crystal form A of p-hydroxybenzoate.

**2. Hygroscopicity experiment**

2.1 Experimental objective: The hygroscopicity of different crystal forms of salts of the compound was investigated under various relative humidity conditions to provide a basis for screening and storage of the crystal forms of salts of the compound.
2.2 Experimental scheme: The crystal forms of salts of the compound were placed in saturated water vapor at different relative humidities to reach a dynamic equilibrium between the compound and the water vapor. The percentage weight gain of the crystal forms of salts of the compound due to moisture absorption was calculated after the equilibrium.
2.3 Experimental results:
2.3.1 Hygroscopicity of crystal forms of salts

1) The crystal form A of phosphate had a weight gain of 0.533% after moisture absorption under RH 80% condition, indicating weak hygroscopicity. After 1 cycle of moisture absorption and desorption under 0-95% relative humidity condition, there was no change in the XRPD pattern of the crystal form A of phosphate, that is, no crystal form transformation occurred.
2) The crystal form B of hydrochloride had a weight gain of 1.157% after moisture absorption under RH 80% condition, indicating weak hygroscopicity. In addition, after 1 cycle of moisture absorption and desorption under 0-95% relative humidity condition, there was no change in the XRPD pattern of the crystal form B of hydrochloride, that is, no crystal form transformation occurred.
3) The crystal form A of sulphate had a weight gain of 4.616% after moisture absorption under RH 80% condition, indicating hygroscopicity. In addition, after 1 cycle of moisture absorption and desorption under 0-95% relative humidity condition, there was no change in the XRPD pattern of the crystal form A of sulphate, that is, no crystal form transformation occurred.
4) The crystal form A of citrate had a weight gain of 0.573% after moisture absorption under RH 80% condition, indicating weak hygroscopicity. In addition, after 1 cycle of moisture absorption and desorption under 0-95% relative humidity condition, there was no change in the XRPD pattern of the crystal form A of citrate, that is, no crystal form transformation occurred.
5) The crystal form A of oxalate had a weight gain of 1.707% after moisture absorption under RH 80% condition, indicating weak hygroscopicity. In addition, after 1 cycle of moisture absorption and desorption under 0-95% relative humidity condition, there was no change in the XRPD pattern of the crystal form A of oxalate, that is, no crystal form transformation occurred.
6) The crystal form A of maleate had a weight gain of 2.050% after moisture absorption under RH 80% condition, indicating hygroscopicity. In addition, after 1 cycle of moisture absorption and desorption under 0-95% relative humidity condition, there was no change in the XRPD pattern of the crystal form A of maleate, that is, no crystal form transformation occurred.
7) The crystal form A of salicylate had a weight gain of 0.1374% after moisture absorption under RH 80% condition, indicating weak hygroscopicity. In addition, after 1 cycle of moisture absorption and desorption under 0-95% relative humidity condition, there was no change in the XRPD pattern of the crystal form A of salicylate, that is, no crystal form transformation occurred.

2.4 Experimental conclusion: The above-mentioned crystal forms did not undergo crystal form transformation under different relative humidity conditions, which is beneficial to the stability and long-term storage of the samples.

**3. Solid stability experiment**

3.1 Experimental objective: To investigate the physical and chemical stability for different crystal forms of salts under the conditions of a high temperature of 60°C (GW), a high humidity of RH = 92.5% (GS), and both a high temperature of 50°C and a high humidity of RH = 75% (JS) and provide a basis for the screening of the crystal forms of salts and the storage of the crystal forms of salts of the compound.
3.2 Experimental scheme: Different crystal forms of salts (crystal form A of hydrochloride, crystal form A of sulphate, crystal form A of citrate, crystal form A of oxalate, crystal form A of maleate and crystal form A of salicylate) were accurately weighed, and the stability of the crystal forms was investigated for 7 and 14 days under the three conditions of 60°C (GW), 92.5% RH (room temperature, GS), and 50°C/75% RH (JS). The changes of related substances were

calculated for the salts by a chromatographic peak area normalization method (see 1.2.2 for the chromatographic conditions).

3.3 Experimental results: as shown in Table 24 below.

Table 24

| Condition \ Days | Stability result (% maximum increase in single impurity) | |
|---|---|---|
| | **7 days** | **14 days** |
| **Crystal form A of hydrochloride high temperature 60°C** | 0.79 | 1.10 |
| **Crystal form A of hydrochloride room temperature and high humidity (92.5% RH)** | <0.05 | <0.05 |
| **Crystal form A of hydrochloride high temperature and high humidity (50°C, 75%RH)** | 0.20 | 0.17 |
| **Crystal form A of sulphate high temperature 60°C** | 0.21 | 0.32 |
| **Crystal form A of sulphate room temperature and high humidity (92.5% RH)** | <0.05 | <0.05 |
| **Crystal form A of sulphate high temperature and high humidity (50°C, 75%RH)** | <0.05 | 0.08 |
| **Crystal form A of citrate high temperature 60°C** | <0.05 | <0.05 |
| **Crystal form A of citrate room temperature and high humidity (92.5% RH)** | <0.05 | <0.05 |
| **Crystal form A of citrate high temperature and high humidity (50°C, 75%RH)** | <0.05 | <0.05 |
| **Crystal form A of oxalate high temperature 60°C** | <0.05 | <0.05 |

| | | |
|---|---|---|
| Crystal form A of oxalate room temperature and high humidity (92.5% RH) | <0.05 | <0.05 |
| Crystal form A of oxalate high temperature and high humidity (50°C, 75%RH) | <0.05 | <0.05 |
| Crystal form A of maleate high temperature 60°C | <0.05 | <0.05 |
| Crystal form A of maleate room temperature and high humidity (92.5% RH) | <0.05 | <0.05 |
| Crystal form A of maleate high temperature and high humidity (50°C, 75%RH) | <0.05 | 0.17 |
| Crystal form A of salicylate high temperature 60°C | <0.05 | <0.05 |
| Crystal form A of salicylate room temperature and high humidity (92.5% RH) | <0.05 | <0.05 |
| Crystal form A of salicylate high temperature and high humidity (50°C, 75%RH) | <0.05 | 0.20 |

3.4 Experimental conclusion: From the stability data, it can be seen that crystal form A of citrate and the crystal form A of oxalate were both relatively stable under high temperature, high humidity, and both high temperature and high humidity conditions, and no significant impurity increase was found. Therefore, they can well meet the requirements of subsequent drug development. The crystal form A of maleate and the crystal form A of salicylate were relatively stable under high temperature and high humidity conditions, and no significant impurity increase was found, indicating that the crystal form A of maleate and the crystal form A of salicylate were insensitive to both the changes of temperature and humidity. Therefore, they both satisfy the requirements for subsequent drug storage. The crystal form A of hydrochloride and the crystal form A of sulphate could both remain stable after being placed for a period of time under various conditions, and no significant impurity increase was found, indicating that the crystal form A of hydrochloride and the crystal form A of sulphate could effectively improve the operational flexibility in the subsequent development of drug preparations.

**4. Solubility experiments in different media**

4.1 Experimental objective: The solubility of the crystal form A of phosphate, crystal form A of hydrochloride, crystal form A of sulphate, crystal form A of citrate, crystal form A of oxalate, crystal form A of maleate and crystal form A of salicylate in media such as artificial simulated gastric fluid (FaSSGF), fasted-state artificial simulated intestinal fluid (FaSSIF), and fed-state artificial simulated intestinal fluid (FeSSIF) was compared to provide a basis for the evaluation of the potential of the crystal forms of salts being manufactured as a drug.

4.2 Experimental scheme: About 2 mg of the compound was suspended in different media for 24 hours, and the thermodynamic solubility of the compound was determined at 37°C by HPLC and an external standard method.

4.3 Experimental results: as shown in the table below.

Table 25

| | FaSSIF | FeSSIF | FaSSGF |
|---|---|---|---|
| Crystal form A of phosphate | 0.203 | 1.196 | 0.010 |
| Crystal form A of hydrochloride | 0.214 | >1.016 | 0.072 |
| Crystal form A of sulphate | 0.034 | 1.114 | 0.096 |

(continued)

| | FaSSIF | FeSSIF | FaSSGF |
|---|---|---|---|
| Crystal form A of citrate | 0.159 | >0.870 | 0.053 |
| Crystal form A of oxalate | 0.035 | 1.228 | 0.070 |
| Crystal form A of maleate | 0.029 | >1.020 | 0.075 |
| Crystal form A of salicylate | 0.101 | 0.672 | 0.020 |

4.4 Experimental conclusion: The above-mentioned crystal forms of salts all have good solubility in various media and meet the requirements of being manufactured as a drug.

**5. Solid stability experiment**

5.1 Experimental objective: To investigate and compare the stability of the crystal form A of phosphate under the three conditions of a high temperature of 60°C (GW, sealed), 50°C/75% RH (JS, unsealed), and room temperature/92.5% RH (GS, unsealed) and provide a basis for the screening of the crystal forms of salts and the storage of the salts of the compound.

5.2 Experimental scheme: About 2 mg of crystal form A of phosphate was weighed into 2 mL dry and clean glass vials, respectively. The vials were respectively placed under the conditions of 60°C (GW, sealed), 50°C/75% RH (JS, unsealed), and room temperature/92.5% RH (GS, unsealed) for 5, 10, 20 and 30 days. Samples were collected at different time points to investigate the changes in its content and related substances by HPLC analysis (see 1.2.3 for the chromatographic conditions).

5.3 Experimental results: as shown in the table below.

Table 26

| Days / Condition | Stability result of crystal form A of phosphate (% maximum increase in single impurity) | | | |
|---|---|---|---|---|
| | 5 days | 10 days | 20 days | 30 days |
| **High temperature 60°C** | <0.10 | <0.10 | <0.10 | <0.10 |
| **Room temperature and high humidity (92.5% RH)** | <0.10 | <0.10 | <0.10 | <0.10 |
| **High temperature and high humidity (50°C, 75%RH)** | <0.10 | <0.10 | <0.10 | <0.10 |

5.4 Experimental conclusion: The crystal form A of phosphate was relatively stable under high temperature, high humidity, and both high temperature and high humidity conditions, and no significant impurity increase was found. Therefore, the crystal form can well meet the requirements of subsequent long-term storage and formulation processes.

**IV. Study on crystal form of free base of Example 2**

1. **Preparation of different crystal forms of free base**

(1) Preparation of crystal form A: About 20 mg of the amorphous free base was weighed into a 2 mL glass vial, and 200 μL of methanol or ethanol was added. The mixture was stirred at room temperature for 2 hours and a solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form A was obtained. After detection and analysis, the crystal form A had an XRPD diagram as shown in FIG. 25 and a DSC diagram as shown in FIG. 26 as follows.

(2) Preparation of crystal form B: About 20 mg of the amorphous free base was weighed into a 2 mL glass vial, and 200 μL of acetonitrile was added. The mixture was stirred at room temperature for 2 hours and a solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form C was obtained. After detection and analysis, the crystal form B had an XRPD diagram as shown in FIG. 27 and a DSC diagram as shown in FIG. 28 as follows.

(3) Preparation of crystal form C: About 20 mg of the amorphous free base was weighed into a 2 mL glass vial, and 200 μL of 1,4-dioxane was added. The mixture was stirred at room temperature for 2 hours and a solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form B was obtained. After detection and analysis, the crystal form C had an XRPD diagram as shown in FIG. 29 and a DSC diagram as shown in FIG. 30 as follows.

(4) Preparation of crystal form D: About 20 mg of the amorphous free base was weighed into a 2 mL glass vial, and 200 μL of water was added. The mixture was stirred at room temperature for 2 hours and a solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form D was obtained. After detection and analysis, the crystal form D had an XRPD diagram as shown in FIG. 31 and a DSC diagram as shown in FIG. 32 as follows.

(5) Preparation of crystal form E: About 10 mg of the amorphous free base was weighed into a 2 mL glass vial, 100 μL of ethyl formate was added, and the mixture was dissolved to give a clear solution. An appropriate amount of water or n-heptane was then added, and the mixture was stirred at room temperature for 24 hours. A solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form E was obtained. After detection and analysis, the crystal form E had an XRPD diagram as shown in FIG. 33 and a DSC diagram as shown in FIG. 34 as follows.

(6) Preparation of crystal form F: About 20 mg of the amorphous free base was weighed into a 2 mL glass vial, and 200 μL of ethyl formate was added. The mixture was stirred at room temperature for 2 hours and a solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form F was obtained. After detection and analysis, the crystal form F had an XRPD diagram as shown in FIG. 35 and a DSC diagram as shown in FIG. 36 as follows.

(7) Preparation of crystal form G: About 20 mg of the amorphous free base was weighed into a 2 mL glass vial, and 200 μL of N,N-dimethylacetamide was added. The mixture was stirred at room temperature for 2 hours and a solid precipitated. The sample was centrifuged at 10000 rpm for 3 min, and the supernatant was removed. The resulting solid was vacuum-dried in a vacuum drying oven at 40°C. The vacuum-dried samples were characterized by XRD analysis. The crystal form G was obtained. After detection and analysis, the crystal form G had an XRPD diagram as shown in FIG. 37 and a DSC diagram as shown in FIG. 38 as follows.

## 2. Stability experiment of solid crystal forms of free base

2.1 Experimental objective: To investigate the physical and chemical stability for crystal form A of free base under the conditions of a high temperature of 60°C, room temperature and a high humidity of 92.5% RH and 50°C, RH = 75% for 5, 10, 20 and 30 days and provide a basis for the storage of the compound.

2.2 Experimental scheme: About 2 mg of crystal form A of free base was taken and investigated under the conditions of 60°C in an oven, room temperature and a high humidity of RH = 92.5%, and 50°C, RH = 75% for 5, 10, 20 and 30 days. The content was determined by HPLC and an external standard method, and the changes of related substances were calculated by a chromatographic peak area normalization method (see 1.2.3 for the chromatographic conditions).

2.3 Experimental results: Physical and chemical stability results of crystal form A of free base under different conditions are shown in Table 27 below:

Table 27

| Condition \ Days | Stability result of crystal form A of free base (% maximum increase in single impurity) | | | |
|---|---|---|---|---|
| | 5 days | 10 days | 20 days | 30 days |
| High temperature 60°C | <0.05 | <0.05 | <0.05 | <0.10 |
| Room temperature and high humidity (92.5% RH) | <0.05 | <0.05 | <0.05 | <0.10 |
| High temperature and high humidity (50°C, 75%RH) | <0.05 | <0.05 | <0.05 | <0.10 |

Experimental conclusion: The crystal form A of free base exhibited excellent stability under all conditions and can well meet the requirements of subsequent long-term storage and formulation processes.

### 3. Hygroscopicity experiment

3.1 Experimental objective: The hygroscopicity of crystal form A of free base of the compound was investigated under various relative humidity conditions to provide a basis for screening and storage of the crystal forms of the compound.

3.2 Experimental scheme: The crystal form A of free base of the compound was placed in saturated water vapor at different relative humidities to reach a dynamic equilibrium between the compound and the water vapor. The percentage weight gain of the compound due to moisture absorption was calculated after the equilibrium.

3.3 Experimental results: The crystal form A of free base has a weight gain after moisture absorption of about 0.1719% under the condition of RH 80%, indicating weak hygroscopicity. After 1 cycle of moisture absorption and desorption under 0-95% relative humidity, there was no change in the XRPD pattern of the crystal form A of free base, i.e., no crystal form transformation occurred, which is beneficial to the stability and long-term storage of the samples.

### 4. Solubility experiments in different media

4.1 Experimental objective: The solubility of the crystal form A of free base in media such as pH1 buffer, artificial simulated gastric fluid (FaSSGF), fasted-state artificial simulated intestinal fluid (FaSSIF), and fed-state artificial simulated intestinal fluid (FeSSIF) was tested to provide a basis for the evaluation of the potential of the crystal form being manufactured as a drug.

4.2 Experimental scheme: About 1.0 mg of crystal form A of free base was weighed into 2 mL glass vials, and pH1 buffer solution, fasted-state artificial simulated intestinal fluid (FaSSIF), fed-state artificial simulated intestinal fluid (FeSSIF), and fasted-state simulated gastric fluid (FaSSGF) (1 mL for each medium) were added to each vial, respectively. The vials were placed on a micromixer for shaking overnight with the temperature set at 37°C. After 24 h, the sample solutions were filtered through 0.45 μm nylon filter membranes, and the filtrates were collected. The concentrations were determined by an external standard method and HPLC.

4.3 Experimental results: as shown in Table 28

Table 28

| | Crystal form A of free base |
|---|---|
| Vehicle | Solubility mg/mL |
| pH 1 | 0.558 |
| FaSSIF (simulated intestinal fluid, fasted) | 0.010 |
| FeSSIF (simulated intestinal fluid, fed) | 0.209 |
| FaSSGF (simulated gastric fluid, fasted) | 0.008 |
| Conclusion: The crystal form A of free base has good solubility in various media, and meets the requirements of being manufactured as a drug. | |

## Claims

1. A crystal form of a compound as shown in general formula (I), or a stereoisomer thereof, or an acid addition salt of any of the foregoing,

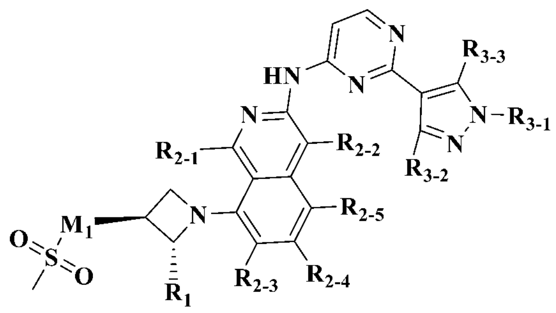

(I)

**characterized in that**:

$M_1$ is independently selected from a bond, $NR_4$ or $CR_5R_6$;
$R_1$ is selected from hydrogen, deuterium, halogen, $C_{1-6}$ alkyl, halogenated $C_{1-6}$ alkyl or deuterated $C_{1-6}$ alkyl;
$R_{2-1}$, $R_{2-2}$, $R_{2-3}$ and $R_{2-4}$ are each independently selected from hydrogen, deuterium, halogen, $C_{1-6}$ alkyl, halogenated $C_{1-6}$ alkyl or deuterated $C_{1-6}$ alkyl;
$R_{2-5}$ is selected from amino, $C_{1-6}$ alkyl, halogenated $C_{1-6}$ alkyl or deuterated $C_{1-6}$ alkyl, and the amino, $C_{1-6}$ alkyl, halogenated $C_{1-6}$ alkyl or deuterated $C_{1-6}$ alkyl may be optionally further substituted with one or more of deuterium, halogen, cyano, hydroxyl, nitro, $C_{1-6}$ alkyl, halogenated $C_{1-6}$ alkyl and deuterated $C_{1-6}$ alkyl;
$R_{3-1}$, $R_{3-2}$ and $R_{3-3}$ are each independently selected from hydrogen, deuterium, halogen, $C_{1-6}$ alkyl, halogenated $C_{1-6}$ alkyl or deuterated $C_{1-6}$ alkyl;
$R_4$ is selected from hydrogen, deuterium, $C_{1-6}$ alkyl, halogenated $C_{1-6}$ alkyl or deuterated $C_{1-6}$ alkyl;
$R_5$ and $R_6$ are each independently selected from hydrogen, deuterium, halogen, $C_{1-6}$ alkyl, halogenated $C_{1-6}$ alkyl or deuterated $C_{1-6}$ alkyl;
the acid in the acid addition salt is selected from an inorganic acid or an organic acid, wherein the inorganic acid is selected from hydrochloric acid, sulphuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid, or L-malic acid;
preferably, the acid in the acid addition salt is selected from phosphoric acid, hydrochloric acid, sulphuric acid, hydrobromic acid, citric acid, oxalic acid, maleic acid, salicylic acid or p-hydroxybenzoic acid.

2. The crystal form or the acid addition salt according to claim 1, **characterized in that**

$R_1$ is selected from hydrogen, deuterium, halogen, $C_{1-3}$ alkyl, halogenated $C_{1-3}$ alkyl or deuterated $C_{1-3}$ alkyl;
$R_{2-1}$, $R_{2-2}$, $R_{2-3}$ and $R_{2-4}$ are each independently selected from hydrogen, deuterium, halogen, $C_{1-3}$ alkyl, halogenated $C_{1-3}$ alkyl or deuterated $C_{1-3}$ alkyl;
$R_{2-5}$ is selected from amino, $C_{1-3}$ alkyl, halogenated $C_{1-3}$ alkyl or deuterated $C_{1-3}$ alkyl, and the amino, $C_{1-3}$ alkyl, halogenated $C_{1-3}$ alkyl or deuterated $C_{1-3}$ alkyl may be optionally further substituted with one or more of

deuterium, halogen, cyano, hydroxyl, nitro, $C_{1-3}$ alkyl, halogenated $C_{1-3}$ alkyl and deuterated $C_{1-3}$ alkyl;
$R_{3-1}$, $R_{3-2}$ and $R_{3-3}$ are each independently selected from hydrogen, deuterium, halogen, $C_{1-3}$ alkyl, halogenated $C_{1-3}$ alkyl or deuterated $C_{1-3}$ alkyl;
$R_4$ is selected from hydrogen, deuterium, $C_{1-3}$ alkyl, halogenated $C_{1-3}$ alkyl or deuterated $C_{1-3}$ alkyl;
$R_5$ and $R_6$ are each independently selected from hydrogen, deuterium, halogen, $C_{1-3}$ alkyl, halogenated $C_{1-3}$ alkyl or deuterated $C_{1-3}$ alkyl.

**3.** The crystal form or the acid addition salt according to claim 1, **characterized in that** the compound is as shown in any one of:

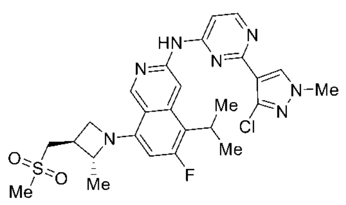
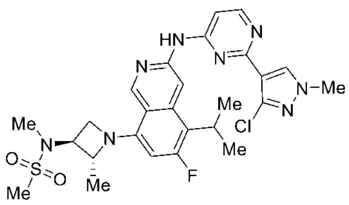
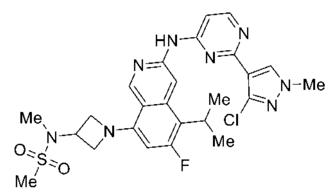
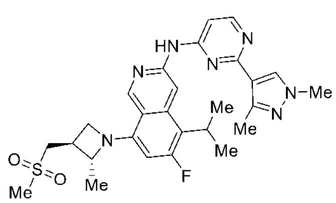
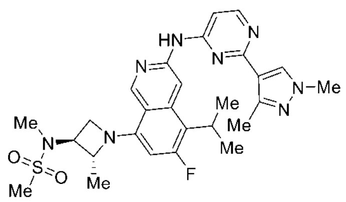
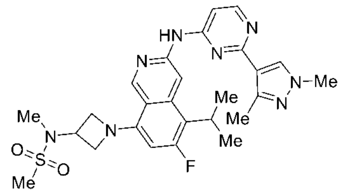
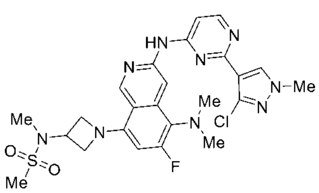
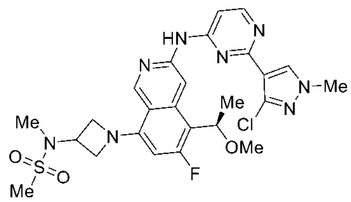
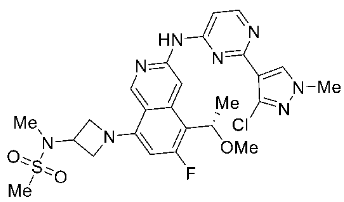
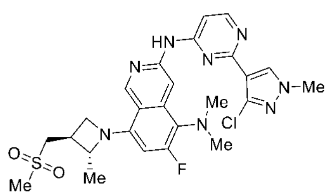
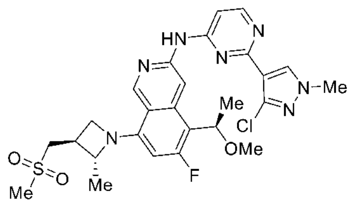
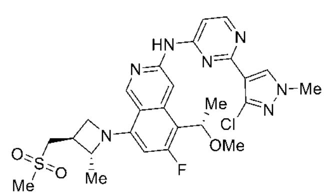
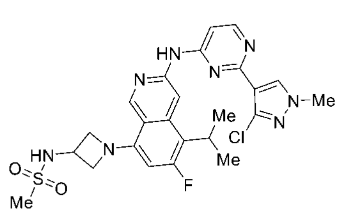
or
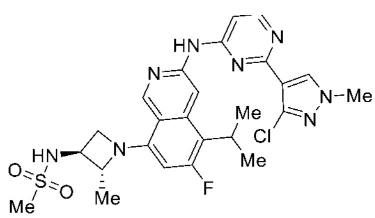
.

**4.** The crystal form or the acid addition salt according to any one of claims 1-3, **characterized in that** the number of the acid molecules in the acid addition salt is 1, 2 or 3.

**5.** The crystal form or the acid addition salt according to any one of claims 1-4, **characterized in that** the acid addition salt is a hydrate or an anhydrate, preferably an anhydrate.

**6.** A crystal form of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, **characterized in that** the crystal form is

crystal form A of phosphate, crystal form B of phosphate, crystal form A of hydrochloride, crystal form B of hydrochloride, crystal form A of sulphate, crystal form A of hydrobromide, crystal form B of hydrobromide, crystal form A of citrate, crystal form A of oxalate, crystal form A of maleate, crystal form A of salicylate or crystal form A of p-hydroxybenzoate, wherein

the crystal form A of phosphate has an X-ray powder diffraction pattern comprising a diffraction peak at 2θ of 4.5 ±0.2°, a diffraction peak at 2θ of 8.8±0.2°, a diffraction peak at 2θ of 10.8±0.2°, a diffraction peak at 2θ of 12.9 ±0.2°, a diffraction peak at 2θ of 14.4±0.2°, a diffraction peak at 2θ of 15.3±0.2°, a diffraction peak at 2θ of 16.3 ±0.2°, a diffraction peak at 2θ of 17.1±0.2°, a diffraction peak at 2θ of 17.9±0.2°, or a diffraction peak at 2θ of 25.2 ±0.2°; preferably comprising any 2-5, 3-5, 3-6, 3-8, 5-8, 6-8, or 8-10 of the diffraction peaks; and more preferably comprising any 6, 7, 8, 9 or 10 of the diffraction peaks;

the crystal form B of phosphate has an X-ray powder diffraction pattern comprising diffraction peaks at 2θ (±0.2°) of 5.3±0.2°, 16.0±0.2°, 19.9±0.2°, 20.3±0.2°, 21.4±0.2° and 22.3±0.2°; preferably, the crystal form B of phosphate has an X-ray powder diffraction pattern further comprising diffraction peaks at 2θ (±0.2°) of 12.5±0.2° and 13.0±0.2°; more preferably, the crystal form B of phosphate has an X-ray powder diffraction pattern further comprising diffraction peaks at 2θ (±0.2°) of 14.1±0.2° and 24.7±0.2°;

the crystal form A of hydrochloride has an X-ray powder diffraction pattern comprising diffraction peaks at 2θ (±0.2°) of 6.4±0.2°, 7.1±0.2°, 14.2±0.2°, 19.3±0.2°, 24.6±0.2° and 25.1±0.2°; preferably, the crystal form A of hydrochloride has an X-ray powder diffraction pattern further comprising diffraction peaks at 20 (±0.2°) of 9.3 ±0.2° and 12.7±0.2°; more preferably, the crystal form A of hydrochloride has an X-ray powder diffraction pattern further comprising diffraction peaks at 2θ (±0.2°) of 11.2±0.2° and 15.5±0.2°;

the crystal form B of hydrochloride has an X-ray powder diffraction pattern comprising diffraction peaks at 2θ (±0.2°) of 9.6±0.2°, 15.8±0.2°, 16.9±0.2°, 25.7±0.2°, 27.5±0.2° and 27.9±0.2°; preferably, the crystal form B of hydrochloride has an X-ray powder diffraction pattern further comprising diffraction peaks at 2θ (±0.2°) of 15.1 ±0.2° and 23.4±0.2°; more preferably, the crystal form B of hydrochloride has an X-ray powder diffraction pattern further comprising diffraction peaks at 2θ (±0.2°) of 19.1±0.2° and 26.2±0.2°;

the crystal form A of sulphate has an X-ray powder diffraction pattern comprising diffraction peaks at 2θ (±0.2°) of 5.5±0.2°, 6.1±0.2°, 6.3±0.2°, 16.1±0.2°, 26.1±0.2° and 26.8±0.2°; preferably, the crystal form A of sulphate has an X-ray powder diffraction pattern further comprising diffraction peaks at 2θ (±0.2°) of 15.7±0.2° and 22.9 ±0.2°; more preferably, the crystal form A of sulphate has an X-ray powder diffraction pattern further comprising diffraction peaks at 2θ (±0.2°) of 14.1±0.2° and 22.0±0.2°;

the crystal form A of hydrobromide has an X-ray powder diffraction pattern comprising diffraction peaks at 2θ (±0.2°) of 7.1±0.2°, 14.3±0.2°, 21.6±0.2°, 22.1±0.2°, 25.1±0.2° and 27.0±0.2°; preferably, the crystal form A of hydrobromide has an X-ray powder diffraction pattern further comprising diffraction peaks at 2θ (±0.2°) of 11.3 ±0.2° and 21.2±0.2°; more preferably, the crystal form A of hydrobromide has an X-ray powder diffraction pattern further comprising diffraction peaks at 2θ (±0.2°) of 26.7±0.2° and 30.5±0.2°;

the crystal form B of hydrobromide has an X-ray powder diffraction pattern comprising diffraction peaks at 2θ (±0.2°) of 6.9±0.2°, 18.5±0.2°, 19.4±0.2°, 22.8±0.2°, 24.9±0.2° and 25.3±0.2°; preferably, the crystal form B of hydrobromide has an X-ray powder diffraction pattern further comprising diffraction peaks at 2θ (±0.2°) of 7.1 ±0.2° and 19.7±0.2°; more preferably, the crystal form B of hydrobromide has an X-ray powder diffraction pattern further comprising diffraction peaks at 2θ (±0.2°) of 15.3±0.2° and 22.6±0.2°;

the crystal form A of citrate has an X-ray powder diffraction pattern comprising diffraction peaks at 2θ (±0.2°) of 12.2±0.2°, 16.7±0.2°, 18.7±0.2°, 19.4±0.2°, 21.9±0.2° and 22.3±0.2°; preferably, the X-ray powder diffraction pattern further comprises diffraction peaks at 2θ (±0.2°) of 16.2±0.2° and 23.5±0.2°; more preferably, the X-ray powder diffraction pattern further comprises diffraction peaks at 2θ (±0.2°) of 11.7±0.2° and 15.6±0.2°;

the crystal form A of oxalate has an X-ray powder diffraction pattern comprising diffraction peaks at 2θ (±0.2°) of 4.3±0.2°, 17.5±0.2°, 17.8±0.2°, 18.2±0.2°, 20.7±0.2° and 25.8±0.2°; preferably, the crystal form A of oxalate has an X-ray powder diffraction pattern further comprising diffraction peaks at 2θ (±0.2°) of 8.4±0.2° and 16.8 ±0.2°; more preferably, the crystal form A of oxalate has an X-ray powder diffraction pattern further comprising diffraction peaks at 2θ (±0.2°) of 21.0±0.2° and 27.4±0.2°;

the crystal form A of maleate has an X-ray powder diffraction pattern comprising diffraction peaks at 20 (±0.2°) of 7.1±0.2°, 8.1±0.2°, 17.0±0.2°, 19.0±0.2°, 25.7±0.2° and 26.9±0.2°; preferably, the crystal form A of maleate has an X-ray powder diffraction pattern further comprising diffraction peaks at 2θ (±0.2°) of 9.0±0.2° and 22.9 ±0.2°; more preferably, the crystal form A of maleate has an X-ray powder diffraction pattern further comprising diffraction peaks at 20 (±0.2°) of 13.4±0.2° and 20.6±0.2°;

the crystal form A of salicylate has an X-ray powder diffraction pattern comprising diffraction peaks at 2θ (±0.2°) of 8.3±0.2°, 16.5±0.2°, 18.5±0.2°, 20.4±0.2°, 22.3±0.2° and 24.4±0.2°; preferably, the crystal form A of salicylate has an X-ray powder diffraction pattern further comprising diffraction peaks at 2θ (±0.2°) of 22.0±0.2° and 28.0±0.2°; more preferably, the crystal form A of salicylate has an X-ray powder diffraction pattern further comprising diffraction peaks at 2θ (±0.2°) of 16.3±0.2° and 21.0±0.2°;
the crystal form A of p-hydroxybenzoate has an X-ray powder diffraction pattern comprising diffraction peaks at 2θ (±0.2°) of 5.9±0.2°, 17.2±0.2°, 17.5±0.2°, 18.9±0.2°, 22.3±0.2° and 25.2±0.2°; preferably, the crystal form A of p-hydroxybenzoate has an X-ray powder diffraction pattern further comprising diffraction peaks at 2θ (±0.2°) of 16.1±0.2° and 22.0±0.2°; more preferably, the crystal form A of p-hydroxybenzoate has an X-ray powder diffraction pattern further comprising diffraction peaks at 2θ (±0.2°) of 23.7±0.2° and 25.6±0.2°.

**7.** The crystal form according to claim 6, **characterized in that**

the crystal form A of phosphate has an X-ray powder diffraction pattern comprising at least one or more, preferably 2, and more preferably 3 of the diffraction peaks at 2θ of 4.5±0.2°, 8.8±0.2°, and 10.8±0.2°; optionally, the X-ray powder diffraction pattern can further comprise at least one, preferably 2, 3, 4 or 5 of the diffraction peaks at 2θ of 12.9±0.2°, 14.4±0.2°, 16.3±0.2°, and 17.1±0.2°;
preferably, the crystal form A of phosphate has an X-ray powder diffraction pattern optionally further comprising one or more, preferably at least any 2-3 or 4-5, and further preferably any 2, 3, 4 or 5 of the diffraction peaks at 2θ of 9.0±0.2°, 15.3±0.2°, 18.0±0.2°, 19.3±0.2°, 21.8±0.2°, 25.2±0.2°, and 27.9±0.2°;
further preferably, the crystal form A of phosphate has an X-ray powder diffraction pattern comprising diffraction peaks at 2θ of 4.5±0.2° and 8.8±0.2°; preferably, further comprising diffraction peaks at 10.8±0.2° and 17.1 ±0.2°; more preferably, further comprising diffraction peaks at 12.9±0.2° and 14.4±0.2°; further preferably, further comprising diffraction peaks at 15.3±0.2° and 16.3±0.2°; even further preferably, further comprising diffraction peaks at 18.0±0.2° and 25.2±0.2°;
for example, the crystal form A of phosphate has an X-ray powder diffraction pattern comprising diffraction peaks at 2θ of
8.8±0.2°, 10.8±0.2°, 12.9±0.2°, 14.4±0.2°, and 17.1±0.2°;
4.5±0.2°, 10.8±0.2°, 12.9±0.2°, 14.4±0.2°, and 17.1±0.2°;
4.5±0.2°, 8.8±0.2°, 12.9±0.2°, 14.4±0.2°, and 17.1±0.2°;
4.5±0.2°, 8.8±0.2°, 10.8±0.2°, 14.4±0.2°, and 17.1±0.2°;
4.5±0.2°, 8.8±0.2°, 10.8±0.2°, 12.9±0.2°, and 17.1±0.2°;
4.5±0.2°, 8.8±0.2°, 10.8±0.2°, 12.9±0.2°, and 14.4±0.2°;
4.5±0.2°, 8.8±0.2°, 10.8±0.2°, 12.9±0.2°, 14.4±0.2°, and 17.1±0.2°;
8.8±0.2°, 10.8±0.2°, 12.9±0.2°, 14.4±0.2°, 15.3±0.2°, and 17.1±0.2°;
4.5±0.2°, 10.8±0.2°, 12.9±0.2°, 14.4±0.2°, 16.3±0.2°, and 17.1±0.2°;
4.5±0.2°, 8.8±0.2°, 12.9±0.2°, 14.4±0.2°, 15.3±0.2°, and 17.1±0.2°;
4.5±0.2°, 8.8±0.2°, 10.8±0.2°, 14.4±0.2°, 16.3±0.2°, and 17.1±0.2°;
4.5±0.2°, 8.8±0.2°, 10.8±0.2°, 12.9±0.2°, 15.3±0.2°, and 17.1±0.2°;
4.5±0.2°, 8.8±0.2°, 10.8±0.2°, 12.9±0.2°, 14.4±0.2°, and 16.3±0.2°;
8.8±0.2°, 10.8±0.2°, 12.9±0.2°, 14.4±0.2°, 15.3±0.2°, 16.3±0.2°, and 17.1±0.2°;
4.5±0.2°, 10.8±0.2°, 12.9±0.2°, 14.4±0.2°, 15.3±0.2°, 16.3±0.2°, and 17.1±0.2°;
4.5±0.2°, 8.8±0.2°, 12.9±0.2°, 14.4±0.2°, 15.3±0.2°, 16.3±0.2°, and 17.1±0.2°;
4.5±0.2°, 8.8±0.2°, 10.8±0.2°, 14.4±0.2°, 15.3±0.2°, 16.3±0.2°, and 17.1±0.2°;
4.5±0.2°, 8.8±0.2°, 10.8±0.2°, 12.9±0.2°, 15.3±0.2°, 16.3±0.2°, and 17.1±0.2°;
4.5±0.2°, 8.8±0.2°, 10.8±0.2°, 12.9±0.2°, 14.4±0.2°, 16.3±0.2°, and 17.1±0.2°;
4.5±0.2°, 8.8±0.2°, 10.8±0.2°, 12.9±0.2°, 14.4±0.2°, 15.3±0.2°, and 17.1±0.2°;
4.5±0.2°, 8.8±0.2°, 10.8±0.2°, 12.9±0.2°, 14.4±0.2°, 15.3±0.2°, and 16.3±0.2°;
4.5±0.2°, 8.8±0.2°, 10.8±0.2°, 12.9±0.2°, 14.4±0.2°, 15.3±0.2°, 16.3±0.2°, and 17.1±0.2°;
8.8±0.2°, 10.8±0.2°, 12.9±0.2°, 14.4±0.2°, 15.3+0.2°, 16.3±0.2°, 17.1±0.2°, and 18.0±0.2°;
4.5±0.2°, 10.8±0.2°, 12.9±0.2°, 14.4±0.2°, 15.3±0.2°, 16.3±0.2°, 17.1±0.2°, and 25.2±0.2°;
4.5±0.2°, 8.8±0.2°, 12.9±0.2°, 14.4±0.2°, 15.3±0.2°, 16.3±0.2°, 17.1±0.2°, and 18.0±0.2°;
4.5±0.2°, 8.8±0.2°, 10.8±0.2°, 14.4±0.2°, 15.3+0.2°, 16.3±0.2°, 17.1±0.2°, and 25.2±0.2°;
4.5±0.2°, 8.8±0.2°, 10.8±0.2°, 12.9±0.2°, 15.3+0.2°, 16.3±0.2°, 17.1±0.2°, and 18.0±0.2°;
4.5±0.2°, 8.8±0.2°, 10.8±0.2°, 12.9±0.2°, 14.4±0.2°, 16.3±0.2°, 17.1±0.2°, and 25.2±0.2°;
4.5±0.2°, 8.8±0.2°, 10.8±0.2°, 12.9±0.2°, 14.4±0.2°, 15.3±0.2°, 17.1±0.2°, and 18.0±0.2°;
4.5±0.2°, 8.8±0.2°, 10.8±0.2°, 12.9±0.2°, 14.4±0.2°, 15.3±0.2°, 16.3±0.2°, and 25.2±0.2°;
8.8±0.2°, 10.8±0.2°, 12.9±0.2°, 14.4±0.2°, 15.3±0.2°, 16.3±0.2°, 17.1±0.2°, 18.0±0.2°, and 25.2±0.2°;
4.5±0.2°, 9.0±0.2°, 10.8±0.2°, 12.9±0.2°, 14.4±0.2°, 15.3±0.2°, 16.3±0.2°, 17.1±0.2°, 18.0±0.2°, and 25.2 ±0.2°;
4.5±0.2°, 8.8±0.2°, 12.9±0.2°, 14.4±0.2°, 15.3±0.2°, 16.3±0.2°, 17.1±0.2°, 18.0±0.2°, 19.3±0.2°, and 25.2±0.2°;
4.5±0.2°, 8.8±0.2°, 10.8±0.2°, 14.4±0.2°, 15.3±0.2°, 16.3±0.2°, 17.1±0.2°, 18.0±0.2°, 21.8±0.2°, and 25.2 ±0.2°;
4.5±0.2°, 8.8±0.2°, 10.8±0.2°, 12.9±0.2°, 15.3±0.2°, 16.3±0.2°, 17.1±0.2°, 18.0±0.2°, 25.2±0.2°, and 27.9 ±0.2°;
4.5±0.2°, 8.8±0.2°, 9.0±0.2°, 10.8±0.2°, 12.9±0.2°, 14.4±0.2°, 16.3±0.2°, 17.1±0.2°, 18.0±0.2°, and 25.2 ±0.2°;
4.5±0.2°, 8.8±0.2°, 10.8±0.2°, 12.9±0.2°, 14.4±0.2°, 15.3±0.2°, 17.1±0.2°, 18.0±0.2°, 19.3±0.2°, and 25.2 ±0.2°;
4.5±0.2°, 8.8±0.2°, 10.8±0.2°, 12.9±0.2°, 14.4±0.2°, 15.3±0.2°, 16.3±0.2°, 18.0±0.2°, 21.8±0.2°, and 25.2 ±0.2°;
4.5±0.2°, 8.8±0.2°, 10.8±0.2°, 12.9±0.2°, 14.4±0.2°, 15.3±0.2°, 16.3±0.2°, 17.1±0.2°, 25.2±0.2°, and 27.9 ±0.2°;
4.5±0.2°, 8.8±0.2°, 10.8±0.2°, 12.9±0.2°, 14.4±0.2°, 15.3±0.2°, 16.3±0.2°, 17.1±0.2°, 18.0±0.2°, and 19.3 ±0.2°;
4.5±0.2°, 8.8±0.2°, 10.8±0.2°, 12.9±0.2°, 14.4±0.2°, 15.3±0.2°, 16.3±0.2°, 17.1±0.2°, 18.0±0.2°, and 25.2 ±0.2°;
8.8±0.2°, 9.0±0.2°, 10.8±0.2°, 12.9±0.2°, 14.4±0.2°, 15.3±0.2°, 16.3±0.2°, 17.1±0.2°, 18.0±0.2°, and 25.2 ±0.2°;
4.5±0.2°, 10.8±0.2°, 12.9±0.2°, 14.4±0.2°, 15.3±0.2°, 16.3±0.2°, 17.1±0.2°, 18.0±0.2°, 19.3±0.2°, and 25.2 ±0.2°;
4.5±0.2°, 8.8±0.2°, 12.9±0.2°, 14.4±0.2°, 15.3±0.2°, 16.3±0.2°, 17.1±0.2°, 18.0±0.2°, 21.8±0.2°, and 25.2 ±0.2°;
4.5±0.2°, 8.8±0.2°, 10.8±0.2°, 14.4±0.2°, 15.3±0.2°, 16.3±0.2°, 17.1±0.2°, 18.0±0.2°, 25.2±0.2°, and 27.9 ±0.2°;
4.5±0.2°, 8.8±0.2°, 9.0±0.2°, 10.8±0.2°, 12.9±0.2°, 15.3±0.2°, 16.3±0.2°, 17.1±0.2°, 18.0±0.2°, and 25.2 ±0.2°;
4.5±0.2°, 8.8±0.2°, 10.8±0.2°, 12.9±0.2°, 14.4±0.2°, 16.3±0.2°, 17.1±0.2°, 18.0±0.2°, 19.3±0.2°, and 25.2 ±0.2°;
4.5±0.2°, 8.8±0.2°, 10.8±0.2°, 12.9±0.2°, 14.4±0.2°, 15.3±0.2°, 17.1±0.2°, 18.0±0.2°, 21.8±0.2°, and 25.2 ±0.2°;
4.5±0.2°, 8.8±0.2°, 10.8±0.2°, 12.9±0.2°, 14.4±0.2°, 15.3±0.2°, 16.3±0.2°, 18.0±0.2°, 25.2±0.2°, and 27.9 ±0.2°;
4.5±0.2°, 8.8±0.2°, 9.0±0.2°, 10.8±0.2°, 12.9±0.2°, 14.4±0.2°, 15.3±0.2°, 16.3±0.2°, 17.1±0.2°, and 25.2 ±0.2°;
4.5±0.2°, 8.8±0.2°, 10.8±0.2°, 12.9±0.2°, 14.4±0.2°, 15.3±0.2°, 16.3±0.2°, 17.1±0.2°, 18.0±0.2°, and 19.3 ±0.2°;
further preferably, the crystal form A of phosphate has an X-ray powder diffraction pattern substantially as shown in FIG. 1; even further preferably, the crystal form A of phosphate has a DSC pattern substantially as shown in FIG. 2;
the crystal form B of phosphate has an X-ray powder diffraction pattern substantially as shown in FIG. 3;
even further preferably, the crystal form B of phosphate has a DSC pattern substantially as shown in FIG. 4;
the crystal form A of hydrochloride has an X-ray powder diffraction pattern substantially as shown in FIG. 5;
even further preferably, the crystal form A of hydrochloride has a DSC pattern substantially as shown in FIG. 6;
the crystal form B of hydrochloride has an X-ray powder diffraction pattern substantially as shown in FIG. 7;
even further preferably, the crystal form B of hydrochloride has a DSC pattern substantially as shown in FIG. 8;
the crystal form A of sulphate has an X-ray powder diffraction pattern substantially as shown in FIG. 9;
even further preferably, the crystal form A of sulphate has a DSC pattern substantially as shown in FIG. 10;
the crystal form A of hydrobromide has an X-ray powder diffraction pattern substantially as shown in FIG. 11;
even further preferably, the crystal form A of hydrobromide has a DSC pattern substantially as shown in FIG. 12;
the crystal form B of hydrobromide has an X-ray powder diffraction pattern substantially as shown in FIG. 13;
even further preferably, the crystal form B of hydrobromide has a DSC pattern substantially as shown in FIG. 14;
the crystal form A of citrate has an X-ray powder diffraction pattern substantially as shown in FIG. 15;
even further preferably, the crystal form A of citrate has a DSC pattern substantially as shown in FIG. 16;
the crystal form A of oxalate has an X-ray powder diffraction pattern substantially as shown in FIG. 17;
even further preferably, the crystal form A of oxalate has a DSC pattern substantially as shown in FIG. 18;
the crystal form A of maleate has an X-ray powder diffraction pattern substantially as shown in FIG. 19;
even further preferably, the crystal form A of maleate has a DSC pattern substantially as shown in FIG. 20;

the crystal form A of salicylate has an X-ray powder diffraction pattern substantially as shown in FIG. 21; even further preferably, the crystal form A of salicylate has a DSC pattern substantially as shown in FIG. 22; the crystal form A of p-hydroxybenzoate has an X-ray powder diffraction pattern substantially as shown in FIG. 23; even further preferably, the crystal form A of p-hydroxybenzoate has a DSC pattern substantially as shown in FIG. 24.

**8.** The crystal form according to any one of claims 6-7, **characterized in that** in the X-ray powder diffraction patterns of the crystal form A of phosphate, the crystal form B of phosphate, the crystal form A of hydrochloride, the crystal form B of hydrochloride, the crystal form A of sulphate, the crystal form A of hydrobromide, the crystal form B of hydrobromide, the crystal form A of citrate, the crystal form A of oxalate, the crystal form A of maleate, the crystal form A of salicylate, and the crystal form A of p-hydroxybenzoate of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, the positions of the diffraction peaks with the top ten relative peak intensities have a 2θ deviation of ±0.2° to ±0.5°, preferably ±0.2° to ±0.3°, and most preferably ±0.2° from the corresponding positions of the diffraction peaks in FIG. 1, FIG. 3, FIG. 5, FIG. 7, FIG. 9, FIG. 11, FIG. 13, FIG. 15, FIG. 17, FIG. 19, FIG. 21, and FIG. 23, respectively.

**9.** The crystal form or the acid addition salt according to claim 1, **characterized in that** the crystal form is crystal form A, crystal form B, crystal form C, crystal form D, crystal form E, crystal form F or crystal form G of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, wherein

the crystal form A has an X-ray powder diffraction pattern comprising diffraction peaks at 2θ (±0.2°) of 13.0±0.2°, 14.0±0.2°, 15.9±0.2°, 19.8±0.2°, 20.2±0.2° and 22.3±0.2°; preferably, the crystal form A has an X-ray powder diffraction pattern further comprising diffraction peaks at 2θ (±0.2°) of 12.4±0.2° and 24.6±0.2°; more preferably, the crystal form A has an X-ray powder diffraction pattern further comprising diffraction peaks at 2θ (±0.2°) of 5.3 ±0.2° and 21.3±0.2°;
the crystal form B has an X-ray powder diffraction pattern comprising diffraction peaks at 2θ (±0.2°) of 11.3±0.2°, 11.6±0.2°, 17.4±0.2°, 21.9±0.2°, 22.2±0.2° and 23.3±0.2°; preferably, the crystal form B has an X-ray powder diffraction pattern further comprising diffraction peaks at 2θ (±0.2°) of 12.0±0.2° and 15.5±0.2°; more preferably, the crystal form B has an X-ray powder diffraction pattern further comprising diffraction peaks at 2θ (±0.2°) of 15.9 ±0.2° and 27.2±0.2°;
the crystal form C has an X-ray powder diffraction pattern comprising diffraction peaks at 2θ (±0.2°) of 11.6±0.2°, 18.5±0.2°, 19.3±0.2°, 21.5±0.2°, 23.2±0.2° and 23.4±0.2°; preferably, the crystal form C has an X-ray powder diffraction pattern further comprising diffraction peaks at 2θ (±0.2°) of 7.2±0.2° and 18.8±0.2°; more preferably, the crystal form C has an X-ray powder diffraction pattern further comprising diffraction peaks at 2θ (±0.2°) of 8.6 ±0.2° and 22.3±0.2°;
the crystal form D has an X-ray powder diffraction pattern comprising diffraction peaks at 2θ (±0.2°) of 12.2±0.2°, 16.1±0.2°, 19.7±0.2°, 21.2±0.2°, 21.9±0.2° and 24.0±0.2°; preferably, the crystal form D has an X-ray powder diffraction pattern further comprising diffraction peaks at 2θ (±0.2°) of 15.9±0.2° and 21.4±0.2°; more preferably, the crystal form D has an X-ray powder diffraction pattern further comprising diffraction peaks at 2θ (±0.2°) of 24.5 ±0.2° and 29.0±0.2°;
the crystal form E has an X-ray powder diffraction pattern comprising diffraction peas at 2θ (±0.2°) of 12.3±0.2°, 16.6±0.2°, 20.0±0.2°, 21.2±0.2°, 21.6±0.2° and 24.4±0.2°; preferably, the crystal form E has an X-ray powder diffraction pattern further comprising diffraction peaks at 2θ (±0.2°) of 8.1±0.2° and 24.1±0.2°; more preferably, the X-ray powder diffraction pattern further comprises diffraction peaks at 2θ (±0.2°) of 16.0±0.2° and 19.8±0.2°;
the crystal form F has an X-ray powder diffraction pattern comprising diffraction peaks at 2θ (±0.2°) of 14.2±0.2°, 17.2±0.2°, 19.7±0.2°, 21.2±0.2°, 25.3±0.2° and 26.2±0.2°; preferably, the crystal form F has an X-ray powder diffraction pattern further comprising diffraction peaks at 2θ (±0.2°) of 6.6±0.2° and 23.3±0.2°; more preferably, the crystal form F has an X-ray powder diffraction pattern further comprising diffraction peaks at 2θ (±0.2°) of 15.1 ±0.2° and 18.1±0.2°;
the crystal form G has an X-ray powder diffraction pattern comprising diffraction peaks at 2θ (±0.2°) of 10.4±0.2°, 16.3±0.2°, 18.8+0.2°, 21.3+0.2°, 22.2±0.2° and 23.9±0.2°; preferably, the crystal form G has an X-ray powder diffraction pattern further comprising diffraction peaks at 2θ (±0.2°) of 9.4±0.2° and 25.7±0.2°; more preferably, the crystal form G has an X-ray powder diffraction pattern further comprising diffraction peaks at 2θ (±0.2°) of 16.6 ±0.2° and 23.2±0.2°.

**10.** The crystal form or the acid addition salt according to claim 9, **characterized in that** the crystal form A has an X-ray powder diffraction pattern substantially as shown in FIG. 25;

even further preferably, the crystal form A has a DSC pattern as shown in FIG. 26;
the crystal form B has an X-ray powder diffraction pattern substantially as shown in FIG. 27;
even further preferably, the crystal form B has a DSC pattern substantially as shown in FIG. 28;
the crystal form C has an X-ray powder diffraction pattern substantially as shown in FIG. 29;
even further preferably, the crystal form C has a DSC pattern substantially as shown in FIG. 30;
the crystal form D has an X-ray powder diffraction pattern substantially as shown in FIG. 31;
even further preferably, the crystal form D has a DSC pattern substantially as shown in FIG. 32;
the crystal form E has an X-ray powder diffraction pattern substantially as shown in FIG. 33;
even further preferably, the crystal form E has a DSC pattern substantially as shown in FIG. 34;
the crystal form F has an X-ray powder diffraction pattern substantially as shown in FIG. 35;
even further preferably, the crystal form F has a DSC pattern substantially as shown in FIG. 36;
the crystal form G has an X-ray powder diffraction pattern substantially as shown in FIG. 37;
even further preferably, the crystal form G has a DSC pattern substantially as shown in FIG. 38.

**11.** The crystal form or the acid addition salt according to any one of claims 9-10, **characterized in that** in the X-ray powder diffraction patterns of the crystal form A, the crystal form B, the crystal form C, the crystal form D, the crystal form E, the crystal form F, and the crystal form G of N-((2R,3S)-1-(3-((2-(3-chloro-1-methyl-1H-pyrazol-4-yl)pyrimidin-4-yl)amino)-6-fluoro-5-isopropylisoquinolin-8-yl)-2-methylazetidin-3-yl)-N-methylmethanesulphonamide, the positions of the diffraction peaks with the top ten relative peak intensities have a 2θ deviation of ±0.2° to +0.5°, preferably ±0.2° to ±0.3°, and most preferably ±0.2° from the corresponding positions of the diffraction peaks in FIG. 25, FIG. 27, FIG. 29, FIG. 31, FIG. 33, FIG. 35, and FIG. 37, respectively.

**12.** A method for preparing the crystal form or the acid addition salt according to any one of claims 1-5, **characterized in that** the method involves method I or method II:

method I comprises the following steps:

1) dissolving the compound in an organic solvent;
2) adding a counterion acid, wherein the amount of the counterion acid is preferably 1.2 equivalents; the counterion acid can be dissolved in an organic solvent;
3) stirring and mixing, and evaporating the solvent;
4) performing separation to obtain the acid addition salt;

method II comprises the following steps:

1) suspending the compound in an organic solvent;
2) adding a counterion acid, wherein the amount of the counterion acid is preferably 1.2 equivalents; the counterion acid can be dissolved in an organic solvent;
3) stirring until dissolution, and then further stirring to allow precipitation;
4) performing separation to obtain the acid addition salt;

wherein: the solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, ethanol, acetone or ethyl acetate;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid,

p-hydroxybenzoic acid or L-malic acid.

**13.** A method for preparing the crystal form according to any one of claims 6-8, **characterized in that** the method involves method I or method II:

method I comprises the following steps:

1) weighing an appropriate amount of the compound and dissolving the compound in a good solvent;
2) weighing an appropriate amount of a counterion acid and dissolving the counterion acid in an organic solvent, wherein the amount of the counterion acid is preferably 1.2 equivalents;
3) combining the two solutions, and stirring to allow precipitation, or dropwise adding a poor solvent and then stirring to allow precipitation;
4) subjecting the mixture to rapid centrifugation or to standing followed by blow-drying to obtain the crystal form,

wherein: the good solvent is selected from methanol, ethanol, acetone, ethyl acetate, 2-butanone, 3-pentanone or 1,4-dioxane, preferably acetone, 2-butanone, 3-pentanone or ethyl acetate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, ethanol, acetone or ethyl acetate; the good solvent and the organic solution are mutually miscible when used;
the poor solvent is selected from heptane, water, methyl tert-butyl ether, isopropyl ether;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid or L-malic acid;
method II comprises the following steps:

1) weighing an appropriate amount of the compound and dissolving the compound in a solvent;
2) mixing with a counterion acid to obtain the crystal form, wherein the amount of the counterion acid is preferably 1.2 equivalents;

the solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, ethanol, acetone or ethyl acetate;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, pamoic acid,

propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, n-butyric acid, p-hydroxybenzoic acid or L-malic acid.

**14.** A method for preparing the crystal form or the acid addition salt according to any one of claims 1-5 or 9-11, **characterized in that** the method involves method I or method II:

method I comprises the following steps:

1) weighing an appropriate amount of the compound, and dissolving the compound in a good solvent with heating;
2) quickly placing the resulting solution at room temperature, and stirring same until a solid precipitates;
3) quickly centrifuging the resulting suspension, removing the supernatant, and drying the residual solid in a vacuum drying oven at 40°C to a constant weight to obtain a target crystal form,
wherein:
the good solvent is selected from methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butyl alcohol, 2-butanone, 3-pentanone, N-methylpyrrolidone, or dimethyl sulphoxide, preferably tetrahydrofuran, dichloromethane, 2-methyl-tetrahydrofuran and 3-pentanone;

method II comprises the following steps:

1) weighing an appropriate amount of a free base, and dissolving the free base in a good solvent;
2) adding an anti-solvent to the resulting solution at a given temperature, preferably at 0°C-25°C, and stirring until a solid precipitates;
3) quickly centrifuging the resulting suspension, removing the supernatant, and drying the residual solid in a vacuum drying oven at 40°C to a constant weight to obtain a target product,

wherein: the good solvent is selected from acetone, 88% acetone, tetrahydrofuran, 2-methyltetrahydrofuran, dichloromethane, 1,4-dioxane, toluene, isopropanol, n-butanol, isobutanol, N,N-dimethylformamide, N,N-dimethylacetamide, n-propanol, tert-butyl alcohol, 2-butanone, 3-pentanone, N-methylpyrrolidone, ethyl formate, or dimethyl sulphoxide, preferably acetone, dichloromethane, tetrahydrofuran, 2-methyltetrahydrofuran, ethyl formate, butanone, 3-pentanone, or 1,4-dioxane;
the poor solvent is selected from heptane, water, methyl tert-butyl ether, or isopropyl ether.

**15.** A pharmaceutical composition comprising a therapeutically effective dose of the crystal form or the acid addition salt according to any one of claims 1-5 or 9-11, the crystal form according to any one of claims 6-8, and one or more pharmaceutically acceptable carriers, diluents or excipients.

**16.** Use of the crystal form or the acid addition salt according to any one of claims 1-5 or 9-11, the crystal form according to any one of claims 6-8, or the pharmaceutical composition according to claim 15 in the preparation of an EGFR inhibitor.

**17.** The use according to claim 16; the EGFR is a mutated EGFR, preferably comprising one or more mutations of Del19, L858R, T790M or C797S, and more preferably comprising mutations L858R/T790M, Del19/T790M, Del19/C797S, L858R/C797S, Del19/T790M/C797S or L858R/T790M/C797S.

**18.** Use of the crystal form or the acid addition salt according to any one of claims 1-5 or 9-11, the crystal form according to any one of claims 6-8, or the pharmaceutical composition according to claim 15 in the preparation of a medicament for treating cancer; preferably, the cancer is selected from ovarian cancer, cervical cancer, colorectal cancer, breast cancer, pancreatic cancer, glioma, glioblastoma, melanoma, prostate cancer, leukaemia, lymphoma, non-Hodgkin's lymphoma, gastric cancer, lung cancer, hepatocellular carcinoma, gastric cancer, gastrointestinal stromal tumour, thyroid cancer, bile duct cancer, endometrial cancer, renal cancer, anaplastic large cell lymphoma, multiple myeloma, melanoma or mesothelioma; more preferably, the cancer is non-small cell lung cancer; further preferably, the cancer is a non-small cell lung cancer with EGFR mutations of L858R/T790M, Del19/T790M, Del19/C797S, L858R/C797S, Del19/T790M/C797S or L858R/T790M/C797S.

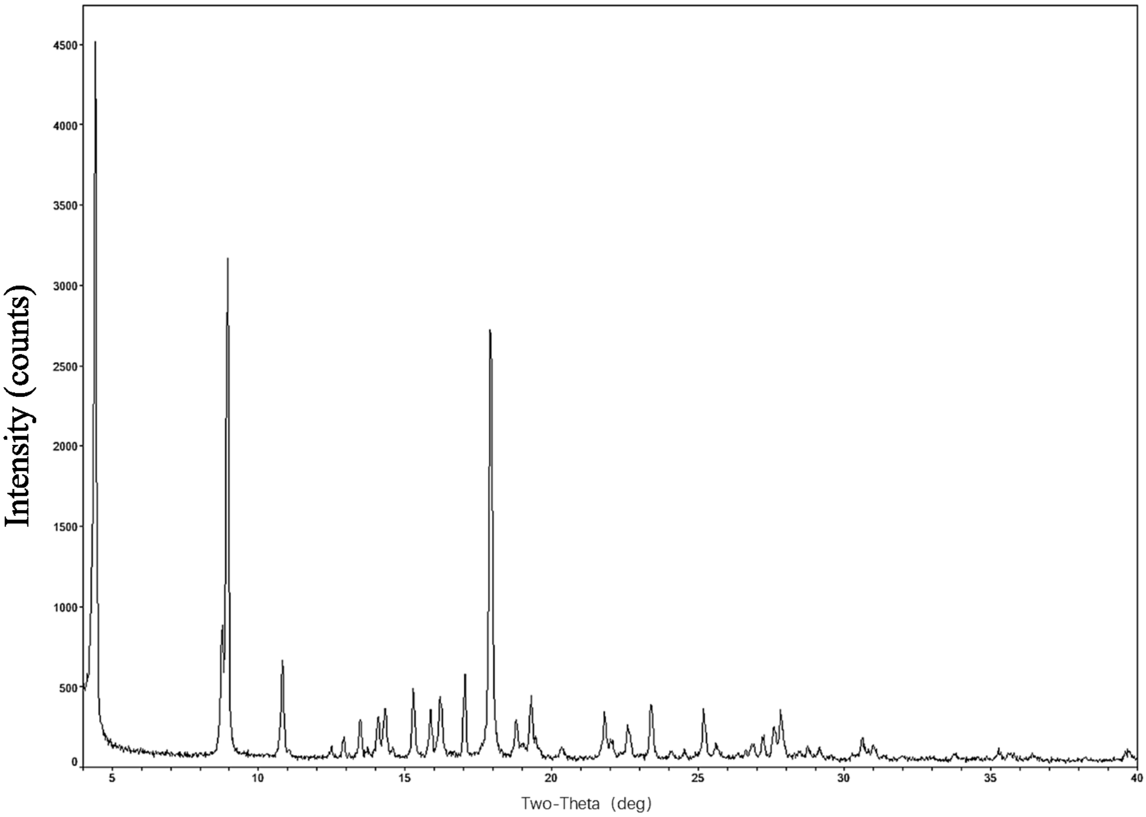
Intensity (counts)
Two-Theta (deg)

*FIG. 1*

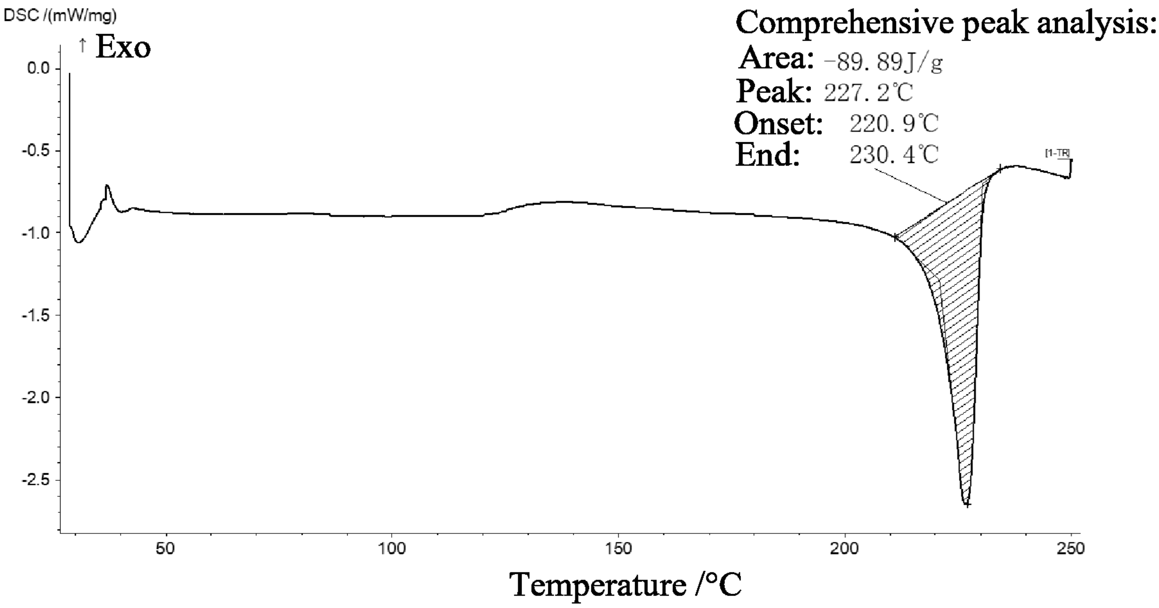
DSC /(mW/mg)
↑ Exo
Comprehensive peak analysis:
Area: -89. 89J/g
Peak: 227. 2℃
Onset: 220. 9℃
End: 230. 4℃
Temperature /°C

*FIG. 2*

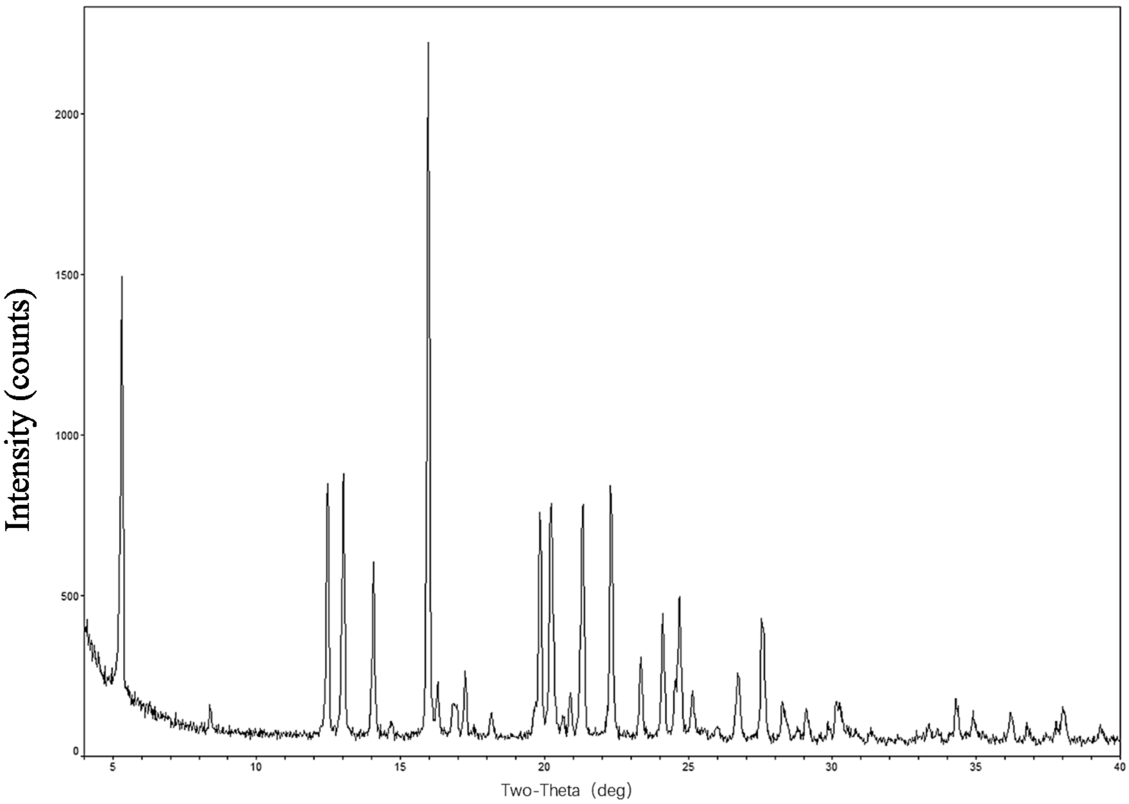
Intensity (counts)
2000
1500
1000
500
0
5
10
15
20
25
30
35
40
Two-Theta (deg)

*FIG. 3*

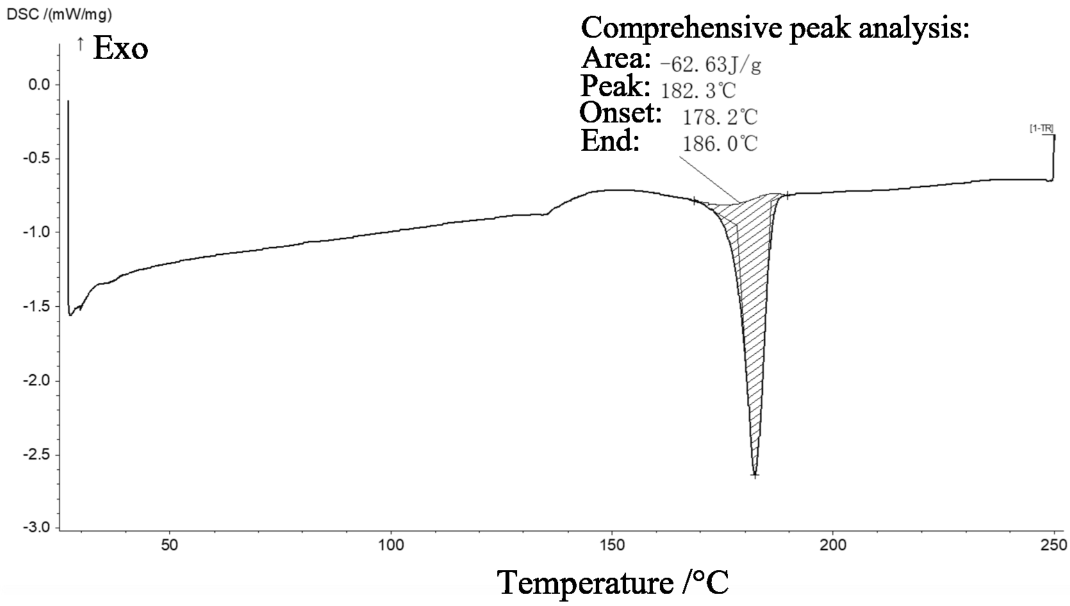
DSC /(mW/mg)
↑ Exo
Comprehensive peak analysis:
Area: -62. 63J/g
Peak: 182. 3℃
Onset: 178. 2℃
End: 186. 0℃
[1-TR]
0.0
-0.5
-1.0
-1.5
-2.0
-2.5
-3.0
50
100
150
200
250
Temperature /°C

*FIG. 4*

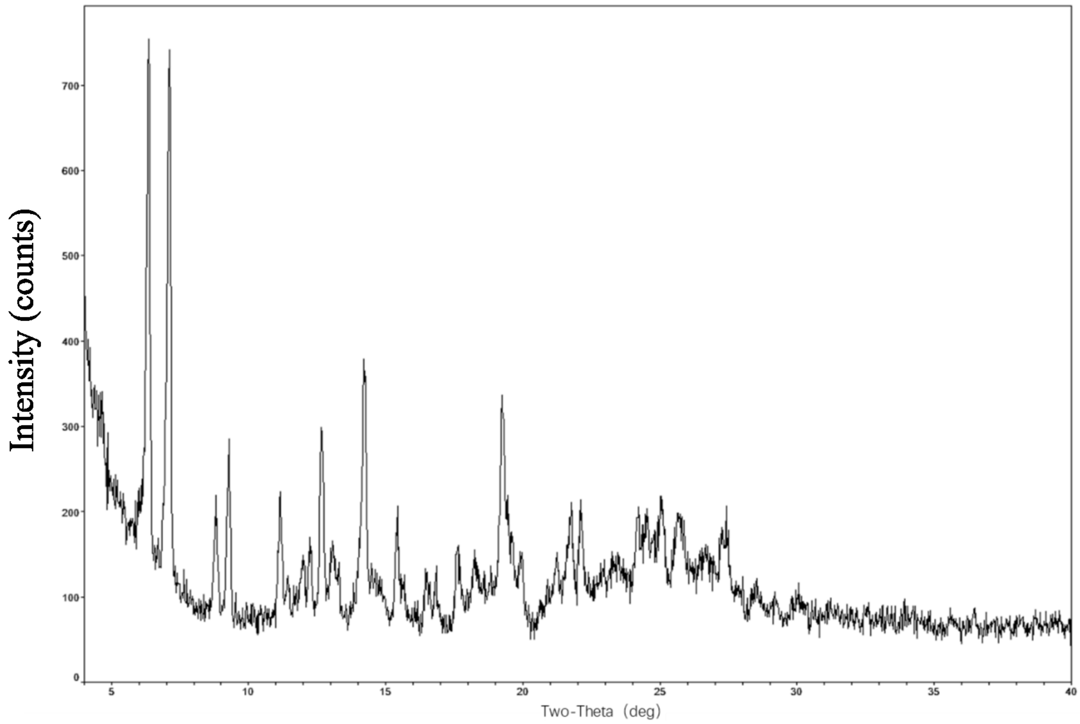
Intensity (counts)
700
600
500
400
300
200
100
0
5
10
15
20
25
30
35
40
Two-Theta (deg)

*FIG. 5*

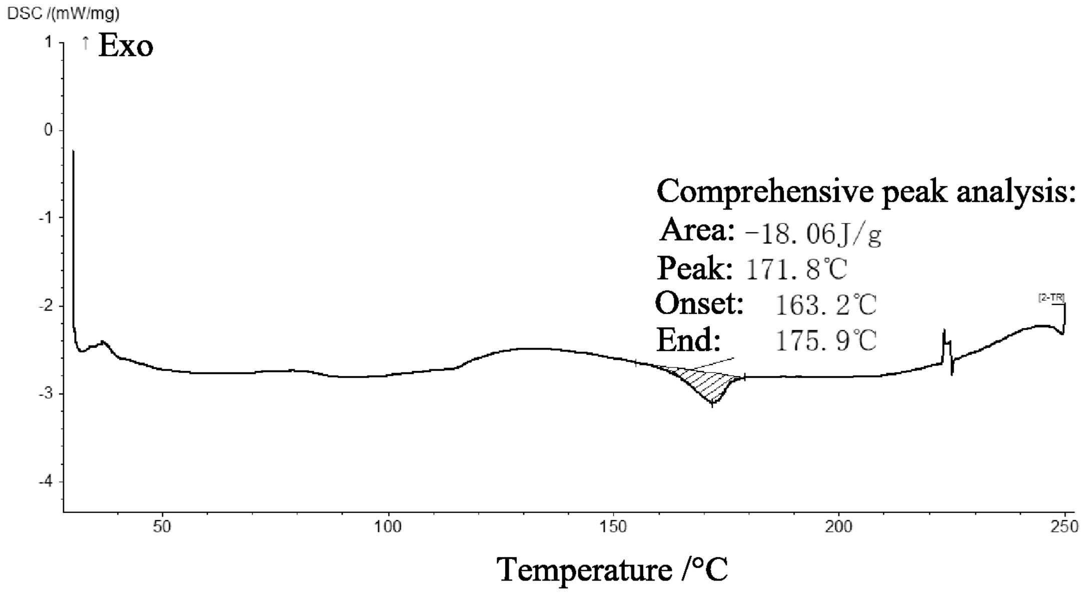
DSC /(mW/mg)
↑ Exo
1
0
-1
-2
-3
-4
Comprehensive peak analysis:
Area: -18. 06J/g
Peak: 171. 8℃
Onset: 163. 2℃
End: 175. 9℃
[2-TR]
50
100
150
200
250
Temperature /°C

*FIG. 6*

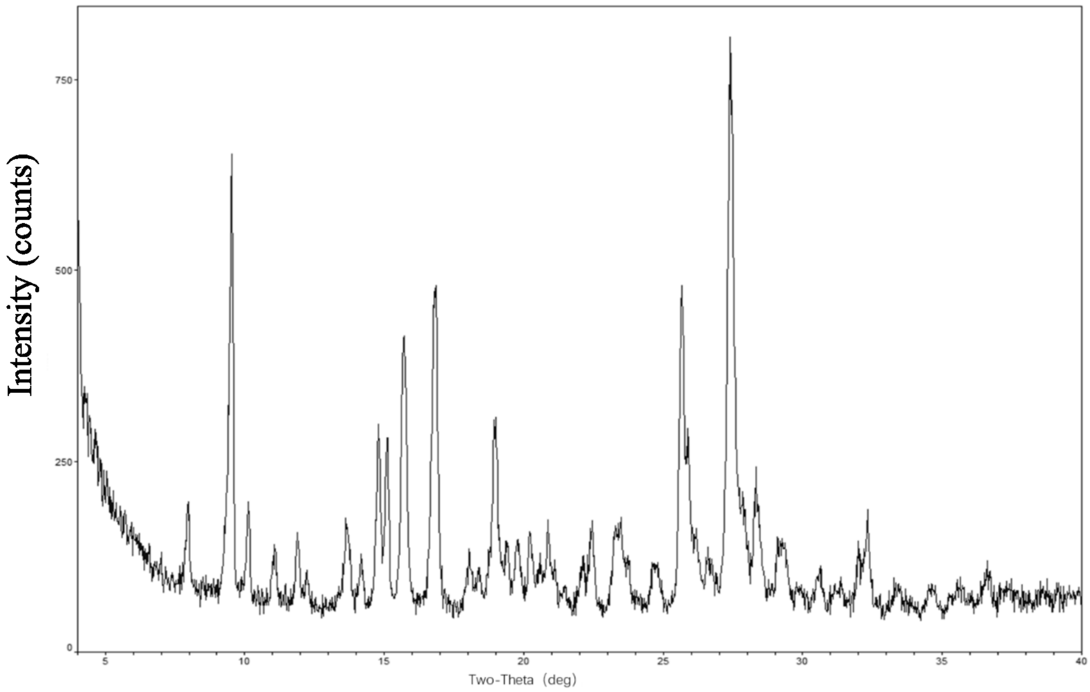
Intensity (counts)
750
500
250
0
5
10
15
20
25
30
35
40
Two-Theta (deg)

*FIG. 7*

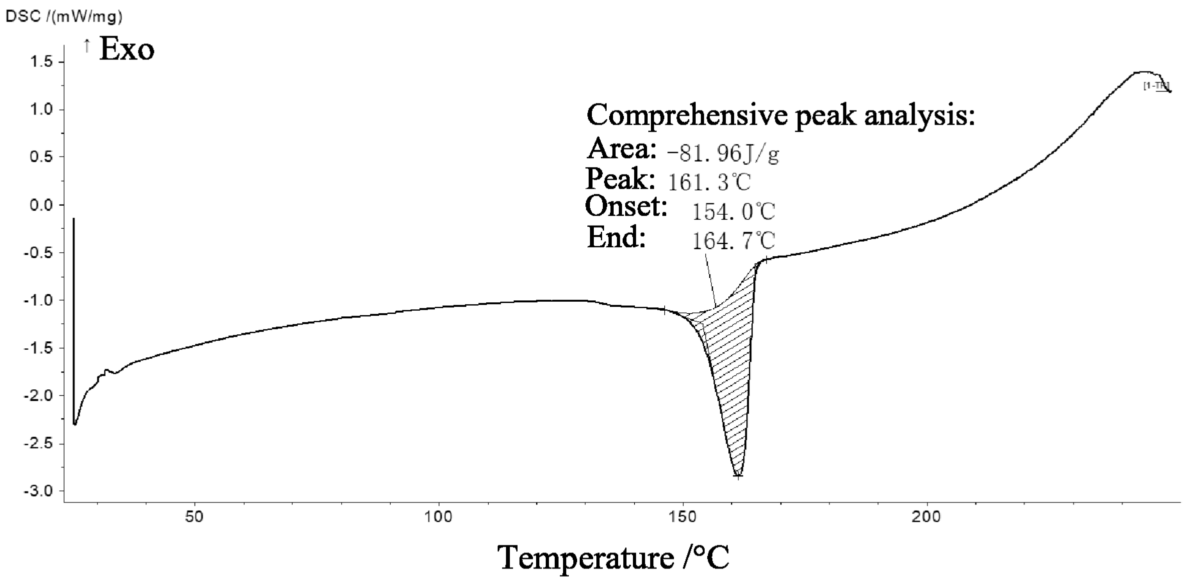
DSC /(mW/mg)
↑ Exo
1.5
1.0
0.5
0.0
-0.5
-1.0
-1.5
-2.0
-2.5
-3.0
Comprehensive peak analysis:
Area: -81. 96J/g
Peak: 161. 3℃
Onset: 154. 0℃
End: 164. 7℃
50
100
150
200
Temperature /°C

*FIG. 8*

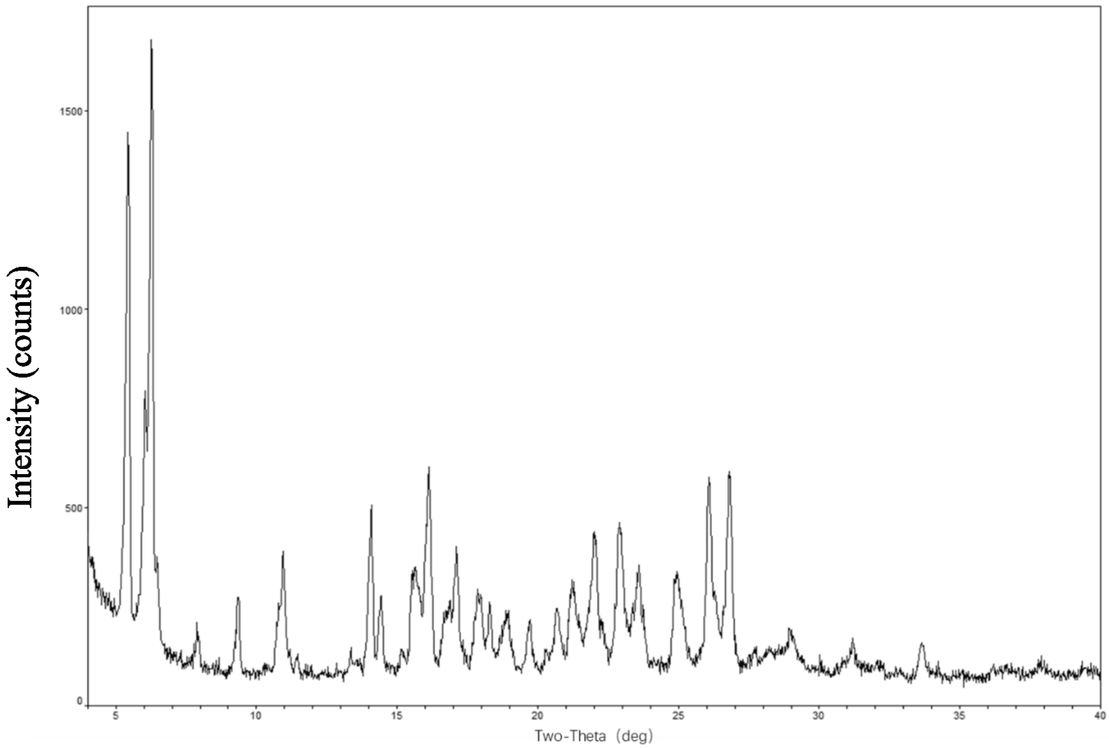
Intensity (counts)
1500
1000
500
0
5
10
15
20
25
30
35
40
Two-Theta (deg)

*FIG. 9*

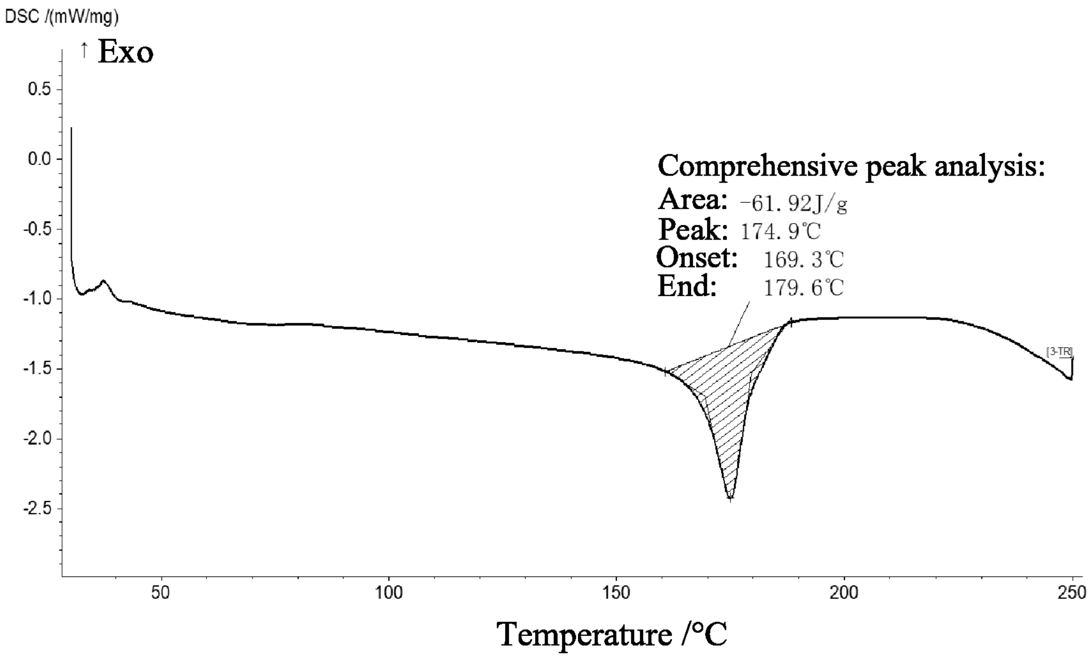
DSC /(mW/mg)
↑ Exo
0.5
0.0
-0.5
-1.0
-1.5
-2.0
-2.5
Comprehensive peak analysis:
Area: -61. 92J/g
Peak: 174. 9℃
Onset: 169. 3℃
End: 179. 6℃
50
100
150
200
250
Temperature /°C

*FIG. 10*

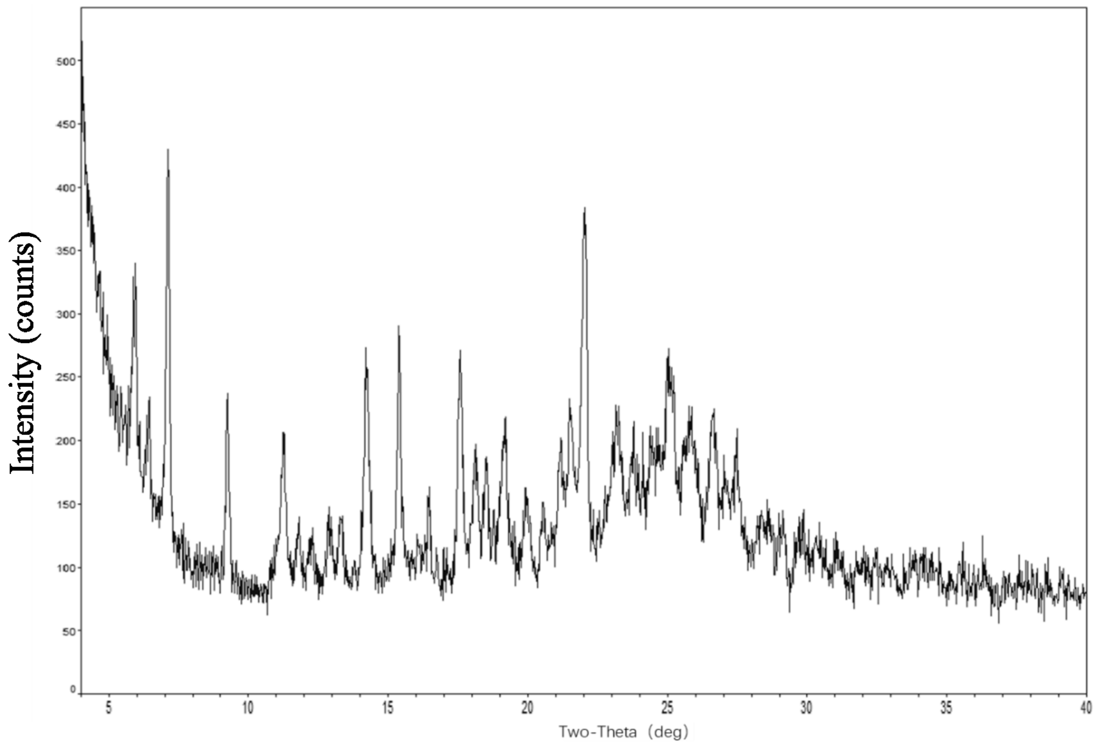
Intensity (counts)
500
450
400
350
300
250
200
150
100
50
0
5
10
15
20
25
30
35
40
Two-Theta (deg)

*FIG. 11*

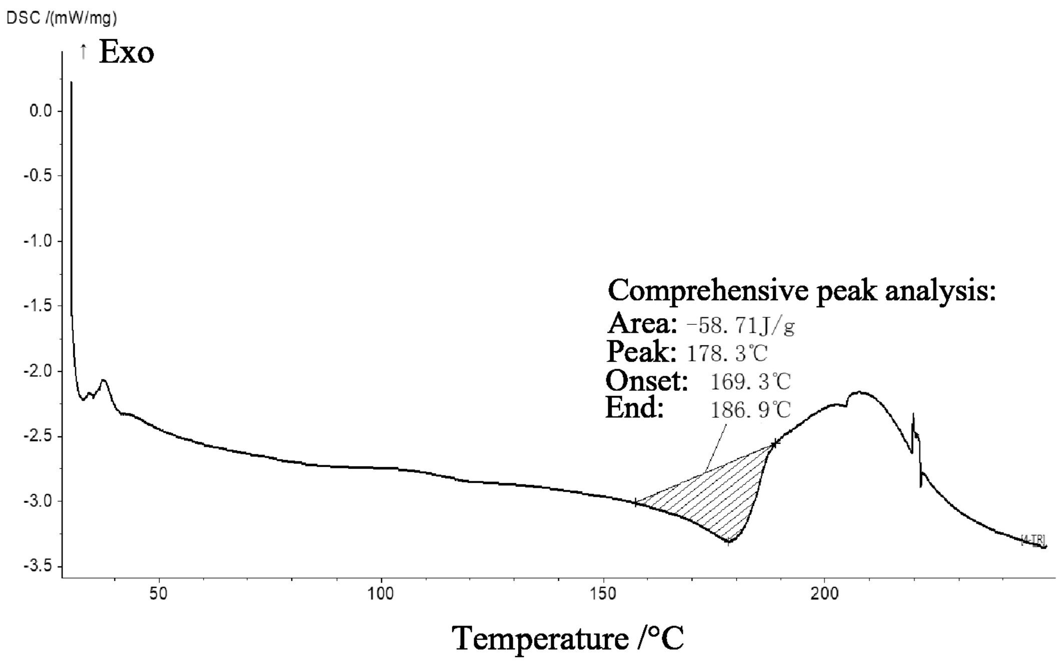
DSC /(mW/mg)
↑ Exo
0.0
-0.5
-1.0
-1.5
-2.0
-2.5
-3.0
-3.5
Comprehensive peak analysis:
Area: -58. 71J/g
Peak: 178. 3℃
Onset: 169. 3℃
End: 186. 9℃
50
100
150
200
Temperature /°C

*FIG. 12*

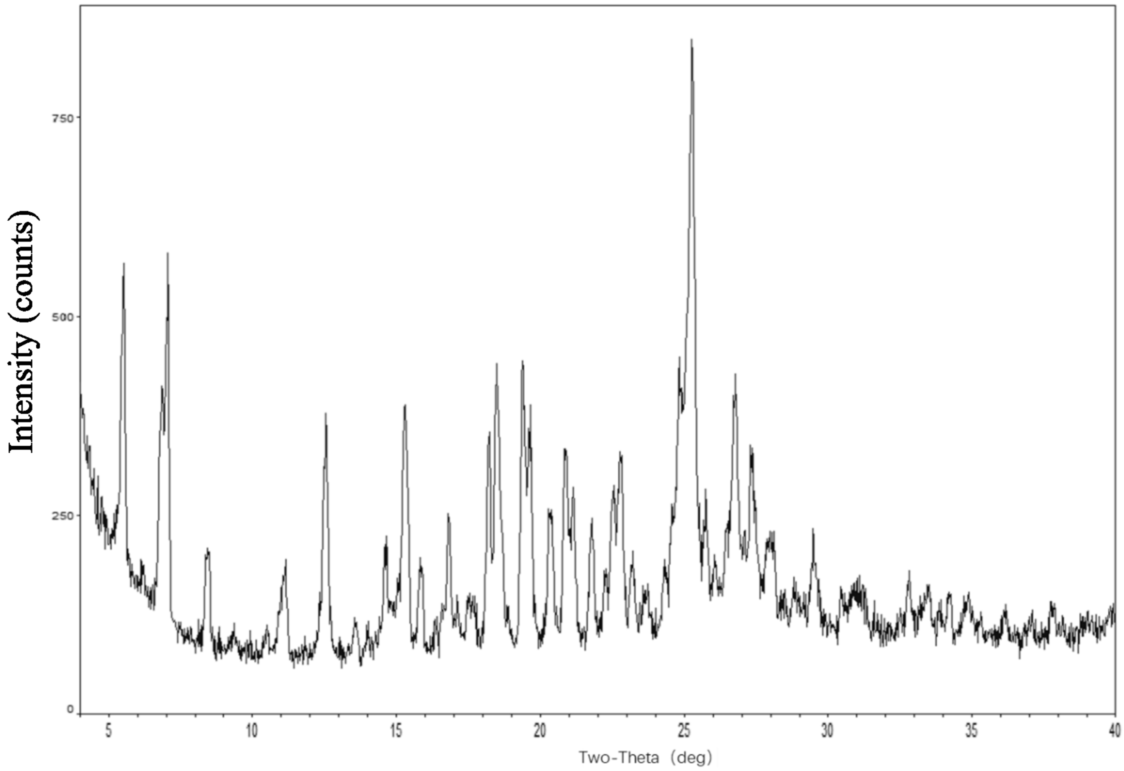
Intensity (counts)
750
500
250
0
5
10
15
20
25
30
35
40
Two-Theta (deg)

*FIG. 13*

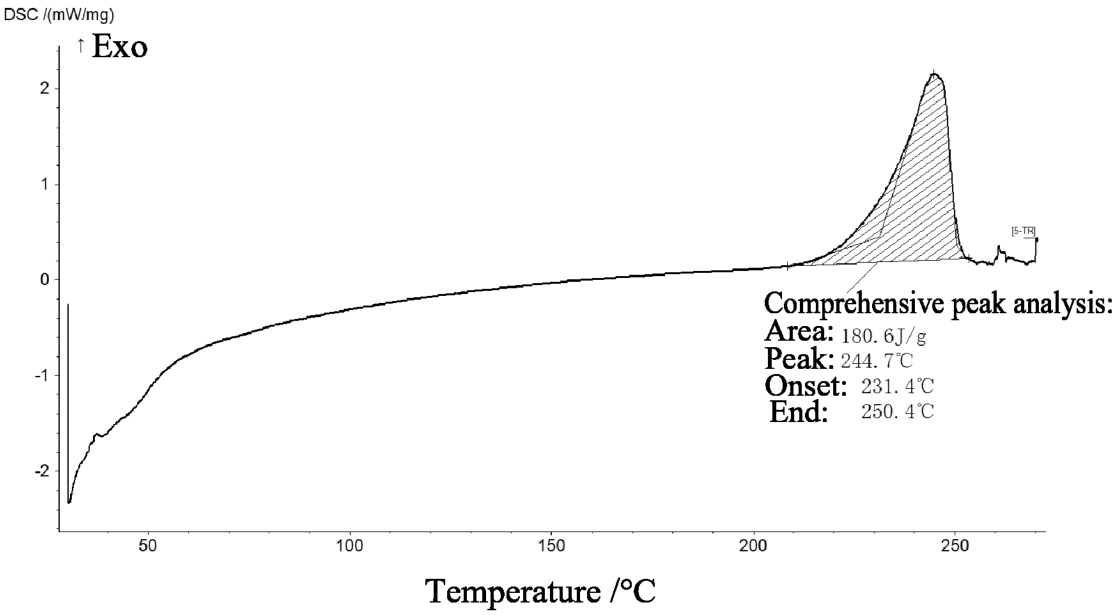
DSC /(mW/mg)
↑ Exo
2
1
0
-1
-2
50
100
150
200
250
Comprehensive peak analysis:
Area: 180. 6J/g
Peak: 244. 7℃
Onset: 231. 4℃
End: 250. 4℃
Temperature /°C

*FIG. 14*

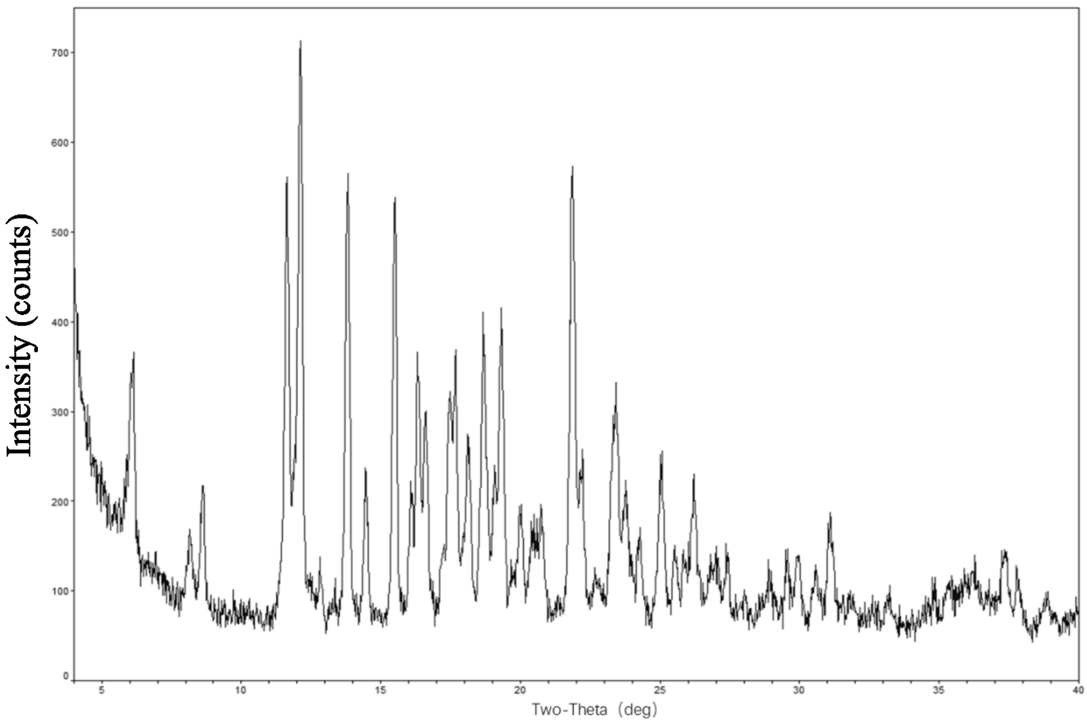
Intensity (counts)
Two-Theta (deg)
700
600
500
400
300
200
100
0
5
10
15
20
25
30
35
40

*FIG. 15*

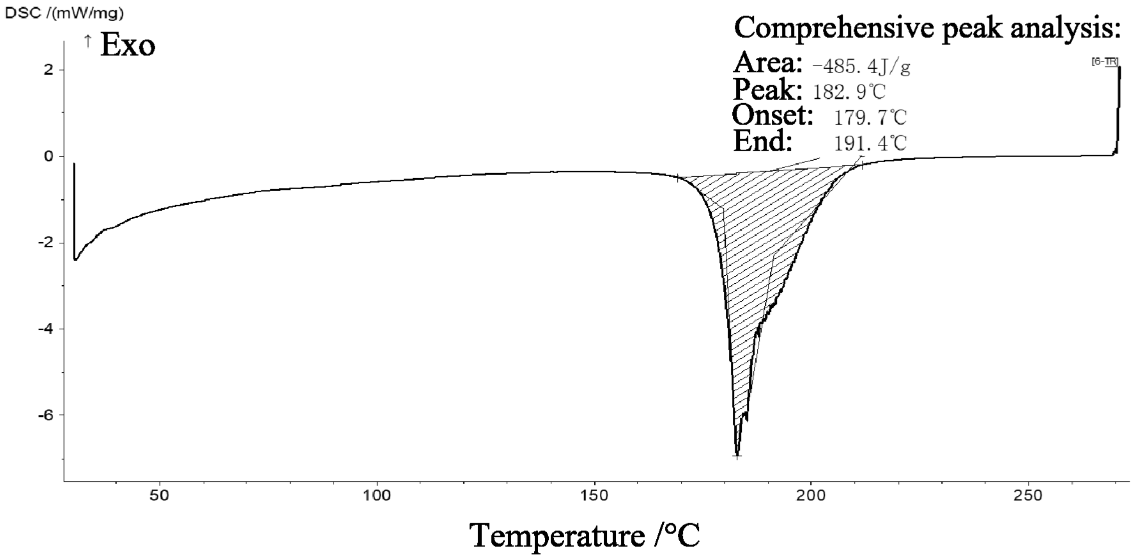
DSC /(mW/mg)
↑ Exo
Comprehensive peak analysis:
Area: -485. 4J/g
Peak: 182. 9℃
Onset: 179. 7℃
End: 191. 4℃
2
0
-2
-4
-6
50
100
150
200
250
Temperature /°C

*FIG. 16*

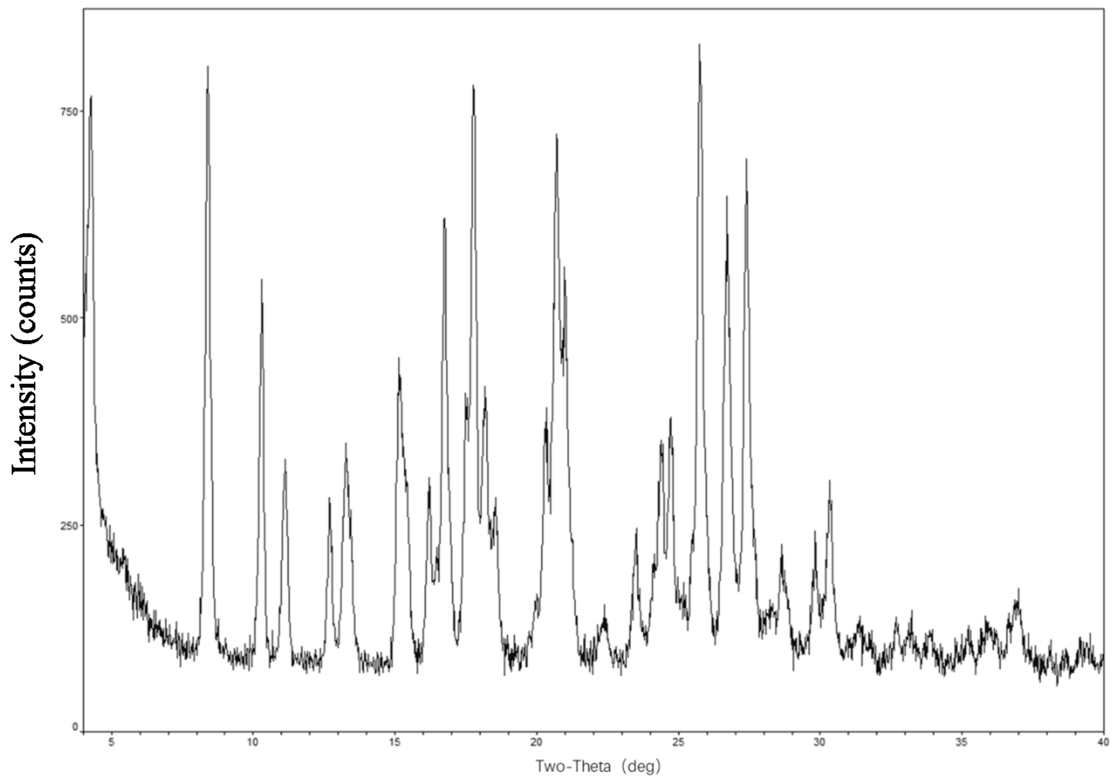
Intensity (counts)
750
500
250
0
5
10
15
20
25
30
35
40
Two-Theta (deg)

*FIG. 17*

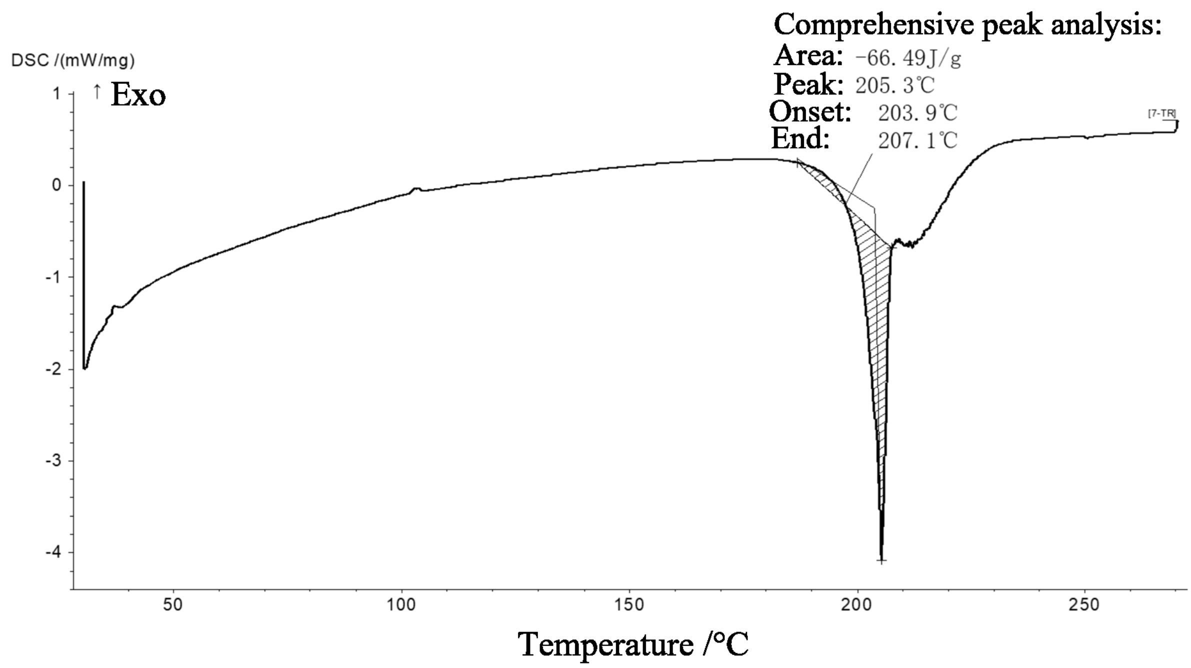
Comprehensive peak analysis:
Area: -66. 49J/g
Peak: 205. 3℃
Onset: 203. 9℃
End: 207. 1℃
DSC /(mW/mg)
↑ Exo
1
0
-1
-2
-3
-4
50
100
150
200
250
Temperature /°C

*FIG. 18*

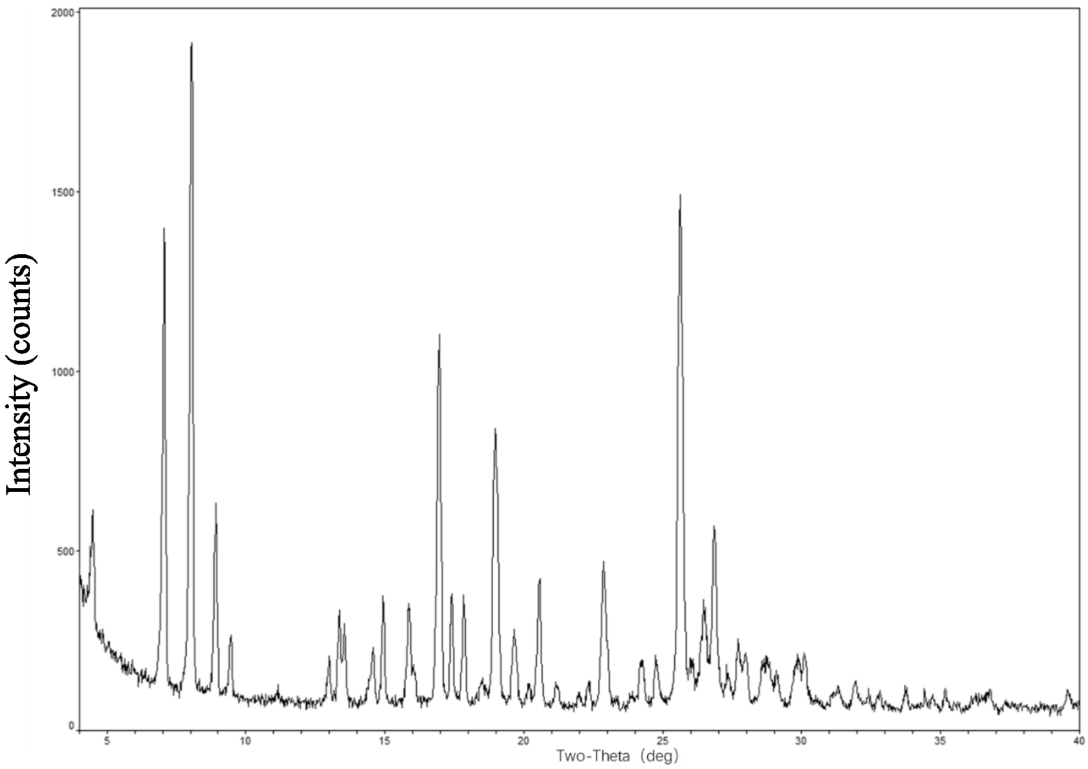
Intensity (counts)
Two-Theta (deg)

*FIG. 19*

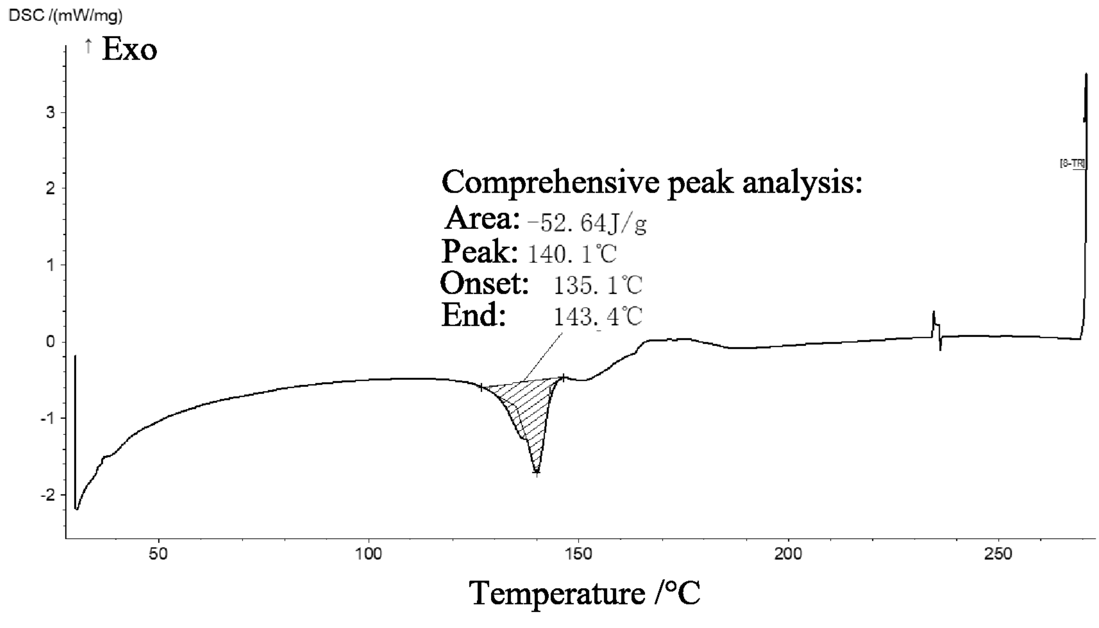
DSC /(mW/mg)
↑ Exo
Comprehensive peak analysis:
Area: -52. 64J/g
Peak: 140. 1℃
Onset: 135. 1℃
End: 143. 4℃
Temperature /°C

*FIG. 20*

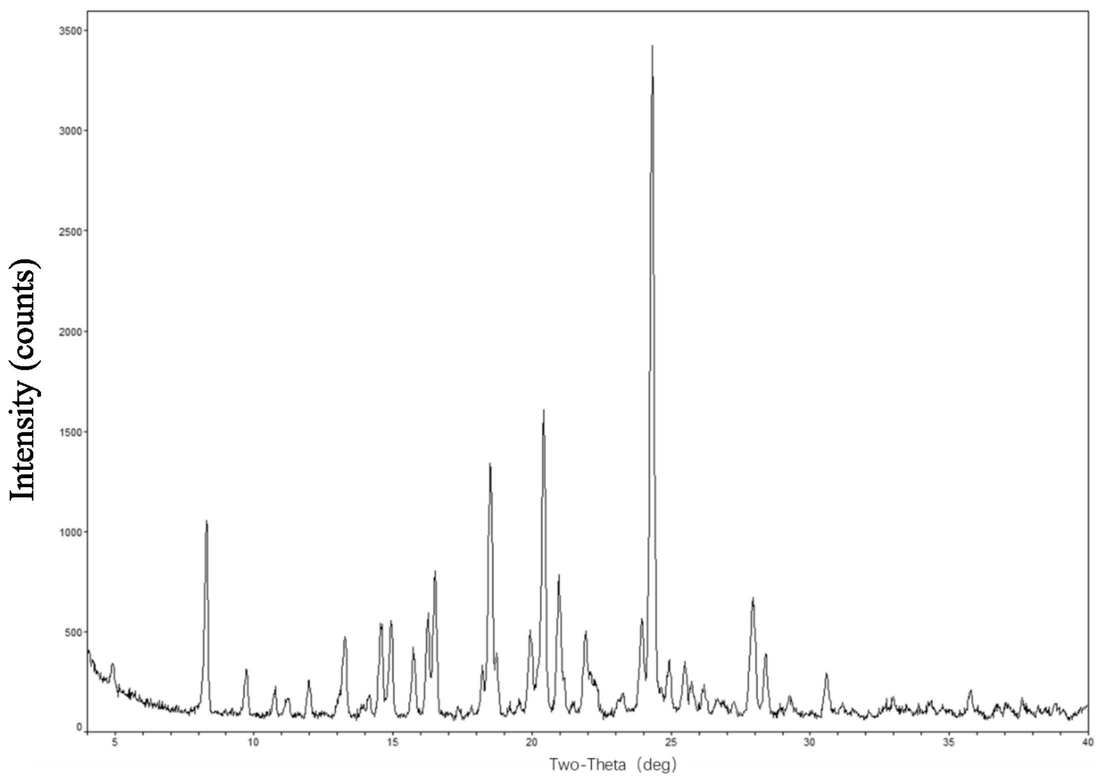
Intensity (counts)
Two-Theta (deg)

*FIG. 21*

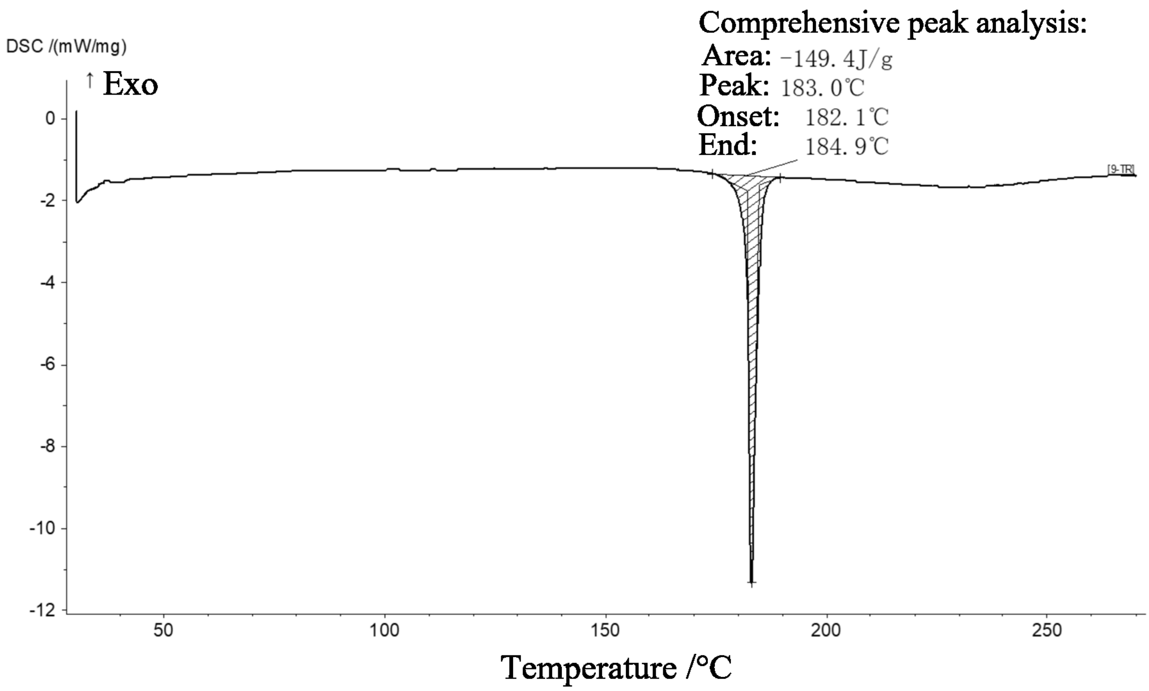
DSC /(mW/mg)
↑ Exo
Comprehensive peak analysis:
Area: -149. 4J/g
Peak: 183. 0℃
Onset: 182. 1℃
End: 184. 9℃
Temperature /°C

*FIG. 22*

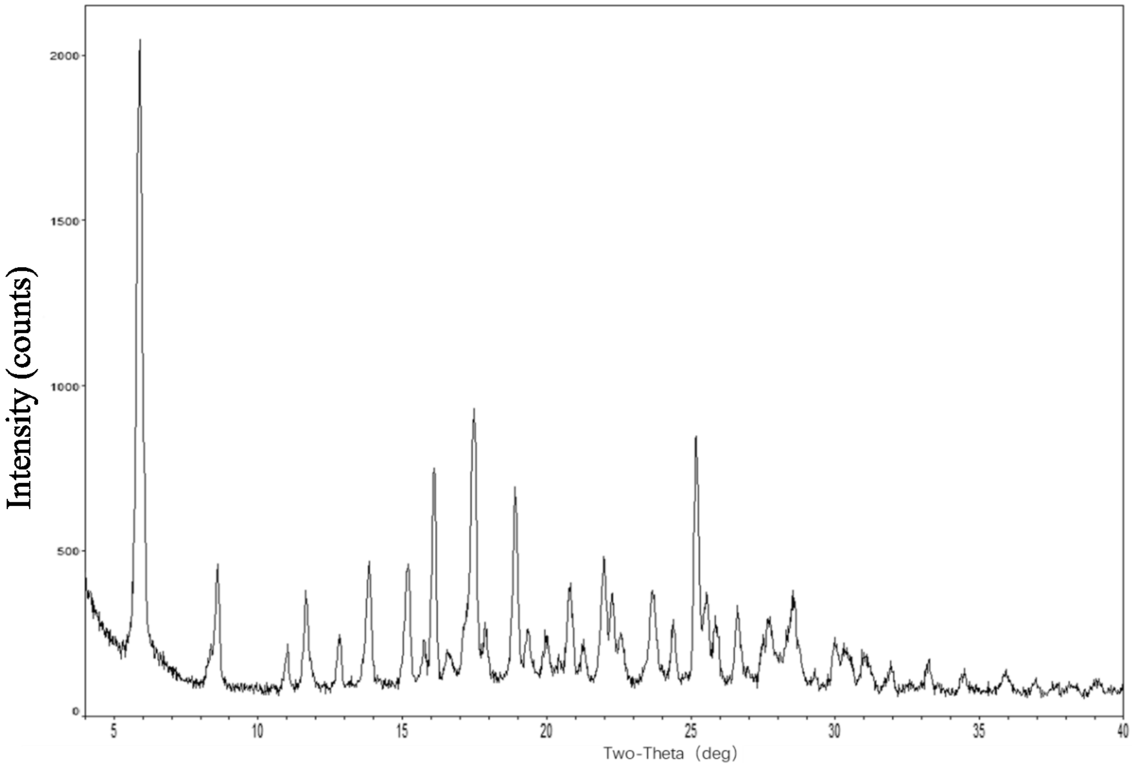
Intensity (counts)
2000
1500
1000
500
0
5
10
15
20
25
30
35
40
Two-Theta (deg)

*FIG. 23*

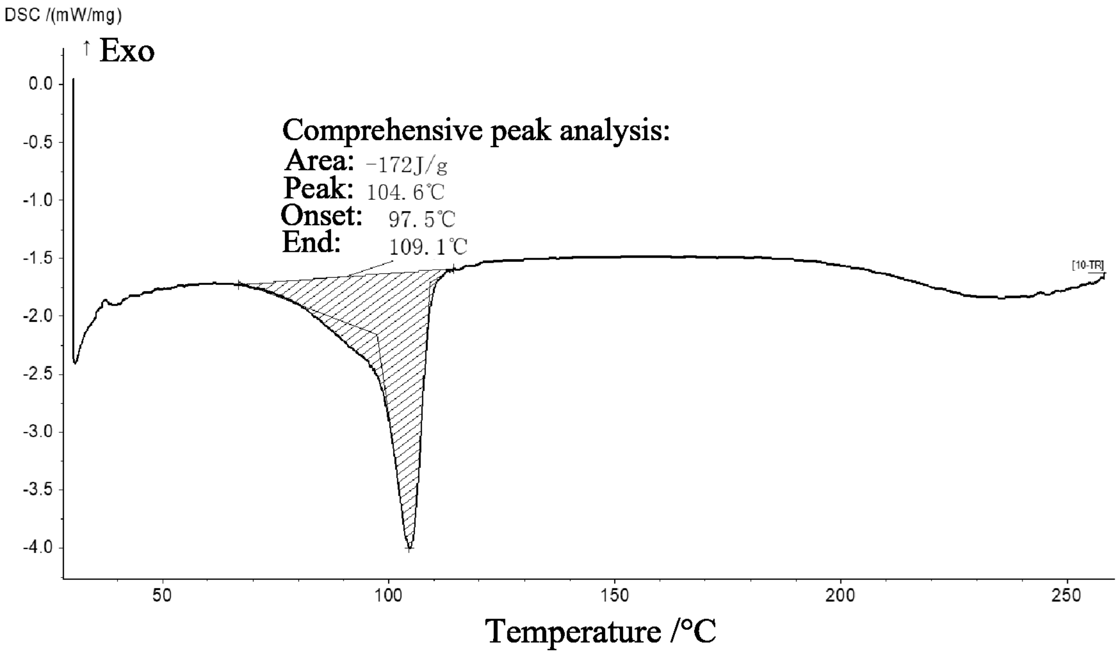
DSC /(mW/mg)
↑ Exo
Comprehensive peak analysis:
Area: -172J/g
Peak: 104. 6℃
Onset: 97. 5℃
End: 109. 1℃
[10-TR]
0.0
-0.5
-1.0
-1.5
-2.0
-2.5
-3.0
-3.5
-4.0
50
100
150
200
250
Temperature /°C

*FIG. 24*

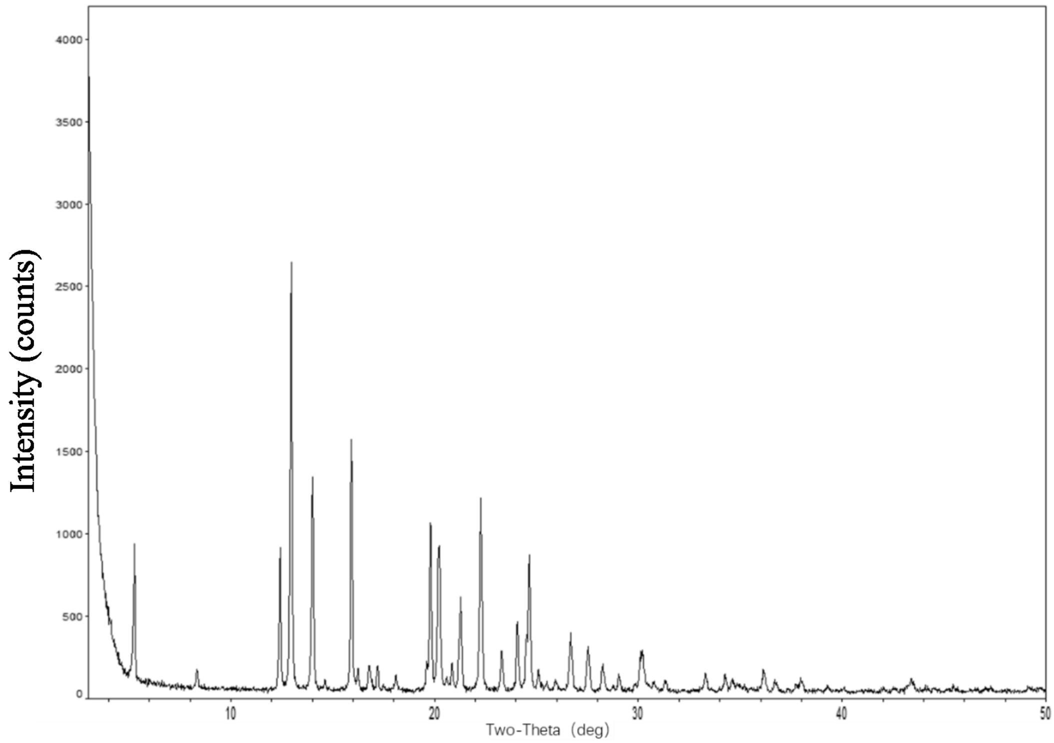
Intensity (counts)
Two-Theta (deg)
4000
3500
3000
2500
2000
1500
1000
500
0
10
20
30
40
50

*FIG. 25*

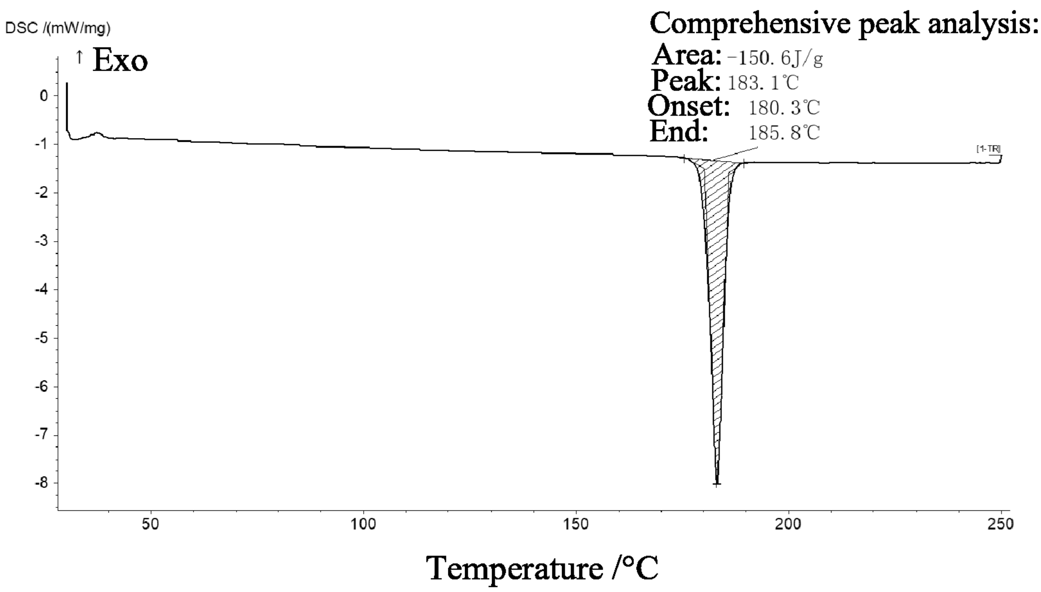
DSC /(mW/mg)
↑ Exo
Comprehensive peak analysis:
Area: -150. 6J/g
Peak: 183. 1℃
Onset: 180. 3℃
End: 185. 8℃
[1-TR]
0
-1
-2
-3
-4
-5
-6
-7
-8
50
100
150
200
250
Temperature /°C

*FIG. 26*

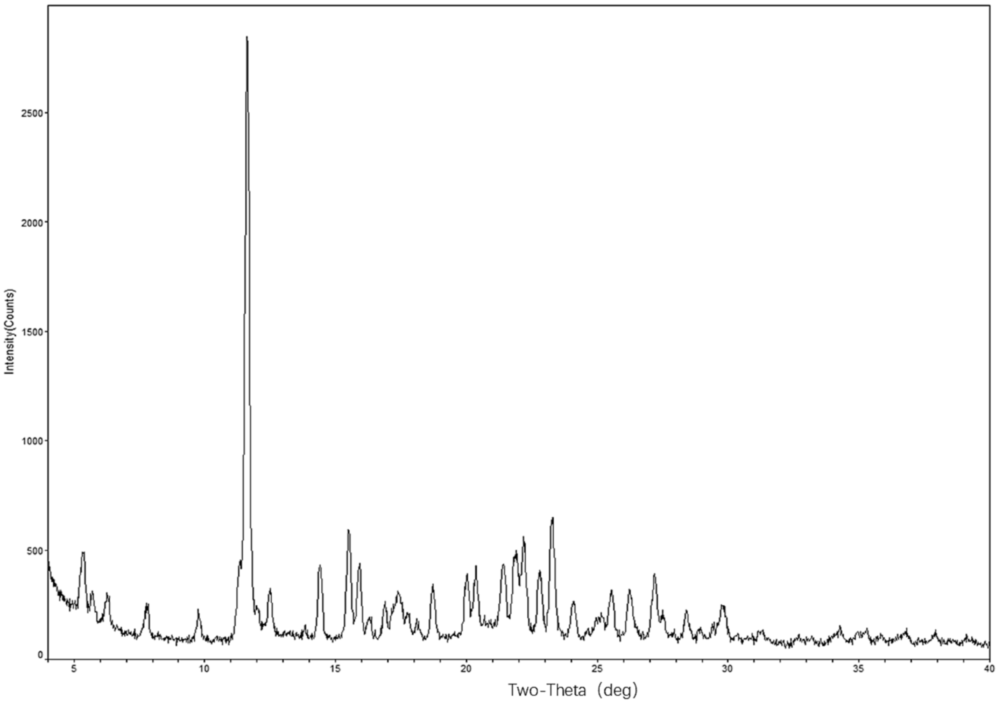
Intensity(Counts)
2500
2000
1500
1000
500
0
5
10
15
20
25
30
35
40
Two-Theta (deg)

*FIG. 27*

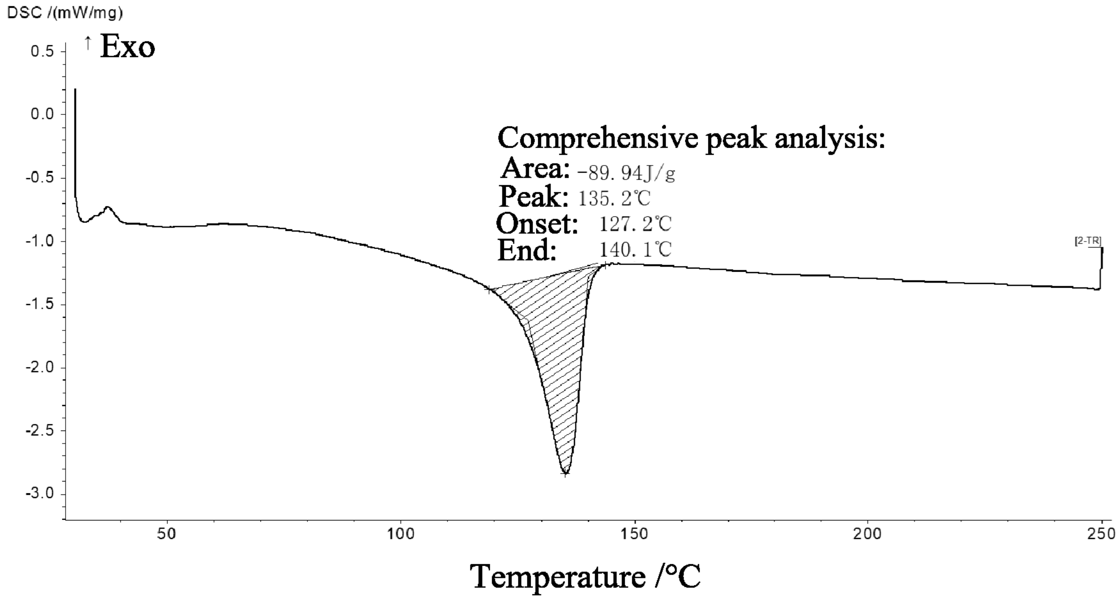
DSC /(mW/mg)
↑ Exo
0.5
0.0
-0.5
-1.0
-1.5
-2.0
-2.5
-3.0
Comprehensive peak analysis:
Area: -89. 94J/g
Peak: 135. 2℃
Onset: 127. 2℃
End: 140. 1℃
[2-TR]
50
100
150
200
250
Temperature /°C

*FIG. 28*

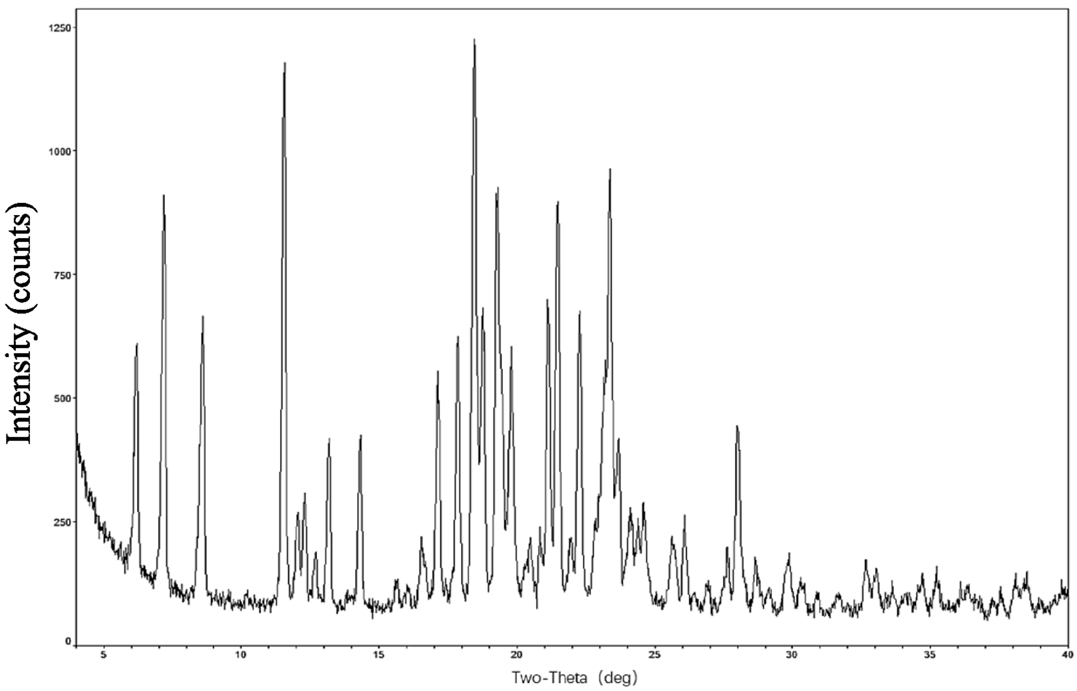
Intensity (counts)
1250
1000
750
500
250
0
5
10
15
20
25
30
35
40
Two-Theta (deg)

*FIG. 29*

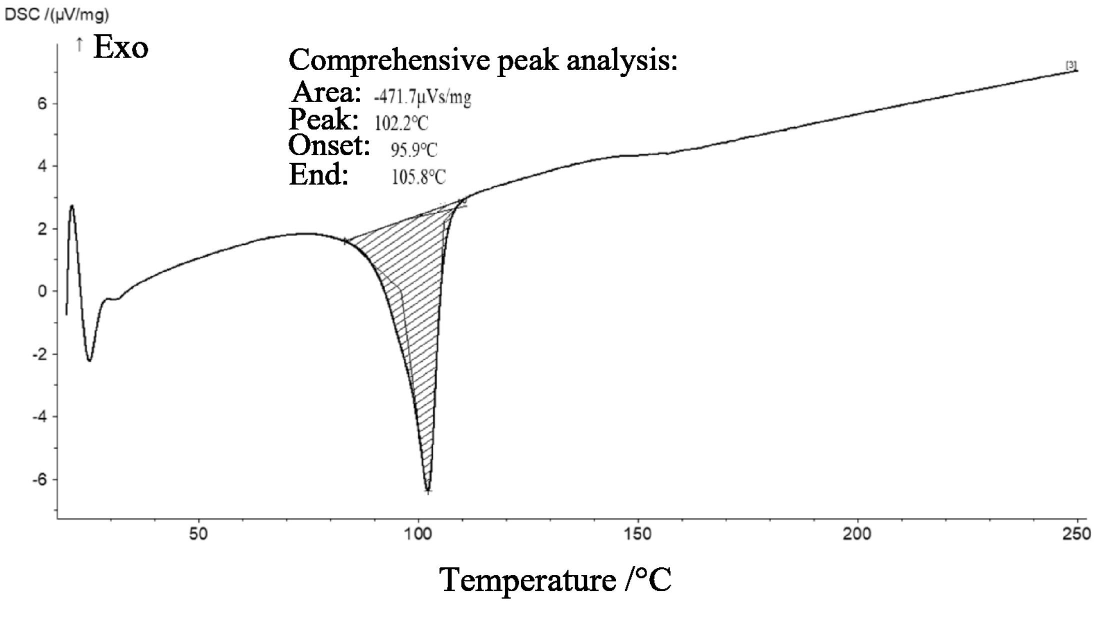
DSC /(μV/mg)
↑ Exo
Comprehensive peak analysis:
Area: -471.7μVs/mg
Peak: 102.2°C
Onset: 95.9°C
End: 105.8°C
[3]
6
4
2
0
-2
-4
-6
50
100
150
200
250
Temperature /°C

*FIG. 30*

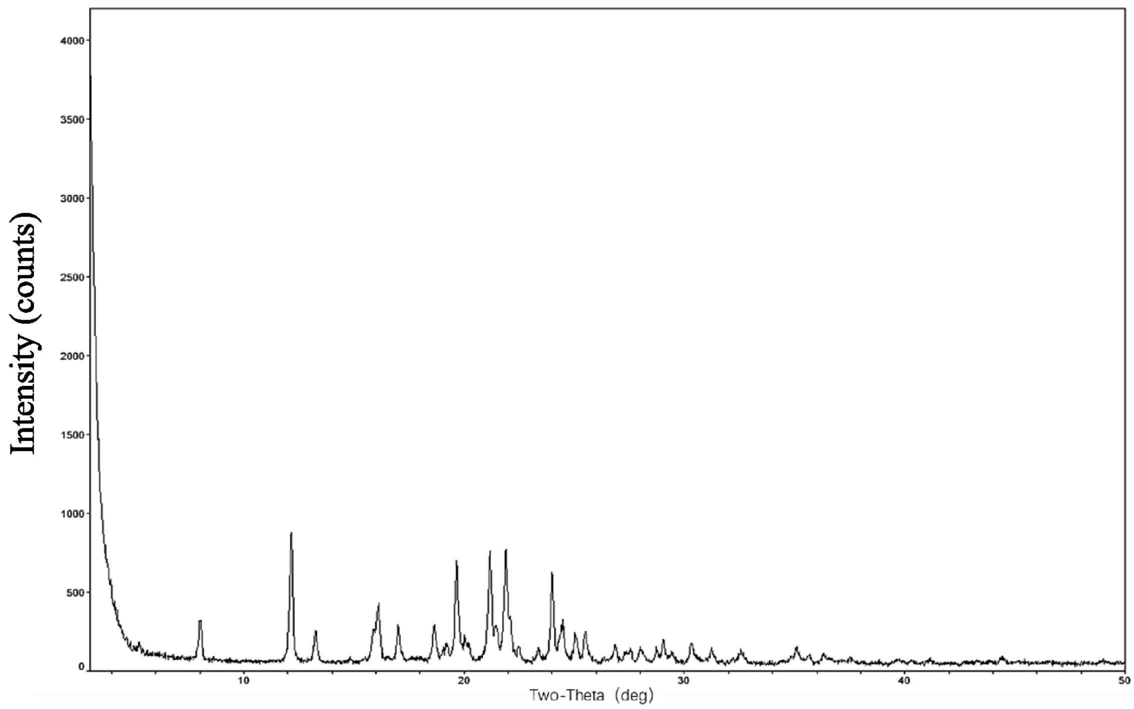
Intensity (counts)
4000
3500
3000
2500
2000
1500
1000
500
0
10
20
30
40
50
Two-Theta (deg)

*FIG. 31*

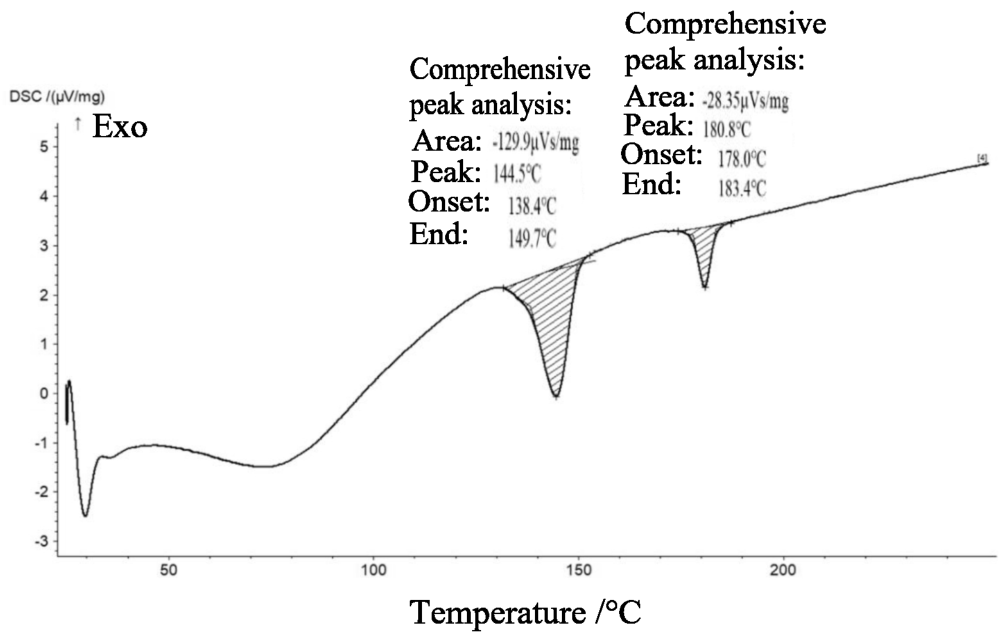
DSC /(μV/mg)
↑ Exo
Comprehensive peak analysis:
Area: -129.9μVs/mg
Peak: 144.5°C
Onset: 138.4°C
End: 149.7°C
Comprehensive peak analysis:
Area: -28.35μVs/mg
Peak: 180.8°C
Onset: 178.0°C
End: 183.4°C
5
4
3
2
1
0
-1
-2
-3
50
100
150
200
Temperature /°C

*FIG. 32*

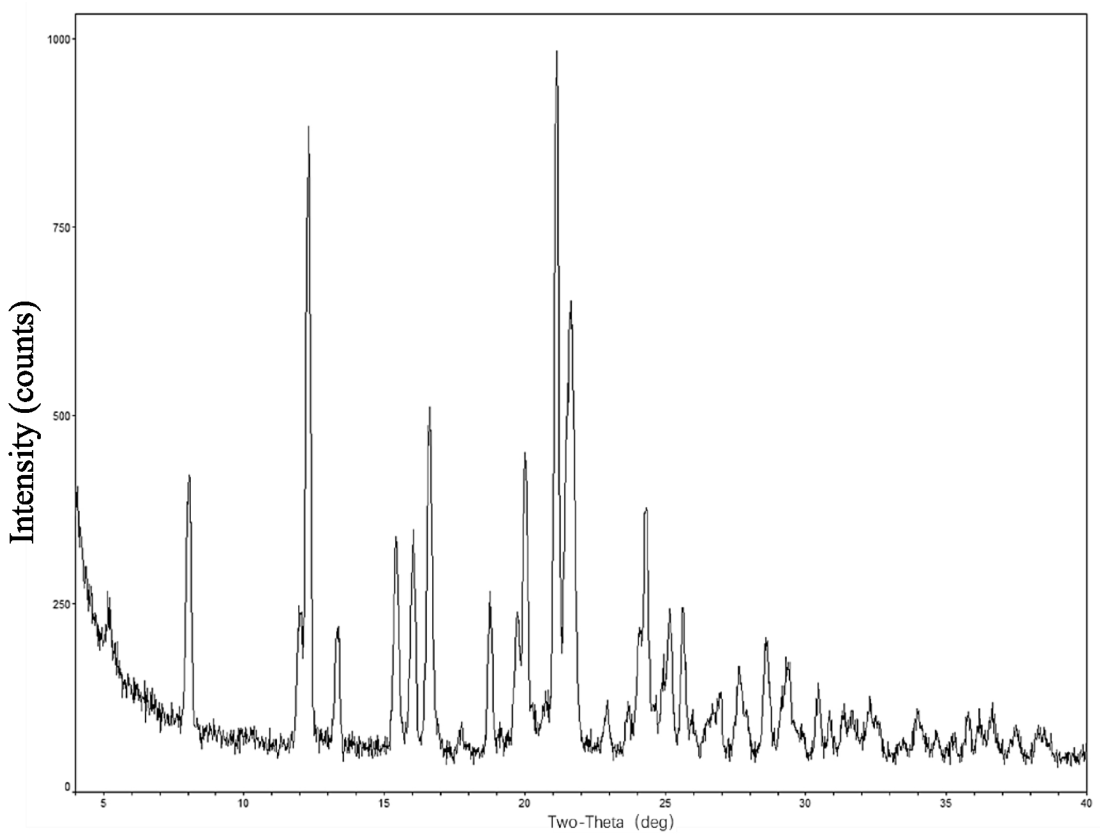

Intensity (counts)
1000
750
500
250
0
5
10
15
20
25
30
35
40
Two-Theta (deg)


*FIG. 33*

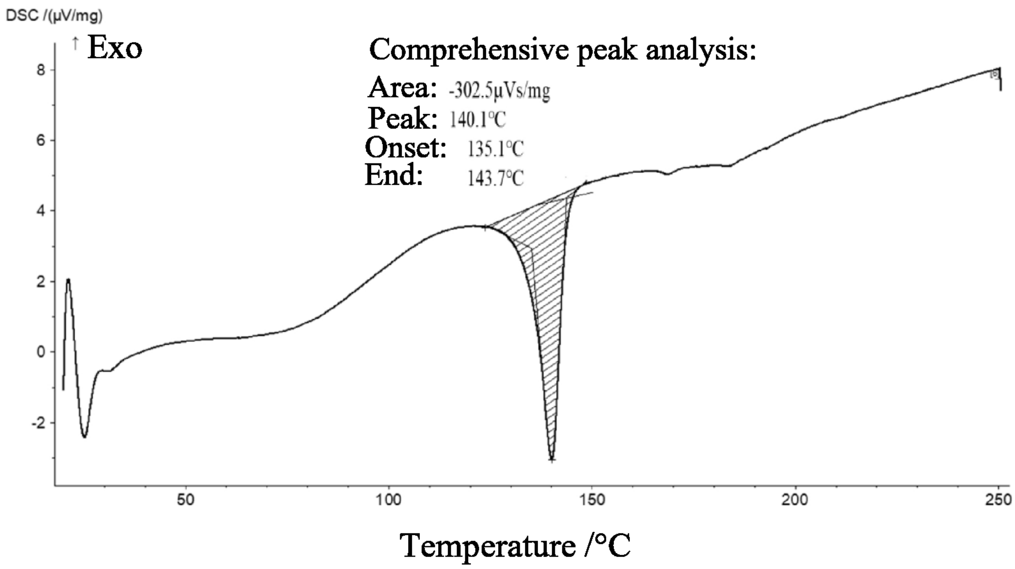

DSC /(μV/mg)
↑ Exo
Comprehensive peak analysis:
Area: -302.5μVs/mg
Peak: 140.1°C
Onset: 135.1°C
End: 143.7°C
8
6
4
2
0
-2
50
100
150
200
250
Temperature /°C


*FIG. 34*

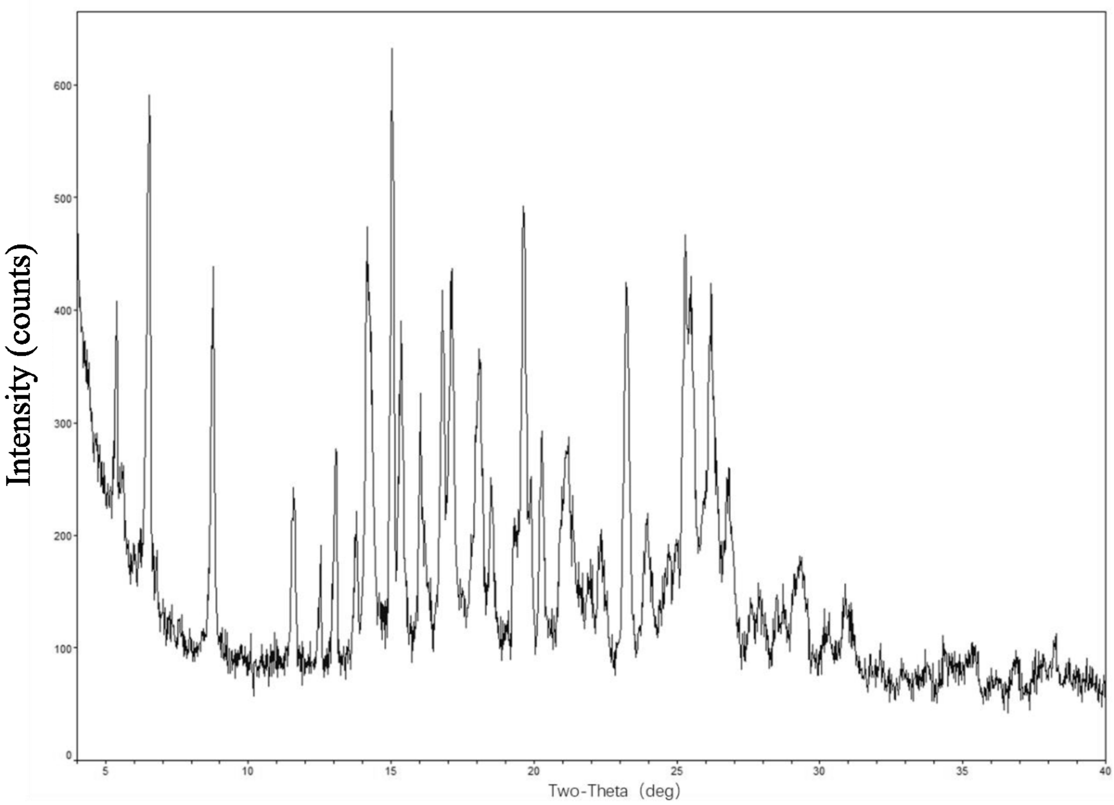
Intensity (counts)
0
100
200
300
400
500
600
5
10
15
20
25
30
35
40
Two-Theta (deg)

*FIG. 35*

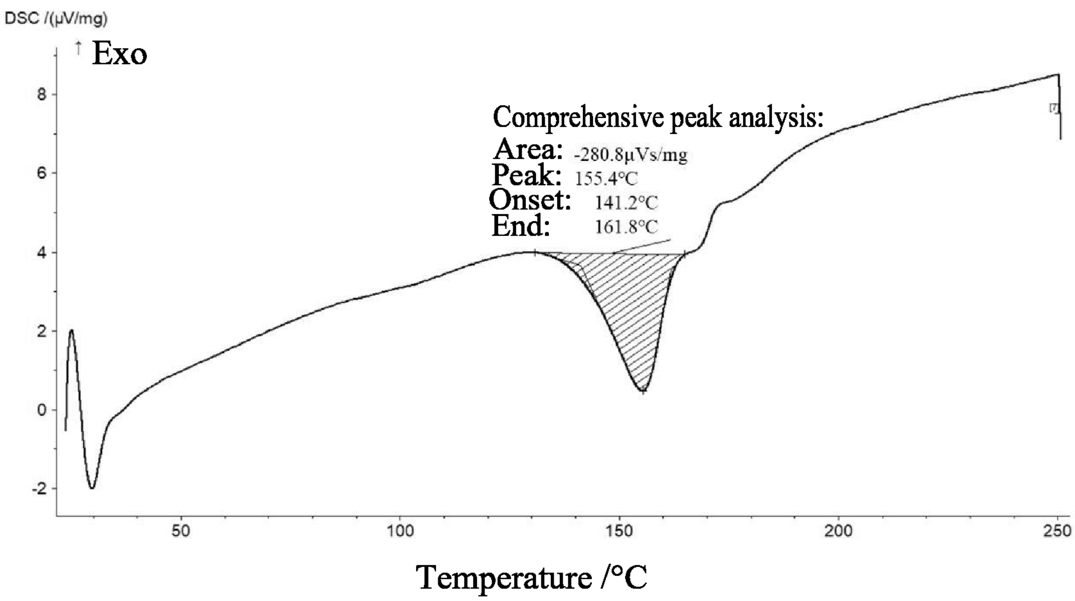
DSC /(μV/mg)
↑ Exo
Comprehensive peak analysis:
Area: -280.8μVs/mg
Peak: 155.4°C
Onset: 141.2°C
End: 161.8°C
8
6
4
2
0
-2
50
100
150
200
250
Temperature /°C

*FIG. 36*

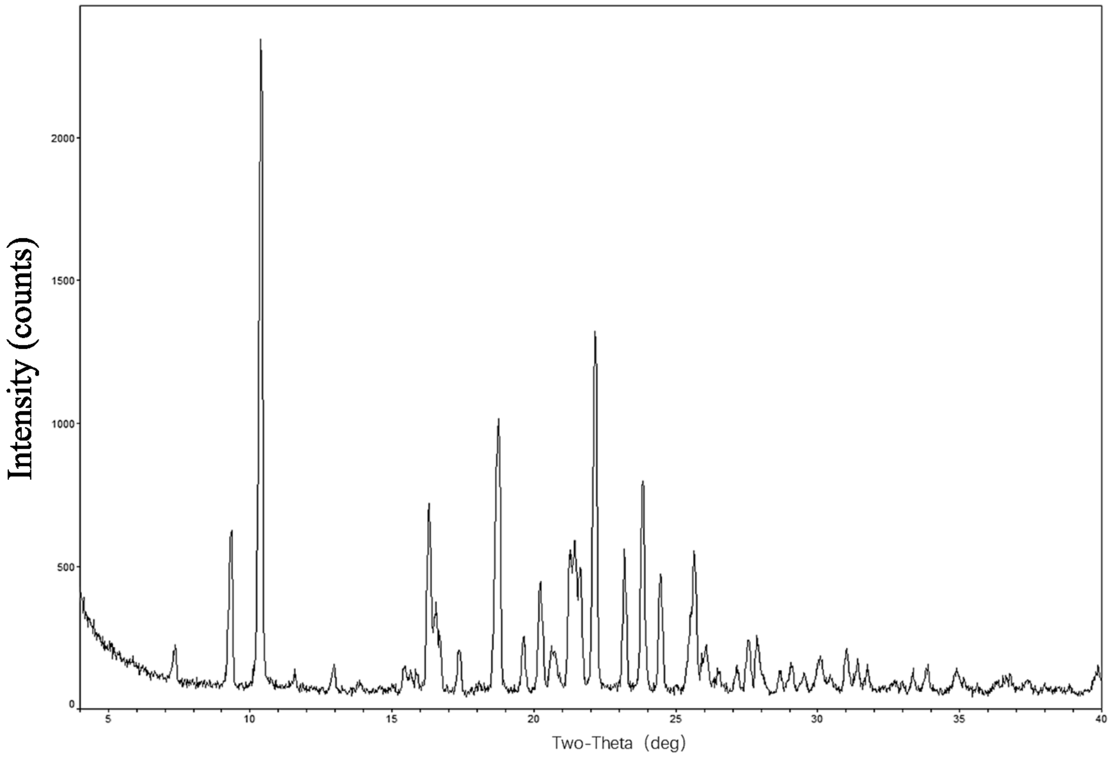
Intensity (counts)
2000
1500
1000
500
0
5
10
15
20
25
30
35
40
Two-Theta (deg)

*FIG. 37*

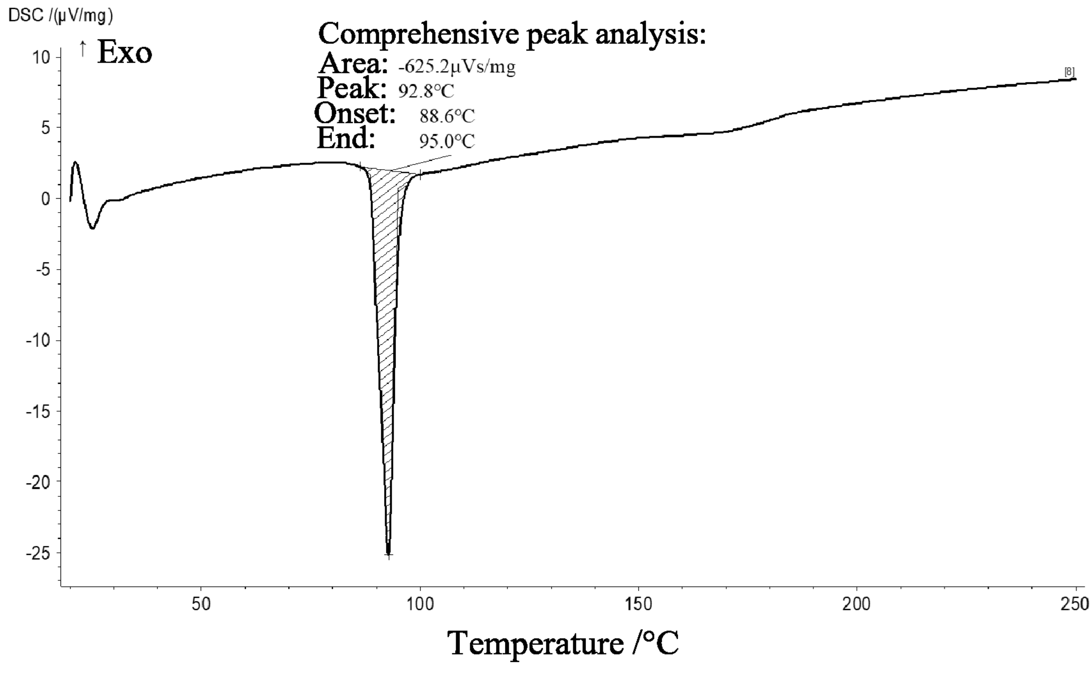
DSC /(μV/mg)
↑ Exo
Comprehensive peak analysis:
Area: -625.2μVs/mg
Peak: 92.8°C
Onset: 88.6°C
End: 95.0°C
[8]
10
5
0
-5
-10
-15
-20
-25
50
100
150
200
250
Temperature /°C

*FIG. 38*

**INTERNATIONAL SEARCH REPORT**

International application No.
**PCT/CN2024/119020**

**A. CLASSIFICATION OF SUBJECT MATTER**

C07D 401/14(2006.01)i； C07D471/04(2006.01)i； A61P35/00(2006.01)i； A61K31/506(2006.01)i

According to International Patent Classification (IPC) or to both national classification and IPC

**B. FIELDS SEARCHED**

Minimum documentation searched (classification system followed by classification symbols)

IPC:C07D A61P A61K

Documentation searched other than minimum documentation to the extent that such documents are included in the fields searched

Electronic data base consulted during the international search (name of data base and, where practicable, search terms used)

CNTXT; CNABS; ENTXT; DWPI; ENTXTC; WPABS; STN; CNKI; ISI-Web of Science: 上海翰森, 江苏豪森, 癌症, 肿瘤, cancer, tumor, EGFR, 晶型, 结晶, 晶体, 盐, 酸加成盐, 结构式I, crystal+, salt, 异喹啉, +quinoline

**C. DOCUMENTS CONSIDERED TO BE RELEVANT**

| Category* | Citation of document, with indication, where appropriate, of the relevant passages | Relevant to claim No. |
|---|---|---|
| PX | WO 2023174406 A1 (SHANGHAI HANSOH BIOMEDICAL CO., LTD. et al.) 21 September 2023 (2023-09-21)<br>claims 1-16 | 1-18 |
| X | WO 2022271630 A1 (BLUEPRINT MEDICINES CORP.) 29 December 2022 (2022-12-29)<br>description, pages 2-26, and embodiments | 1-18 |
| X | CN 116478136 A (SUZHOU PUHE BIOPHARMA CO., LTD.) 25 July 2023 (2023-07-25)<br>claims 1-37, and description, paragraphs [0069]-[0376], [0604], [0606], and [0608] | 1-18 |
| X | CN 115650958 A (CHINA PHARMACEUTICAL UNIVERSITY) 31 January 2023 (2023-01-31)<br>claims 1-10 | 1-18 |
| X | CN 115724827 A (SHANGHAI HANSOH BIOMEDICAL CO., LTD. et al.) 03 March 2023 (2023-03-03)<br>claims 1-18 | 1-18 |
| A | CN 116554150 A (SUZHOU PUHE BIOPHARMA CO., LTD.) 08 August 2023 (2023-08-08)<br>entire document | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 November 2024** | **03 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/119020**

**C. DOCUMENTS CONSIDERED TO BE RELEVANT**

| Category* | Citation of document, with indication, where appropriate, of the relevant passages | Relevant to claim No. |
|---|---|---|
| A | US 2016257682 A1 (GENENTECH, INC.) 08 September 2016 (2016-09-08)<br>entire document | 1-18 |

Form PCT/ISA/210 (second sheet) (July 2022)

**INTERNATIONAL SEARCH REPORT**
**Information on patent family members**

International application No.
**PCT/CN2024/119020**

| Patent document cited in search report | | | Publication date (day/month/year) | Patent family member(s) | | | Publication date (day/month/year) |
|---|---|---|---|---|---|---|---|
| WO | 2023174406 | A1 | 21 September 2023 | TW | 202400579 | A | 01 January 2024 |
| | | | | AU | 2023233629 | A1 | 26 September 2024 |
| WO | 2022271630 | A1 | 29 December 2022 | US | 2024300946 | A1 | 12 September 2024 |
| | | | | CN | 117940424 | A | 26 April 2024 |
| CN | 116478136 | A | 25 July 2023 | WO | 2023134266 | A1 | 20 July 2023 |
| | | | | AU | 2022432354 | A1 | 01 August 2024 |
| | | | | TW | 202330494 | A | 01 August 2023 |
| | | | | KR | 20240134949 | A | 10 September 2024 |
| | | | | EP | 4450495 | A1 | 23 October 2024 |
| | | | | HK | 40088838 | A0 | 06 October 2023 |
| | | | | SG | 11202404911 | A | 30 August 2024 |
| CN | 115650958 | A | 31 January 2023 | CN | 115650958 | B | 17 May 2024 |
| CN | 115724827 | A | 03 March 2023 | WO | 2023025320 | A1 | 02 March 2023 |
| | | | | TW | 202328100 | A | 16 July 2023 |
| | | | | AU | 2022334647 | A1 | 15 February 2024 |
| | | | | JP | 2024532835 | A | 10 September 2024 |
| | | | | KR | 20240051987 | A | 22 April 2024 |
| | | | | EP | 4393916 | A1 | 03 July 2024 |
| | | | | CA | 3229800 | A1 | 02 March 2023 |
| | | | | SG | 11202400752 | A | 28 March 2024 |
| | | | | CN | 117813299 | A | 02 April 2024 |
| | | | | IN | 202427016856 | A | 24 May 2024 |
| | | | | MX | 2024002332 | A1 | 22 February 2024 |
| CN | 116554150 | A | 08 August 2023 | | None | | |
| US | 2016257682 | A1 | 08 September 2016 | US | 9951064 | B2 | 24 April 2018 |
| | | | | WO | 2014210354 | A1 | 31 December 2014 |
| | | | | EP | 3052494 | A1 | 10 August 2016 |
| | | | | EP | 3052494 | B1 | 26 December 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023082178 W